# EUROPEAN PATENT APPLICATION

(11) **EP 2 341 143 A1**
(43) Date of publication of application: **06.07.2011**
(21) Application number: 10182850.7
(22) Date of filing: 11.05.2000
(51) Int. Cl.: C12N 15/12, C07K 14/705, C07K 16/28, A61K 38/17, G01N 33/50

(54) **OX2 receptor homolog**

(30) Priority: 13.05.1999 GB 9911123; 03.11.1999 GB 9925989
(62) Divisional of application: 00932337.9
(71) Applicant: Medical Research Council, London W1B 1AL (GB); Schering Corporation, Kenilworth, NJ 07033-0530 (US)
(72) Inventor: Barclay, Neil A., Oxford Oxfordshire OX4 1LY (GB); Brown, Marion H., Oxford Oxfordshire OX2 0BA (GB); Gorman, Daniel M., Newark, CA 94560 (US); Lanier, Lewis L., Los Altos, CA 94024 (US); Wright, Gavin J., Huddersfield, HD7 2XP (GB); Cherwinski, Holly, Boulder Creek, CA 95006 (US); Phillips, Joseph H., Palo Alto, CA 94303 (US); Hoek, Robert M., Mountain View, CA 94040 (US); Sedgwick, Jonathan D., Palo Alto, CA 94301 (US)
(74) Representative: Zvesper, Thomas

(57) **Abstract**

Nucleic acids encoding mammalian, e.g., primate, receptors of OX2, purified proteins and fragments thereof Antibodies, both polyclonal and monoclonal, are also provided. Method of using the compositions for both diagnostic and therapeutic utilities are described.

<110> Medical Research Council
      Schering Corporation

<120> OX2 Receptor Homologs

<130> DX01052K1 PCT

<140> PCT/US 00/12998
<141> 2000-05-11

<150> GB 9911123.9
<151> 1999-05-13

<150> GB 9925989.7
<151> 1999-11-03

<160> 24

<170> PatentIn Ver. 2.0

<210> 1
<211> 1574
<212> DNA
<213> Unknown

<220>
<223> Description of Unknown Organism:rodent; surmised
      rattus rattus

<220>

<221> CDS
<222> (91)..(1071)

<220>
<221> mat_peptide
<222> (162)..(1071)

<400> 1
agcggaggga tcctggtcat ggtcaccgct gctcccctac ctgtgaagag aaagagcacc 60

gagtgagccg ctgaaaacca gaaaaccgaa atg ctc tgc ttt tgg aga act tct 114
Met Leu Cys Phe Trp Arg Thr Ser
-20

cac gta gca gta ctc ttg atc tgg ggg gtc ttc gcg gct gag tca agt 162
His Val Ala Val Leu Leu Ile Trp Gly Val Phe Ala Ala Glu Ser Ser
-15 -10 -5 -1

tgt cct gat aag aat caa aca atg cag aac aat tca tca act atg aca 210
Cys Pro Asp Lys Asn Gln Thr Met Gln Asn Asn Ser Ser Thr Met Thr
1               5                   10                  15

gaa gtt aac act aca gtg ttt gta cag atg ggt aaa aag gct ctg ctc 258
Glu Val Asn Thr Thr Val Phe Val Gln Met Gly Lys Lys Ala Leu Leu
            20                  25                  30

tgc tgc cct tct att tca ctg aca aaa gta ata tta ata aca tgg aca 306
Cys Cys Pro Ser Ile Ser Leu Thr Lys Val Ile Leu Ile Thr Trp Thr
        35                  40                  45

ata acc ctc aga gga cag cct tcc tgc ata ata tcc tac aaa gca gac 354
Ile Thr Leu Arg Gly Gln Pro Ser Cys Ile Ile Ser Tyr Lys Ala Asp
    50                  55                  60

aca agg gag acc cat gaa agc aac tgc tcg gac aga agc atc acc tgg 402
Thr Arg Glu Thr His Glu Ser Asn Cys Ser Asp Arg Ser Ile Thr Trp
65                  70                  75                  80

gcc tcc aca cct gac ctc gct cct gac ctt cag atc agt gca gtg gcc 450
Ala Ser Thr Pro Asp Leu Ala Pro Asp Leu Gln Ile Ser Ala Val Ala
                85                  90                  95

ctc cag cat gaa ggg cgt tac tca tgt gat ata gca gta cct gac ggg 498
Leu Gln His Glu Gly Arg Tyr Ser Cys Asp Ile Ala Val Pro Asp Gly
            100                 105                 110

aat ttc caa aac atc tat gac ctc caa gtg ctg gtg ccc cct gaa gta 546
Asn Phe Gln Asn Ile Tyr Asp Leu Gln Val Leu Val Pro Pro Glu Val
        115                 120                 125

acc cac ttt cca ggg gaa aat aga act gca gtt tgt gag gcg att gca 594
Thr His Phe Pro Gly Glu Asn Arg Thr Ala Val Cys Glu Ala Ile Ala
    130                 135                 140

ggc aaa cct gct gcg cag atc tct tgg acg cca gat ggg gat tgt gtc 642
Gly Lys Pro Ala Ala Gln Ile Ser Trp Thr Pro Asp Gly Asp Cys Val
145                 150                 155                 160

gct aag aat gaa tca cac agc aat ggc acc gtg act gtc cgg agc aca 690
Ala Lys Asn Glu Ser His Ser Asn Gly Thr Val Thr Val Arg Ser Thr
                165                 170                 175

tgc cac tgg gag cag agc cac gtg tct gtc gtg ttc tgt gtt gtc tct 738
Cys His Trp Glu Gln Ser His Val Ser Val Val Phe Cys Val Val Ser
            180                 185                 190

cac ttg aca act ggt aac cag tct ctg tct ata gaa ctg ggt aga ggg 786
His Leu Thr Thr Gly Asn Gln Ser Leu Ser Ile Glu Leu Gly Arg Gly
        195                 200                 205

ggt gac caa tta tta gga tca tac att caa tac atc atc cca tct att 834
Gly Asp Gln Leu Leu Gly Ser Tyr Ile Gln Tyr Ile Ile Pro Ser Ile
    210                 215                 220

att att ttg atc atc ata gga tgc att tgt ctt ttg aaa atc agt ggc 882
Ile Ile Leu Ile Ile Ile Gly Cys Ile Cys Leu Leu Lys Ile Ser Gly
225                 230                 235                 240

tgc aga aaa tgt aaa ttg cca aaa tcg gga gct act cca gat att gag 930
Cys Arg Lys Cys Lys Leu Pro Lys Ser Gly Ala Thr Pro Asp Ile Glu
                245                 250                 255

gag gat gaa atg cag ccg tat gct agc tac aca gag aag agc aat cca 978
Glu Asp Glu Met Gln Pro Tyr Ala Ser Tyr Thr Glu Lys Ser Asn Pro
            260                 265                 270

ctc tat gat act gtg acc acg acg gag gca cac cca gcg tca caa ggc 1026
Leu Tyr Asp Thr Val Thr Thr Thr Glu Ala His Pro Ala Ser Gln Gly
        275                 280                 285

aaa gtc aat ggc aca gac tgt ctt act ttg tca gcc atg gga atc 1071
Lys Val Asn Gly Thr Asp Cys Leu Thr Leu Ser Ala Met Gly Ile
    290                 295                 300

tagaaccaag gaaaagaagt caagagacat cataattact gcttttcttt ctttaaactt 1131

ctccaatgga gggaaattag ctcttctgaa gttcttagaa agcacaaatg ttctaatgga 1191

tttgccttta agttcttcta tcattggaag tttggaatct ttgctgctac ctgttaattc 1251

taggaagaac tgatttaatt attacaaaga aagcacattg ttatggtaaa atatcaaatt 1311

gtgcaataca atgatgaaaa ctgagtttcc tcaagaaata actgcagaag gaacaatcat 1371

tactaaagca tttcatgtga gttcttccaa aaaagaaaat ccctgtgtat acgacatgat 1431

tatggtatgt gtgtgccttt atatgtttgt ttacaaatgt gtatatatgc acacatctga 1491

ttatcaagac atctctgtca aaaactcact ggcgttccag atttatgaaa gctaataaag 1551

tgagtattgg agatgttttt ata 1574

<210> 2

<211> 327
<212> PRT
<213> Unknown

<400> 2
Met Leu Cys Phe Trp Arg Thr Ser His Val Ala Val Leu Leu Ile Trp
                -20                 -15                 -10

Gly Val Phe Ala Ala Glu Ser Ser Cys Pro Asp Lys Asn Gln Thr Met
            -5                  -1  1               5

Gln Asn Asn Ser Ser Thr Met Thr Glu Val Asn Thr Thr Val Phe Val
    10                  15                  20

Gln Met Gly Lys Lys Ala Leu Leu Cys Cys Pro Ser Ile Ser Leu Thr
25                  30                  35                  40

Lys Val Ile Leu Ile Thr Trp Thr Ile Thr Leu Arg Gly Gln Pro Ser
                45                  50                  55

Cys Ile Ile Ser Tyr Lys Ala Asp Thr Arg Glu Thr His Glu Ser Asn
            60                  65                  70

Cys Ser Asp Arg Ser Ile Thr Trp Ala Ser Thr Pro Asp Leu Ala Pro
        75                  80                  85

Asp Leu Gln Ile Ser Ala Val Ala Leu Gln His Glu Gly Arg Tyr Ser
    90                  95                  100

Cys Asp Ile Ala Val Pro Asp Gly Asn Phe Gln Asn Ile Tyr Asp Leu
105                 110                 115                 120

Gln Val Leu Val Pro Pro Glu Val Thr His Phe Pro Gly Glu Asn Arg
                125                 130                 135

Thr Ala Val Cys Glu Ala Ile Ala Gly Lys Pro Ala Ala Gln Ile Ser
            140                 145                 150

Trp Thr Pro Asp Gly Asp Cys Val Ala Lys Asn Glu Ser His Ser Asn
        155                 160                 165

Gly Thr Val Thr Val Arg Ser Thr Cys His Trp Glu Gln Ser His Val
    170                 175                 180

Ser Val Val Phe Cys Val Val Ser His Leu Thr Thr Gly Asn Gln Ser
185                 190                 195                 200

Leu Ser Ile Glu Leu Gly Arg Gly Gly Asp Gln Leu Leu Gly Ser Tyr
                205                 210                 215

Ile Gln Tyr Ile Ile Pro Ser Ile Ile Ile Leu Ile Ile Ile Gly Cys
            220                 225                 230

Ile Cys Leu Leu Lys Ile Ser Gly Cys Arg Lys Cys Lys Leu Pro Lys
        235                 240                 245

Ser Gly Ala Thr Pro Asp Ile Glu Glu Asp Glu Met Gln Pro Tyr Ala

250                 255                 260

Ser Tyr Thr Glu Lys Ser Asn Pro Leu Tyr Asp Thr Val Thr Thr Thr
265                 270                 275                 280

Glu Ala His Pro Ala Ser Gln Gly Lys Val Asn Gly Thr Asp Cys Leu
                285                 290                 295

Thr Leu Ser Ala Met Gly Ile
            300


<210> 3
<211> 1604
<212> DNA
<213> Unknown

<220>
<223> Description of Unknown Organism:primate; surmised
      homo sapiens

<220>
<221> CDS
<222> (217)..(1101)

<220>
<221> mat_peptide
<222> (295)..(1101)

<400> 3
cagagaaaag cttctgttcg tccaagttac taaccaggct aaaccacata gacgtgaagg 60

aaggggctag aaggaaggga gtgccccact gttgatgggg taagaggatc ctgtactgag 120

aagttgacca gagagggtct caccatgcgc acagttcctt ctgtaccagt gtggaggaaa 180

agtactgagt gaagggcaga aaaagagaaa acagaa atg ctc tgc cct tgg aga   234
                                        Met Leu Cys Pro Trp Arg
                                            -25

act gct aac cta ggg cta ctg ttg att ttg act atc ttc tta gtg gcc   282
Thr Ala Asn Leu Gly Leu Leu Leu Ile Leu Thr Ile Phe Leu Val Ala
-20                 -15                 -10                 -5

gaa gcg gag ggt gct gct caa cca aac aac tca tta atg ctg caa act   330
Glu Ala Glu Gly Ala Ala Gln Pro Asn Asn Ser Leu Met Leu Gln Thr
            -1   1               5                  10

agc aag gag aat cat gct tta gct tca agc agt tta tgt atg gat gaa   378
Ser Lys Glu Asn His Ala Leu Ala Ser Ser Ser Leu Cys Met Asp Glu
        15                  20                  25

aaa cag att aca cag aac tac tcg aaa gta ctc gca gaa gtt aac act   426
Lys Gln Ile Thr Gln Asn Tyr Ser Lys Val Leu Ala Glu Val Asn Thr
    30                  35                  40

tca tgg cct gta aag atg gct aca aat gct gtg ctt tgt tgc cct cct   474

Ser Trp Pro Val Lys Met Ala Thr Asn Ala Val Leu Cys Cys Pro Pro
 45                  50                  55                  60

atc gca tta aga aat ttg atc ata ata aca tgg gaa ata atc ctg aga     522
Ile Ala Leu Arg Asn Leu Ile Ile Ile Thr Trp Glu Ile Ile Leu Arg
                65                  70                  75

ggc cag cct tcc tgc aca aaa gcc tac aag aaa gaa aca aat gag acc     570
Gly Gln Pro Ser Cys Thr Lys Ala Tyr Lys Lys Glu Thr Asn Glu Thr
            80                  85                  90

aag gaa acc aac tgt act gat gag aga ata acc tgg gtc tcc aga cct     618
Lys Glu Thr Asn Cys Thr Asp Glu Arg Ile Thr Trp Val Ser Arg Pro
        95                  100                 105

gat cag aat tcg gac ctt cag att cgt acc gtg gcc atc act cat gac     666
Asp Gln Asn Ser Asp Leu Gln Ile Arg Thr Val Ala Ile Thr His Asp
    110                 115                 120

ggg tat tac aga tgc ata atg gta aca cct gat ggg aat ttc cat cgt     714
Gly Tyr Tyr Arg Cys Ile Met Val Thr Pro Asp Gly Asn Phe His Arg
125                 130                 135                 140

gga tat cac ctc caa gtg tta gtt aca cct gaa gtg acc ctg ttt caa     762
Gly Tyr His Leu Gln Val Leu Val Thr Pro Glu Val Thr Leu Phe Gln
                145                 150                 155

aac agg aat aga act gca gta tgc aag gca gtt gca ggg aag cca gct     810
Asn Arg Asn Arg Thr Ala Val Cys Lys Ala Val Ala Gly Lys Pro Ala
            160                 165                 170

gcg cat atc tcc tgg atc cca gag ggc gat tgt gcc act aag caa gaa     858
Ala His Ile Ser Trp Ile Pro Glu Gly Asp Cys Ala Thr Lys Gln Glu
        175                 180                 185

tac tgg agc aat ggc aca gtg act gtt aag agt aca tgc cac tgg gag     906
Tyr Trp Ser Asn Gly Thr Val Thr Val Lys Ser Thr Cys His Trp Glu
    190                 195                 200

gtc cac aat gtg tct acc gtg acc tgc cac gtc tcc cat ttg act ggc     954
Val His Asn Val Ser Thr Val Thr Cys His Val Ser His Leu Thr Gly
205                 210                 215                 220

aac aag agt ctg tac ata gag cta ctt cct gtt cca ggt gcc aaa aaa     1002
Asn Lys Ser Leu Tyr Ile Glu Leu Leu Pro Val Pro Gly Ala Lys Lys
                225                 230                 235

atc agc aaa att ata tat tcc ata tat cat cct tac tat tat tat tta     1050
Ile Ser Lys Ile Ile Tyr Ser Ile Tyr His Pro Tyr Tyr Tyr Tyr Leu
            240                 245                 250

gac cat cgt ggg att cat ttg gtt gtt gaa agt caa tgg ctg cag aaa     1098
Asp His Arg Gly Ile His Leu Val Val Glu Ser Gln Trp Leu Gln Lys
        255                 260                 265

ata taaattgaat aaaacagaat ctactccagt tgttgaggag gatgaaatgc          1151
Ile

agccctatgc cagctacaca gagaagaaca atcctctcta tgatactaca aacaaggtga 1211

aggcatctga ggcattacaa agtgaagttg acacagacct ccatacttta taagttgttg 1271

gactctagta ccaagaaaca acaacaaacg agatacatta taattactgt ctgattttct 1331

tacagttcta gaatgaagac ttatattgaa attaggtttt ccaaggttct tagaagacat 1391

tttaatggat tctcattcat acccttgtat aattggaatt tttgattctt agctgctacc 1451

agctagttct ctgaagaact gatgttatta caaagaaaat acatgcccat gaccaaatat 1511

tcaaattgtg caggacagta aataatgaaa accaaatttc ctcaagaaat aactgaagaa 1571

ggagcaagtg tgaacagttt cttgtgtatc ctt 1604

<210> 4
<211> 295
<212> PRT
<213> Unknown

<400> 4
Met Leu Cys Pro Trp Arg Thr Ala Asn Leu Gly Leu Leu Leu Ile Leu
-25 -20 -15

Thr Ile Phe Leu Val Ala Glu Ala Glu Gly Ala Ala Gln Pro Asn Asn
-10 -5 -1 1 5

Ser Leu Met Leu Gln Thr Ser Lys Glu Asn His Ala Leu Ala Ser Ser
10 15 20

Ser Leu Cys Met Asp Glu Lys Gln Ile Thr Gln Asn Tyr Ser Lys Val
25 30 35

Leu Ala Glu Val Asn Thr Ser Trp Pro Val Lys Met Ala Thr Asn Ala
40 45 50

Val Leu Cys Cys Pro Pro Ile Ala Leu Arg Asn Leu Ile Ile Ile Thr
55 60 65 70

Trp Glu Ile Ile Leu Arg Gly Gln Pro Ser Cys Thr Lys Ala Tyr Lys
75 80 85

Lys Glu Thr Asn Glu Thr Lys Glu Thr Asn Cys Thr Asp Glu Arg Ile
90 95 100

Thr Trp Val Ser Arg Pro Asp Gln Asn Ser Asp Leu Gln Ile Arg Thr
105 110 115

Val Ala Ile Thr His Asp Gly Tyr Tyr Arg Cys Ile Met Val Thr Pro
120 125 130

Asp Gly Asn Phe His Arg Gly Tyr His Leu Gln Val Leu Val Thr Pro
135 140 145 150

Glu Val Thr Leu Phe Gln Asn Arg Asn Arg Thr Ala Val Cys Lys Ala
                155                 160                 165

Val Ala Gly Lys Pro Ala Ala His Ile Ser Trp Ile Pro Glu Gly Asp
            170                 175                 180

Cys Ala Thr Lys Gln Glu Tyr Trp Ser Asn Gly Thr Val Thr Val Lys
        185                 190                 195

Ser Thr Cys His Trp Glu Val His Asn Val Ser Thr Val Thr Cys His
    200                 205                 210

Val Ser His Leu Thr Gly Asn Lys Ser Leu Tyr Ile Glu Leu Leu Pro
215                 220                 225                 230

Val Pro Gly Ala Lys Lys Ile Ser Lys Ile Ile Tyr Ser Ile Tyr His
                235                 240                 245

Pro Tyr Tyr Tyr Tyr Leu Asp His Arg Gly Ile His Leu Val Val Glu
            250                 255                 260

Ser Gln Trp Leu Gln Lys Ile
        265


<210> 5
<211> 1490
<212> DNA
<213> Unknown

<220>
<223> Description of Unknown Organism:rodent; surmised
      mus musculus

<220>
<221> CDS
<222> (10)..(987)

<220>
<221> mat_peptide
<222> (85)..(987)

<400> 5
aaaaccgaa atg ttt tgc ttt tgg aga act tct gcc cta gca gtg ctc tta 51
          Met Phe Cys Phe Trp Arg Thr Ser Ala Leu Ala Val Leu Leu
          -25                 -20                 -15

ata tgg ggg gtc ttt gtg gct ggg tca agt tgt act gat aag aat caa    99
Ile Trp Gly Val Phe Val Ala Gly Ser Ser Cys Thr Asp Lys Asn Gln
    -10                 -5                  -1  1               5

aca aca cag aac aac agt tca tct cct ctg aca caa gtg aac act aca    147
Thr Thr Gln Asn Asn Ser Ser Ser Pro Leu Thr Gln Val Asn Thr Thr
                10                  15                  20

gtg tct gta cag ata ggt aca aag gct ctg ctc tgc tgc ttt tct att    195
Val Ser Val Gln Ile Gly Thr Lys Ala Leu Leu Cys Cys Phe Ser Ile

25 30 35

cca ctg aca aaa gca gta tta atc aca tgg ata ata aag ctc aga ggc 243
Pro Leu Thr Lys Ala Val Leu Ile Thr Trp Ile Ile Lys Leu Arg Gly
40 45 50

ctg cca tcc tgc aca ata gca tac aaa gta gat aca aag acc aat gaa 291
Leu Pro Ser Cys Thr Ile Ala Tyr Lys Val Asp Thr Lys Thr Asn Glu
55 60 65

acc agc tgc ttg ggc agg aac atc acc tgg gcc tcc aca cct gac cac 339
Thr Ser Cys Leu Gly Arg Asn Ile Thr Trp Ala Ser Thr Pro Asp His
70 75 80 85

agt cct gaa ctt cag atc agt gca gtg acc ctc cag cat gag ggg act 387
Ser Pro Glu Leu Gln Ile Ser Ala Val Thr Leu Gln His Glu Gly Thr
90 95 100

tac aca tgt gag aca gta aca cct gaa ggg aat ttt gaa aaa aac tat 435
Tyr Thr Cys Glu Thr Val Thr Pro Glu Gly Asn Phe Glu Lys Asn Tyr
105 110 115

gac ctc caa gtg ctg gtg ccc cct gaa gta acc tac ttt cca gag aaa 483
Asp Leu Gln Val Leu Val Pro Pro Glu Val Thr Tyr Phe Pro Glu Lys
120 125 130

aac aga tct gca gtc tgt gag gca atg gca ggc aag cct gct gca cag 531
Asn Arg Ser Ala Val Cys Glu Ala Met Ala Gly Lys Pro Ala Ala Gln
135 140 145

atc tct tgg tct cca gat ggg gac tgt gtc act acg agt gaa tca cac 579
Ile Ser Trp Ser Pro Asp Gly Asp Cys Val Thr Thr Ser Glu Ser His
150 155 160 165

agc aat ggc act gtg act gtc agg agc aca tgc cac tgg gag cag aac 627
Ser Asn Gly Thr Val Thr Val Arg Ser Thr Cys His Trp Glu Gln Asn
170 175 180

aat gtg tct gat gtg tcc tgc att gtc tct cat ttg act ggt aac caa 675
Asn Val Ser Asp Val Ser Cys Ile Val Ser His Leu Thr Gly Asn Gln
185 190 195

tct ctg tcc ata gaa ctg agt aga ggt ggt aac caa tca tta cga cca 723
Ser Leu Ser Ile Glu Leu Ser Arg Gly Gly Asn Gln Ser Leu Arg Pro
200 205 210

tat att cca tac atc ata cca tca att atc att ttg atc atc ata gga 771
Tyr Ile Pro Tyr Ile Ile Pro Ser Ile Ile Ile Leu Ile Ile Ile Gly
215 220 225

tgc att tgt ctt ttg aaa atc agt ggc ttc aga aaa tgc aaa ttg cca 819
Cys Ile Cys Leu Leu Lys Ile Ser Gly Phe Arg Lys Cys Lys Leu Pro
230 235 240 245

aaa tta gaa gct act tca gct att gag gag gat gaa atg cag cct tat 867
Lys Leu Glu Ala Thr Ser Ala Ile Glu Glu Asp Glu Met Gln Pro Tyr
250 255 260

gct agc tat aca gag aag agc aat cca ctc tat gat act gtg act aag 915
Ala Ser Tyr Thr Glu Lys Ser Asn Pro Leu Tyr Asp Thr Val Thr Lys
265 270 275

gtg gag gca ttt cca gta tca caa ggc gaa gtc aat ggc aca gac tgc 963
Val Glu Ala Phe Pro Val Ser Gln Gly Glu Val Asn Gly Thr Asp Cys
280 285 290

ctt act ttg tcg gcc att gga atc tagaaccaag aaaaaagaag tcaagagaca 1017
Leu Thr Leu Ser Ala Ile Gly Ile
295 300

tcataattac tgctttgctt tctttaaaat tcgacaatgg aaggactact tggaaattag 1077

ctcttccaaa gctattaaaa agcacaaatg ttctaatgaa attgcattta aattctatca 1137

ttggaagttt ggaatctctg ctgctacctg ttaattttag gaagaactga tttaattatt 1197

acaaagaaag cacatggtta tggtgaaata tcaagttgtg caataaagta tgatgaaaac 1257

tgagtttcct caagaaataa ctgcaggagg aacaatcatc actaaagaat ttcatgtgag 1317

ttcttacaaa aaaattccta tgtatacatg actatggtat gtgtgtccaa ttacatgttt 1377

atttacaaat gtgtatatat gcacacattt gcttttcagg acatctcctt gtaaaaaaca 1437

cactggagtt ttggatttat aaaagcttat aaagtgagca ttggagatat ttt 1490

<210> 6
<211> 326
<212> PRT
<213> Unknown

<400> 6
Met Phe Cys Phe Trp Arg Thr Ser Ala Leu Ala Val Leu Leu Ile Trp
-25 -20 -15 -10

Gly Val Phe Val Ala Gly Ser Ser Cys Thr Asp Lys Asn Gln Thr Thr
-5 -1 1 5

Gln Asn Asn Ser Ser Ser Pro Leu Thr Gln Val Asn Thr Thr Val Ser
10 15 20

Val Gln Ile Gly Thr Lys Ala Leu Leu Cys Cys Phe Ser Ile Pro Leu
25 30 35

Thr Lys Ala Val Leu Ile Thr Trp Ile Ile Lys Leu Arg Gly Leu Pro
40 45 50 55

Ser Cys Thr Ile Ala Tyr Lys Val Asp Thr Lys Thr Asn Glu Thr Ser
60 65 70

Cys Leu Gly Arg Asn Ile Thr Trp Ala Ser Thr Pro Asp His Ser Pro
75 80 85

Glu Leu Gln Ile Ser Ala Val Thr Leu Gln His Glu Gly Thr Tyr Thr
        90                  95                  100

Cys Glu Thr Val Thr Pro Glu Gly Asn Phe Glu Lys Asn Tyr Asp Leu
    105                 110                 115

Gln Val Leu Val Pro Pro Glu Val Thr Tyr Phe Pro Glu Lys Asn Arg
120                 125                 130                 135

Ser Ala Val Cys Glu Ala Met Ala Gly Lys Pro Ala Ala Gln Ile Ser
                140                 145                 150

Trp Ser Pro Asp Gly Asp Cys Val Thr Thr Ser Glu Ser His Ser Asn
            155                 160                 165

Gly Thr Val Thr Val Arg Ser Thr Cys His Trp Glu Gln Asn Asn Val
        170                 175                 180

Ser Asp Val Ser Cys Ile Val Ser His Leu Thr Gly Asn Gln Ser Leu
    185                 190                 195

Ser Ile Glu Leu Ser Arg Gly Gly Asn Gln Ser Leu Arg Pro Tyr Ile
200                 205                 210                 215

Pro Tyr Ile Ile Pro Ser Ile Ile Ile Leu Ile Ile Ile Gly Cys Ile
                220                 225                 230

Cys Leu Leu Lys Ile Ser Gly Phe Arg Lys Cys Lys Leu Pro Lys Leu
            235                 240                 245

Glu Ala Thr Ser Ala Ile Glu Glu Asp Glu Met Gln Pro Tyr Ala Ser
        250                 255                 260

Tyr Thr Glu Lys Ser Asn Pro Leu Tyr Asp Thr Val Thr Lys Val Glu
    265                 270                 275

Ala Phe Pro Val Ser Gln Gly Glu Val Asn Gly Thr Asp Cys Leu Thr
280                 285                 290                 295

Leu Ser Ala Ile Gly Ile
                300

<210> 7
<211> 1010
<212> DNA
<213> Unknown

<220>
<223> Description of Unknown Organism:primate; surmised
      homo sapiens

<220>
<221> CDS
<222> (1)..(750)

<400> 7

atg ggt gga aag cag atg aca cag aac tat tca aca att ttt gca gaa    48
Met Gly Gly Lys Gln Met Thr Gln Asn Tyr Ser Thr Ile Phe Ala Glu
1               5                   10                  15

ggt aac att tca cag cct gta ctg atg gat ata aat gct gtg ctt tgt    96
Gly Asn Ile Ser Gln Pro Val Leu Met Asp Ile Asn Ala Val Leu Cys
            20                  25                  30

tgc cct cct att gca tta aga aat ttg atc ata ata aca tgg gaa ata    144
Cys Pro Pro Ile Ala Leu Arg Asn Leu Ile Ile Ile Thr Trp Glu Ile
        35                  40                  45

atc ctg aga ggc cag cct tcc tgc aca aaa gcc tac aag aaa gaa aca    192
Ile Leu Arg Gly Gln Pro Ser Cys Thr Lys Ala Tyr Lys Lys Glu Thr
    50                  55                  60

aat gag acc aag gaa acc aac tgt act gtt gag aga ata acc tgg gtc    240
Asn Glu Thr Lys Glu Thr Asn Cys Thr Val Glu Arg Ile Thr Trp Val
65                  70                  75                  80

tct aga cct gat cag aat tcg gac ctt cag att cgt ccg gtg gac acc    288
Ser Arg Pro Asp Gln Asn Ser Asp Leu Gln Ile Arg Pro Val Asp Thr
                85                  90                  95

act cat gac ggg tat tac aga ggc ata gtg gta aca cct gat ggg aat    336
Thr His Asp Gly Tyr Tyr Arg Gly Ile Val Val Thr Pro Asp Gly Asn
            100                 105                 110

ttc cat cgt gga tat cac ctc caa gtg tta gtt aca ccc gaa gtg aac    384
Phe His Arg Gly Tyr His Leu Gln Val Leu Val Thr Pro Glu Val Asn
        115                 120                 125

cta ttt caa agc agg aat ata act gca gta tgc aag gca gtt aca ggg    432
Leu Phe Gln Ser Arg Asn Ile Thr Ala Val Cys Lys Ala Val Thr Gly
    130                 135                 140

aag cca gct gcc cag atc tcc tgg atc cca gag gga tct att ctt gcc    480
Lys Pro Ala Ala Gln Ile Ser Trp Ile Pro Glu Gly Ser Ile Leu Ala
145                 150                 155                 160

act aag caa gaa tac tgg ggc aat ggc aca gtg acg gtt aag agt aca    528
Thr Lys Gln Glu Tyr Trp Gly Asn Gly Thr Val Thr Val Lys Ser Thr
                165                 170                 175

tgc ccc tgg gag ggc cac aag tct act gtg acc tgc cat gtc tcc cat    576
Cys Pro Trp Glu Gly His Lys Ser Thr Val Thr Cys His Val Ser His
            180                 185                 190

ttg act ggc aac aag agt ctg tcc gta aag ttg aat tca ggt ctc aga    624
Leu Thr Gly Asn Lys Ser Leu Ser Val Lys Leu Asn Ser Gly Leu Arg
        195                 200                 205

acc tca gga tct cca gcg ttg tcc tta ctg atc att ctt tat gtg aaa    672
Thr Ser Gly Ser Pro Ala Leu Ser Leu Leu Ile Ile Leu Tyr Val Lys
    210                 215                 220

ctc tct ctt ttt gtg gtc att ctg gtc acc aca gga ttt gtt ttc ttc    720

Leu Ser Leu Phe Val Val Ile Leu Val Thr Thr Gly Phe Val Phe Phe
225                 230                 235                 240

cag agg ata aat cat gtc aga aaa gtt ctt taaagaagaa ggaagggtct      770
Gln Arg Ile Asn His Val Arg Lys Val Leu
                245                 250

tcttttgctt ctcctccttg tctctggact gcaacattgg tgagatgagt gatggtccag 830

cagtgaactt gggccatgga tgatgttaag gatagaagcc actcagtagg atagaagaaa 890

agaaagatgg aagaaggatc ctgggcttga tgaccatgaa gtttccctat aaaccctcaa 950

ccacctattc attgacttct tttgtgttag agtgaataaa attttgttca tgccagtgtt 1010

<210> 8
<211> 250
<212> PRT
<213> Unknown

<400> 8
Met Gly Gly Lys Gln Met Thr Gln Asn Tyr Ser Thr Ile Phe Ala Glu
  1               5                  10                  15

Gly Asn Ile Ser Gln Pro Val Leu Met Asp Ile Asn Ala Val Leu Cys
             20                  25                  30

Cys Pro Pro Ile Ala Leu Arg Asn Leu Ile Ile Ile Thr Trp Glu Ile
         35                  40                  45

Ile Leu Arg Gly Gln Pro Ser Cys Thr Lys Ala Tyr Lys Lys Glu Thr
     50                  55                  60

Asn Glu Thr Lys Glu Thr Asn Cys Thr Val Glu Arg Ile Thr Trp Val
 65                  70                  75                  80

Ser Arg Pro Asp Gln Asn Ser Asp Leu Gln Ile Arg Pro Val Asp Thr
                 85                  90                  95

Thr His Asp Gly Tyr Tyr Arg Gly Ile Val Val Thr Pro Asp Gly Asn
            100                 105                 110

Phe His Arg Gly Tyr His Leu Gln Val Leu Val Thr Pro Glu Val Asn
        115                 120                 125

Leu Phe Gln Ser Arg Asn Ile Thr Ala Val Cys Lys Ala Val Thr Gly
    130                 135                 140

Lys Pro Ala Ala Gln Ile Ser Trp Ile Pro Glu Gly Ser Ile Leu Ala
145                 150                 155                 160

Thr Lys Gln Glu Tyr Trp Gly Asn Gly Thr Val Thr Val Lys Ser Thr
                165                 170                 175

Cys Pro Trp Glu Gly His Lys Ser Thr Val Thr Cys His Val Ser His
            180                 185                 190

Leu Thr Gly Asn Lys Ser Leu Ser Val Lys Leu Asn Ser Gly Leu Arg
        195                 200                 205

Thr Ser Gly Ser Pro Ala Leu Ser Leu Leu Ile Ile Leu Tyr Val Lys
    210                 215                 220

Leu Ser Leu Phe Val Val Ile Leu Val Thr Thr Gly Phe Val Phe Phe
225                 230                 235                 240

Gln Arg Ile Asn His Val Arg Lys Val Leu
                245                 250


<210> 9
<211> 1085
<212> DNA
<213> Unknown

<220>
<223> Description of Unknown Organism:rodent; surmised
      mus musculus

<220>
<221> CDS
<222> (1)..(582)

<400> 9
aga ggc cag cct tcc tgc ata atg gcc tac aaa gta gaa aca aag gag      48
Arg Gly Gln Pro Ser Cys Ile Met Ala Tyr Lys Val Glu Thr Lys Glu
  1               5                  10                  15

acc aat gaa acc tgc ttg ggc agg aac atc acc tgg gcc tcc aca cct      96
Thr Asn Glu Thr Cys Leu Gly Arg Asn Ile Thr Trp Ala Ser Thr Pro
             20                  25                  30

gac cac att cct gac ctt cag atc agt gcg gtg gcc ctc cag cat gag     144
Asp His Ile Pro Asp Leu Gln Ile Ser Ala Val Ala Leu Gln His Glu
         35                  40                  45

ggg aat tac tta tgt gag ata aca aca cct gaa ggg aat ttc cat aaa     192
Gly Asn Tyr Leu Cys Glu Ile Thr Thr Pro Glu Gly Asn Phe His Lys
     50                  55                  60

gtc tat gac ctc caa gtg ctg gtg ccc cct gaa gta acc tac ttt ctc     240
Val Tyr Asp Leu Gln Val Leu Val Pro Pro Glu Val Thr Tyr Phe Leu
 65                  70                  75                  80

ggg gaa aat aga act gca gtt tgt gag gca atg gca ggc aag cct gct     288
Gly Glu Asn Arg Thr Ala Val Cys Glu Ala Met Ala Gly Lys Pro Ala
                 85                  90                  95

gca cag atc tct tgg act cca gat ggg gac tgt gtc act aag agt gag     336
Ala Gln Ile Ser Trp Thr Pro Asp Gly Asp Cys Val Thr Lys Ser Glu
            100                 105                 110

tca cac agc aat ggc act gtg act gtc agg agc act tgc cac tgg gag     384

Ser His Ser Asn Gly Thr Val Thr Val Arg Ser Thr Cys His Trp Glu
        115                 120                 125

cag aac aat gtg tct gct gtg tcc tgc att gtc tct cat tcg act ggt     432
Gln Asn Asn Val Ser Ala Val Ser Cys Ile Val Ser His Ser Thr Gly
    130                 135                 140

aat cag tct ctg tcc ata gaa ctg agt aga ggt acc acc agc acc acc     480
Asn Gln Ser Leu Ser Ile Glu Leu Ser Arg Gly Thr Thr Ser Thr Thr
145                 150                 155                 160

cct tcc ttg ctg acc att ctc tac gtg aaa atg gtc ctt ttg ggg att     528
Pro Ser Leu Leu Thr Ile Leu Tyr Val Lys Met Val Leu Leu Gly Ile
                165                 170                 175

att ctt ctt aaa gtg gga ttt gct ttc ttc cag aag aga aat gtt acc     576
Ile Leu Leu Lys Val Gly Phe Ala Phe Phe Gln Lys Arg Asn Val Thr
            180                 185                 190

aga aca tgaatatcca gatttctgga agctcattag tctgatgaca cataccagaa     632
Arg Thr

aacagcattt gtaatcaact ttctcattgg aatccagctt acccgtccct gctgtcttca 692

tgtttgttag acactcacct ccaaattctt aactgagaag ggctcctgtc taaaggaaat 752

atggggacaa attgtggagc atagaccaaa agaaaggcca tccagagact gccccaccta 812

aggacccatc ccatatacag acaccaaacc cagacactac tgaagatgct gcgaagcgtt 872

tgctgacagg agcctgttat agctgtctcc tgagaggctc agccagagcc tgacaaatac 932

ataggtagat gcttgcagcc aacaactgga ctgagcaaaa aatctccatt ggaggagtta 992

gagaaaggac tgaagagggt gaaagggttt gcagccccat aggaagaaca acaatatcaa 1052

ccaaccagat ctcccagagc tcccagggac taa                              1085

<210> 10
<211> 194
<212> PRT
<213> Unknown

<400> 10
Arg Gly Gln Pro Ser Cys Ile Met Ala Tyr Lys Val Glu Thr Lys Glu
1               5                   10                  15

Thr Asn Glu Thr Cys Leu Gly Arg Asn Ile Thr Trp Ala Ser Thr Pro
            20                  25                  30

Asp His Ile Pro Asp Leu Gln Ile Ser Ala Val Ala Leu Gln His Glu
        35                  40                  45

Gly Asn Tyr Leu Cys Glu Ile Thr Thr Pro Glu Gly Asn Phe His Lys
    50                  55                  60

Val Tyr Asp Leu Gln Val Leu Val Pro Pro Glu Val Thr Tyr Phe Leu
65                  70                  75                  80

Gly Glu Asn Arg Thr Ala Val Cys Glu Ala Met Ala Gly Lys Pro Ala
                85                  90                  95

Ala Gln Ile Ser Trp Thr Pro Asp Gly Asp Cys Val Thr Lys Ser Glu
            100                 105                 110

Ser His Ser Asn Gly Thr Val Thr Val Arg Ser Thr Cys His Trp Glu
        115                 120                 125

Gln Asn Asn Val Ser Ala Val Ser Cys Ile Val Ser His Ser Thr Gly
    130                 135                 140

Asn Gln Ser Leu Ser Ile Glu Leu Ser Arg Gly Thr Thr Ser Thr Thr
145                 150                 155                 160

Pro Ser Leu Leu Thr Ile Leu Tyr Val Lys Met Val Leu Leu Gly Ile
                165                 170                 175

Ile Leu Leu Lys Val Gly Phe Ala Phe Phe Gln Lys Arg Asn Val Thr
            180                 185                 190

Arg Thr

<210> 11
<211> 1354
<212> DNA
<213> Unknown

<220>
<223> Description of Unknown Organism:rodent; surmised
      mus musculus

<220>
<221> CDS
<222> (42)..(875)

<220>
<221> mat_peptide
<222> (117)..(875)

<400> 11
ggcacgagtt acgatttgtg cttaacctga ctccactcca g atg cat gct ttg ggg 56
                                              Met His Ala Leu Gly
                                              -25

agg act ctg gct ttg atg tta ctc atc ttc atc act att ttg gtg cct    104
Arg Thr Leu Ala Leu Met Leu Leu Ile Phe Ile Thr Ile Leu Val Pro
-20                 -15                 -10                 -5

gag tca agt tgt tca gtg aaa gga cgg gag gag atc cca ccg gat gat    152
Glu Ser Ser Cys Ser Val Lys Gly Arg Glu Glu Ile Pro Pro Asp Asp
            -1  1               5                   10

tca ttt cct ttt tca gat gat aat atc ttc cct gat gga gtg ggc gtc 200
Ser Phe Pro Phe Ser Asp Asp Asn Ile Phe Pro Asp Gly Val Gly Val
15 20 25

acc atg gag att gag att atc act cca gtg tct gta cag ata ggt atc 248
Thr Met Glu Ile Glu Ile Ile Thr Pro Val Ser Val Gln Ile Gly Ile
30 35 40

aag gct cag ctt ttc tgt cat cct agt cca tca aaa gaa gca aca ctt 296
Lys Ala Gln Leu Phe Cys His Pro Ser Pro Ser Lys Glu Ala Thr Leu
45 50 55 60

aga ata tgg gaa ata act ccc aga gac tgg cct tcc tgc aga cta ccc 344
Arg Ile Trp Glu Ile Thr Pro Arg Asp Trp Pro Ser Cys Arg Leu Pro
65 70 75

tac aga gca gag ttg cag cag atc agt aaa aaa atc tgt act gag aga 392
Tyr Arg Ala Glu Leu Gln Gln Ile Ser Lys Lys Ile Cys Thr Glu Arg
80 85 90

gga acc act agg gtc cct gca cat cac cag agt tct gac ctt ccc atc 440
Gly Thr Thr Arg Val Pro Ala His His Gln Ser Ser Asp Leu Pro Ile
95 100 105

aaa tca atg gcc ctc aag cat gat ggg cat tac tca tgt cgg ata gaa 488
Lys Ser Met Ala Leu Lys His Asp Gly His Tyr Ser Cys Arg Ile Glu
110 115 120

aca aca gat ggg att ttc caa gag aga cat agc atc caa gtg cca ggg 536
Thr Thr Asp Gly Ile Phe Gln Glu Arg His Ser Ile Gln Val Pro Gly
125 130 135 140

gaa aat aga act gta gtt tgt gag gca att gca agc aag cct gct atg 584
Glu Asn Arg Thr Val Val Cys Glu Ala Ile Ala Ser Lys Pro Ala Met
145 150 155

cag atc ttg tgg act cca gat gag gac tgt gtc act aag agt aaa tca 632
Gln Ile Leu Trp Thr Pro Asp Glu Asp Cys Val Thr Lys Ser Lys Ser
160 165 170

cac aat gac acc atg att gtc agg agc aag tgc cac agg gag aaa aac 680
His Asn Asp Thr Met Ile Val Arg Ser Lys Cys His Arg Glu Lys Asn
175 180 185

aat ggc cac agt gtg ttc tgc ttt atc tcc cat ttg act gat aac tgg 728
Asn Gly His Ser Val Phe Cys Phe Ile Ser His Leu Thr Asp Asn Trp
190 195 200

att ctc tcc atg gaa cag aat cga ggt aca acc agc atc ctg cct tcc 776
Ile Leu Ser Met Glu Gln Asn Arg Gly Thr Thr Ser Ile Leu Pro Ser
205 210 215 220

ttg ctg agc att ctc tat gtg aaa ctg gct gta act gtt ctc atc gta 824
Leu Leu Ser Ile Leu Tyr Val Lys Leu Ala Val Thr Val Leu Ile Val
225 230 235

gga ttt gct ttt ttc cag aag aga aat tat ttc aga gtg cca gaa ggc    872
Gly Phe Ala Phe Phe Gln Lys Arg Asn Tyr Phe Arg Val Pro Glu Gly
            240                 245                 250

tcc tgaggagagt ggtctgtggt taagatgaga tttaccacca tctgaaagac        925
Ser

atcttgtcta ccgcgcagcg tgctgagatt ccgagaagca gccacagaac ctactaggaa 985

gacaaatctg atgtggttgt caatcctttc aatggacctg agtacttcta taaacccgag 1045

tgaggttgtg ctggacccag gagccaggct aggtcatata tgttgatttt tgctgcaaga 1105

cctcatggtt tatctacaaa tcctaaattc tttcacttcc agttttaaaa cttttggccc 1165

aagcatttta tccacagcat aacaccttta aagaaactct cccacggaaa ctgctggttc 1225

catggaatgg aaaattgcaa catggtttac aagacagtgc aaaccaagca gcattccaag 1285

atatgagctt cagaaagtta caggaactgt cttgggacga gaaagaagga ttaaatagtt 1345

cccagtccc                                                         1354

<210> 12
<211> 278
<212> PRT
<213> Unknown

<400> 12
Met His Ala Leu Gly Arg Thr Leu Ala Leu Met Leu Leu Ile Phe Ile
-25                 -20                 -15                 -10

Thr Ile Leu Val Pro Glu Ser Ser Cys Ser Val Lys Gly Arg Glu Glu
                -5                  -1  1               5

Ile Pro Pro Asp Asp Ser Phe Pro Phe Ser Asp Asp Asn Ile Phe Pro
        10                  15                  20

Asp Gly Val Gly Val Thr Met Glu Ile Glu Ile Ile Thr Pro Val Ser
    25                  30                  35

Val Gln Ile Gly Ile Lys Ala Gln Leu Phe Cys His Pro Ser Pro Ser
40                  45                  50                  55

Lys Glu Ala Thr Leu Arg Ile Trp Glu Ile Thr Pro Arg Asp Trp Pro
                60                  65                  70

Ser Cys Arg Leu Pro Tyr Arg Ala Glu Leu Gln Gln Ile Ser Lys Lys
            75                  80                  85

Ile Cys Thr Glu Arg Gly Thr Thr Arg Val Pro Ala His His Gln Ser
        90                  95                  100

Ser Asp Leu Pro Ile Lys Ser Met Ala Leu Lys His Asp Gly His Tyr
    105                 110                 115

Ser Cys Arg Ile Glu Thr Thr Asp Gly Ile Phe Gln Glu Arg His Ser
120                 125                 130                 135

Ile Gln Val Pro Gly Glu Asn Arg Thr Val Val Cys Glu Ala Ile Ala
                140                 145                 150

Ser Lys Pro Ala Met Gln Ile Leu Trp Thr Pro Asp Glu Asp Cys Val
            155                 160                 165

Thr Lys Ser Lys Ser His Asn Asp Thr Met Ile Val Arg Ser Lys Cys
        170                 175                 180

His Arg Glu Lys Asn Asn Gly His Ser Val Phe Cys Phe Ile Ser His
    185                 190                 195

Leu Thr Asp Asn Trp Ile Leu Ser Met Glu Gln Asn Arg Gly Thr Thr
200                 205                 210                 215

Ser Ile Leu Pro Ser Leu Leu Ser Ile Leu Tyr Val Lys Leu Ala Val
                220                 225                 230

Thr Val Leu Ile Val Gly Phe Ala Phe Phe Gln Lys Arg Asn Tyr Phe
            235                 240                 245

Arg Val Pro Glu Gly Ser
        250

<210> 13
<211> 981
<212> DNA
<213> reverse translation

<220>
<221> misc_feature
<222> (1)..(981)
<223> n may be a, c, g, or t

<400> 13
atgytntgyt tytggmgnac nwsncaygtn gcngtnytny tnathtgggg ngtnttygcn 60

gcngarwsnw sntgyccnga yaaraaycar acnatgcara ayaaywsnws nacnatgacn 120

gargtnaaya cnacngtntt ygtncaratg ggnaaraarg cnytnytntg ytgyccnwsn 180

athwsnytna cnaargtnat hytnathacn tggacnatha cnytnmgngg ncarccnwsn 240

tgyathathw sntayaargc ngayacnmgn garacncayg arwsnaaytg ywsngaymgn 300

wsnathacnt gggcnwsnac nccngayytn gcnccngayy tncarathws ngcngtngcn 360

ytncarcayg arggnmgnta ywsntgygay athgcngtnc cngayggnaa yttycaraay 420

athtaygayy tncargtnyt ngtnccnccn gargtnacnc ayttyccngg ngaraaymgn 480

acngcngtnt gygargcnat hgcnggnaar ccngcngcnc arathwsntg gacnccngay 540

ggngaytgyg tngcnaaraa ygarwsncay wsnaayggna cngtnacngt nmgnwsnacn 600

tgycaytggg arcarwsnca ygtnwsngtn gtnttytgyg tngtnwsnca yytnacnacn 660

ggnaaycarw snytnwsnat hgarytnggn mgnggnggng aycarytnyt nggnwsntay 720

athcartaya thathccnws nathathath ytnathatha thggntgyat htgyytnytn 780

aarathwsng gntgymgnaa rtgyaarytn ccnaarwsng gngcnacncc ngayathgar 840

gargaygara tgcarccnta ygcnwsntay acngaraarw snaayccnyt ntaygayacn 900

gtnacnacna cngargcnca yccngcnwsn carggnaarg tnaayggnac ngaytgyytn 960

acnytnwsng cnatgggnat h 981

<210> 14
<211> 885
<212> DNA
<213> reverse translation

<220>
<221> misc_feature
<222> (1)..(885)
<223> n may be a, c, g, or t

<400> 14
atgytntgyc cntggmgnac ngcnaayytn ggnytnytny tnathytnac nathttyytn 60

gtngcngarg cngarggngc ngcncarccn aayaaywsny tnatgytnca racnwsnaar 120

garaaycayg cnytngcnws nwsnwsnytn tgyatggayg araarcarat hacncaraay 180

taywsnaarg tnytngcnga rgtnaayacn wsntggccng tnaaratggc nacnaaygcn 240

gtnytntgyt gyccnccnat hgcnytnmgn aayytnatha thathacntg ggarathath 300

ytnmgnggnc arccnwsntg yacnaargcn tayaaraarg aracnaayga racnaargar 360

acnaaytgya cngaygarmg nathacntgg gtnwsnmgnc cngaycaraa ywsngayytn 420

carathmgna cngtngcnat hacncaygay ggntaytaym gntgyathat ggtnacnccn 480

gayggnaayt tycaymgngg ntaycayytn cargtnytng tnacnccnga rgtnacnytn 540

ttycaraaym gnaaymgnac ngcngtntgy aargcngtng cnggnaarcc ngcngcncay 600

athwsntgga thccngargg ngaytgygcn acnaarcarg artaytggws naayggnacn 660

gtnacngtna arwsnacntg ycaytgggar gtncayaayg tnwsnacngt nacntgycay 720

gtnwsncayy tnacnggnaa yaarwsnytn tayathgary tnytnccngt nccnggngcn 780

aaraarathw snaarathat htaywsnath taycayccnt aytaytayta yytngaycay 840

mgnggnathc ayytngtngt ngarwsncar tggytncara arath 885

<210> 15
<211> 978
<212> DNA
<213> reverse translation

<220>
<221> misc_feature
<222> (1)..(978)
<223> n may be a, c, g, or t

<400> 15
atgttytgyt tytggmgnac nwsngcnytn gcngtnytny tnathtgggg ngtnttygtn 60

gcnggnwsnw sntgyacnga yaaraaycar acnacncara ayaaywsnws nwsnccnytn 120

acncargtna ayacnacngt nwsngtncar athggnacna argcnytnyt ntgytgytty 180

wsnathccny tnacnaargc ngtnytnath acntggatha thaarytnmg nggnytnccn 240

wsntgyacna thgcntayaa rgtngayacn aaracnaayg aracnwsntg yytnggnmgn 300

aayathacnt gggcnwsnac nccngaycay wsnccngary tncarathws ngcngtnacn 360

ytncarcayg arggnacnta yacntgygar acngtnacnc cngarggnaa yttygaraar 420

aaytaygayy tncargtnyt ngtnccnccn gargtnacnt ayttyccnga raaraaymgn 480

wsngcngtnt gygargcnat ggcnggnaar ccngcngcnc arathwsntg gwsnccngay 540

ggngaytgyg tnacnacnws ngarwsncay wsnaayggna cngtnacngt nmgnwsnacn 600

tgycaytggg arcaraayaa ygtnwsngay gtnwsntgya thgtnwsnca yytnacnggn 660

aaycarwsny tnwsnathga rytnwsnmgn ggnggnaayc arwsnytnmg nccntayath 720

ccntayatha thccnwsnat hathathytn athathathg gntgyathtg yytnytnaar 780

athwsnggnt tymgnaartg yaarytnccn aarytngarg cnacnwsngc nathgargar 840

gaygaratgc arccntaygc nwsntayacn garaarwsna ayccnytnta ygayacngtn 900

acnaargtng argcnttycc ngtnwsncar ggngargtna ayggnacnga ytgyytnacn 960

ytnwsngcna thggnath 978

<210> 16
<211> 750
<212> DNA
<213> reverse translation

<220>
<221> misc_feature
<222> (1)..(750)
<223> n may be a, c, g, or t

<400> 16
atgggnggna arcaratgac ncaraaytay wsnacnatht tygcngargg naayathwsn 60

carccngtny tnatggayat haaygcngtn ytntgytgyc cnccnathgc nytnmgnaay 120

ytnathatha thacntggga rathathytn mgnggncarc cnwsntgyac naargcntay 180

aaraargara cnaaygarac naargaracn aaytgyacng tngarmgnat hacntgggtn 240

wsnmgnccng aycaraayws ngayytncar athmgnccng tngayacnac ncaygayggn 300

taytaymgng gnathgtngt nacnccngay ggnaayttyc aymgnggnta ycayytncar 360

gtnytngtna cnccngargt naayytntty carwsnmgna ayathacngc ngtntgyaar 420

gcngtnacng gnaarccngc ngcncarath wsntggathc cngarggnws nathytngcn 480

acnaarcarg artaytgggg naayggnacn gtnacngtna arwsnacntg yccntgggar 540

ggncayaarw snacngtnac ntgycaygtn wsncayytna cnggnaayaa rwsnytnwsn 600

gtnaarytna aywsnggnyt nmgnacnwsn ggnwsnccng cnytnwsnyt nytnathath 660

ytntaygtna arytnwsnyt nttygtngtn athytngtna cnacnggntt ygtnttytty 720

carmgnatha aycaygtnmg naargtnytn 750

<210> 17
<211> 582
<212> DNA
<213> reverse translation

<220>
<221> misc_feature
<222> (1)..(582)
<223> n may be a, c, g, or t

<400> 17
mgnggncarc cnwsntgyat hatggcntay aargtngara cnaargarac naaygaracn 60

tgyytnggnm gnaayathac ntgggcnwsn acnccngayc ayathccnga yytncarath 120

wsngcngtng cnytncarca ygarggnaay tayytntgyg arathacnac nccngarggn 180

aayttycaya argtntayga yytncargtn ytngtnccnc cngargtnac ntayttyytn 240

ggngaraaym gnacngcngt ntgygargcn atggcnggna arccngcngc ncarathwsn 300

tggacnccng ayggngaytg ygtnacnaar wsngarwsnc aywsnaaygg nacngtnacn 360

gtnmgnwsna cntgycaytg ggarcaraay aaygtnwsng cngtnwsntg yathgtnwsn 420

caywsnacng gnaaycarws nytnwsnath garytnwsnm gnggnacnac nwsnacnacn 480

ccnwsnytny tnacnathyt ntaygtnaar atggtnytny tnggnathat hytnytnaar 540

gtnggnttyg cnttyttyca raarmgnaay gtnacnmgna cn                    582

<210> 18
<211> 834
<212> DNA
<213> reverse translation

<220>
<221> misc_feature
<222> (1)..(834)
<223> n may be a, c, g, or t

<400> 18
atgcaygcny tnggnmgnac nytngcnytn atgytnytna thttyathac nathytngtn 60

ccngarwsnw sntgywsngt naarggnmgn gargarathc cnccngayga ywsnttyccn 120

ttywsngayg ayaayathtt yccngayggn gtnggngtna cnatggarat hgarathath 180

acnccngtnw sngtncarat hggnathaar gcncarytnt tytgycaycc nwsnccnwsn 240

aargargcna cnytnmgnat htgggarath acnccnmgng aytggccnws ntgymgnytn 300

ccntaymgng cngarytnca rcarathwsn aaraaratht gyacngarmg nggnacnacn 360

mgngtnccng cncaycayca rwsnwsngay ytnccnatha arwsnatggc nytnaarcay 420

gayggncayt aywsntgymg nathgaracn acngayggna thttycarga rmgncaywsn 480

athcargtnc cnggngaraa ymgnacngtn gtntgygarg cnathgcnws naarccngcn 540

atgcarathy tntggacncc ngaygargay tgygtnacna arwsnaarws ncayaaygay 600

acnatgathg tnmgnwsnaa rtgycaymgn garaaraaya ayggncayws ngtnttytgy 660

ttyathwsnc ayytnacnga yaaytggath ytnwsnatgg arcaraaymg nggnacnacn 720

wsnathytnc cnwsnytnyt nwsnathytn taygtnaary tngcngtnac ngtnytnath 780

gtnggnttyg cnttyttyca raarmgnaay tayttymgng tnccngargg nwsn       834

<210> 19
<211> 1047
<212> DNA
<213> Unknown

<220>
<223> Description of Unknown Organism:primate; surmised
      homo sapiens

<220>
<221> CDS
<222> (1)..(1044)

<220>
<221> mat_peptide
<222> (79)..(1044)

<400> 19
atg ctc tgc cct tgg aga act gct aac cta ggg cta ctg ttg att ttg      48
act atc ttc tta gtg gcc gaa gcg gag ggt gct gct caa cca aac aac      96
tca tta atg ctg caa act agc aag gag aat cat gct tta gct tca agc     144
agt tta tgt atg gat gaa aaa cag att aca cag aac tac tcg aaa gta     192
ctc gca gaa gtt aac act tca tgg cct gta aag atg gct aca aat gct     240
gtg ctt tgt tgc cct cct atc gca tta aga aat ttg atc ata ata aca     288
tgg gaa ata atc ctg aga ggc cag cct tcc tgc aca aaa gcc tac agg     336
aaa gaa aca aat gag acc aag gaa acc aac tgt act gat gag aga ata     384
acc tgg gtc tcc aga cct gat cag aat tcg gac ctt cag att cgt cca     432
gtg gcc atc act cat gac ggg tat tac aga tgc ata atg gta aca cct     480
gat ggg aat ttc cat cgt gga tat cac ctc caa gtg tta gtt aca cct     528
gaa gtg acc ctg ttt caa aac agg aat aga act gca gta tgc aag gca     576
gtt gca ggg aag cca gct gcg cag atc tcc tgg atc cca gag ggc gat     624
tgt gcc act aag caa gaa tac tgg agc aat ggc aca gtg act gtt aag     672
agt aca tgc cac tgg gag gtc cac aat gtg tct acc gtg acc tgc cac     720
gtc tcc cat ttg act ggc aac aag agt ctg tac ata gag cta ctt cct     768
gtt cca ggt gcc aaa aaa tca gca aaa tta tat att cca tat atc atc     816
ctt act att att att ttg acc atc gtg gga ttc att tgg ttg ttg aaa     864
gtc aat ggc tgc aga aaa tat aaa ttg aat aaa aca gaa tct act cca     912
gtt gtt gag gag gat gaa atg cag ccc tat gcc agc tac aca gag aag     960
aac aat cct ctc tat gat act aca aac aag gtg aag gca tct cag gca     1008
tta caa agt gaa gtt gac aca gac ctc cat act tta taa                 1047


<210> 20
<211> 348
<212> PRT
<213> Unknown

<400> 20

<210> 21
<211> 1044
<212> DNA
<213> reverse translation

<220>
<221> misc_feature
<222> (1)..(1044)
<223> n may be a, c, g, or t

<400> 21
atgytntgyc cntggmgnac ngcnaayytn ggnytnytny tnathytnac nathttyytn 60

gtngcngarg cngarggngc ngcncarccn aayaaywsny tnatgytnca racnwsnaar 120

garaaycayg cnytngcnws nwsnwsnytn tgyatggayg araarcarat hacncaraay 180

taywsnaarg tnytngcnga rgtnaayacn wsntggccng tnaaratggc nacnaaygcn 240

gtnytntgyt gyccnccnat hgcnytnmgn aayytnatha thathacntg ggarathath 300

ytnmgnggnc arccnwsntg yacnaargcn taymgnaarg aracnaayga racnaargar 360

acnaaytgya cngaygarmg nathacntgg gtnwsnmgnc cngaycaraa ywsngayytn 420

carathmgnc cngtngcnat hacncaygay ggntaytaym gntgyathat ggtnacnccn 480

gayggnaayt tycaymgngg ntaycayytn cargtnytng tnacnccnga rgtnacnytn 540

ttycaraaym gnaaymgnac ngcngtntgy aargcngtng cnggnaarcc ngcngcncar 600

athwsntgga thccngargg ngaytgygcn acnaarcarg artaytggws naayggnacn 660

gtnacngtna arwsnacntg ycaytgggar gtncayaayg tnwsnacngt nacntgycay 720

gtnwsncayy tnacnggnaa yaarwsnytn tayathgary tnytnccngt nccnggngcn 780

aaraarwsng cnaarytnta yathccntay athathytna cnathathat hytnacnath 840

gtnggnttya thtggytnyt naargtnaay ggntgymgna artayaaryt naayaaracn 900

garwsnacnc cngtngtnga rgargaygar atgcarccnt aygcnwsnta yacngaraar 960

aayaayccny tntaygayac nacnaayaar gtnaargcnw sncargcnyt ncarwsngar 1020

gtngayacng ayytncayac nytn 1044

<210> 22
<211> 813
<212> DNA
<213> Unknown

<220>
<223> Description of Unknown Organism:rodent; surmised
mus musculus

<220>
<221> CDS
<222> (1)..(810)

<220>
<221> mat_peptide
<222> (76)..(810)

<400> 22
atg cat gct ctg ggg agg att ccg act ttg act ttg ctg atc ttc atc     48
Met His Ala Leu Gly Arg Ile Pro Thr Leu Thr Leu Leu Ile Phe Ile
-25                 -20                 -15                 -10

aat att ttt gtg tct ggg tca agt tgt act gat gag aat caa aca ata     96
Asn Ile Phe Val Ser Gly Ser Ser Cys Thr Asp Glu Asn Gln Thr Ile
                -5              -1  1               5

cag aat gac agt tca tct tct ctg aca caa gtt aac act aca atg tct     144
Gln Asn Asp Ser Ser Ser Ser Leu Thr Gln Val Asn Thr Thr Met Ser
        10                  15                  20

gta cag atg gat aaa aag gct ctg ctc tgc tgc ttt tct agt cca ctg     192
Val Gln Met Asp Lys Lys Ala Leu Leu Cys Cys Phe Ser Ser Pro Leu
    25                  30                  35

ata aat gca gta tta atc aca tgg ata ata aaa cac aga cac ctg cct     240
Ile Asn Ala Val Leu Ile Thr Trp Ile Ile Lys His Arg His Leu Pro
40                  45                  50                  55

tcc tgc aca ata gca tac aac cta gat aaa aag acc aat gaa acc agc     288
Ser Cys Thr Ile Ala Tyr Asn Leu Asp Lys Lys Thr Asn Glu Thr Ser
                60                  65                  70

tgc ttg ggc agg aac atc acc tgg gcc tcc aca cct gac cac agt cct     336
Cys Leu Gly Arg Asn Ile Thr Trp Ala Ser Thr Pro Asp His Ser Pro
            75                  80                  85

gaa ctt cag atc agt gca gtg gcc ctc cag cat gag ggg act tac aca     384
Glu Leu Gln Ile Ser Ala Val Ala Leu Gln His Glu Gly Thr Tyr Thr
        90                  95                  100

tgt gag ata gta aca cct gaa ggg aat tta gaa aaa gtc tat gac ctc     432
Cys Glu Ile Val Thr Pro Glu Gly Asn Leu Glu Lys Val Tyr Asp Leu
    105                 110                 115

caa gtg ctg gtg ccc cct gag gta acc tac ttt cca ggg aaa aac aga     480
Gln Val Leu Val Pro Pro Glu Val Thr Tyr Phe Pro Gly Lys Asn Arg
120                 125                 130                 135

act gca gtc tgt gag gca atg gca ggc aag cct gct gca cag atc tct     528
Thr Ala Val Cys Glu Ala Met Ala Gly Lys Pro Ala Ala Gln Ile Ser
                140                 145                 150

tgg act cca gat ggg gac tgt gtc act aag agt gag tca cac agc aat     576
Trp Thr Pro Asp Gly Asp Cys Val Thr Lys Ser Glu Ser His Ser Asn
            155                 160                 165

ggc act gtg act gtc agg agc acg tgc cac tgg gag cag aac aat gtg     624
Gly Thr Val Thr Val Arg Ser Thr Cys His Trp Glu Gln Asn Asn Val
        170                 175                 180

tct gtt gtg tcc tgc tta gtc tct cat tcg act ggt aat cag tct ctg 672
Ser Val Val Ser Cys Leu Val Ser His Ser Thr Gly Asn Gln Ser Leu
185 190 195

tcc ata gaa ctg agt caa ggt aca atg acc acc ccc cgt tcc ttg ctg 720
Ser Ile Glu Leu Ser Gln Gly Thr Met Thr Thr Pro Arg Ser Leu Leu
200 205 210 215

acc att ctc tat gtg aaa atg gcc ctt ttg gtg att att ctt ctt aac 768
Thr Ile Leu Tyr Val Lys Met Ala Leu Leu Val Ile Ile Leu Leu Asn
220 225 230

gta gga ttt gct ttc ttc cag aag aga aat ttt gcc aga aca tga 813
Val Gly Phe Ala Phe Phe Gln Lys Arg Asn Phe Ala Arg Thr
235 240 245

<210> 23
<211> 270
<212> PRT
<213> Unknown

<400> 23
Met His Ala Leu Gly Arg Ile Pro Thr Leu Thr Leu Leu Ile Phe Ile
-25 -20 -15 -10

Asn Ile Phe Val Ser Gly Ser Ser Cys Thr Asp Glu Asn Gln Thr Ile
-5 -1 1 5

Gln Asn Asp Ser Ser Ser Ser Leu Thr Gln Val Asn Thr Thr Met Ser
10 15 20

Val Gln Met Asp Lys Lys Ala Leu Leu Cys Cys Phe Ser Ser Pro Leu
25 30 35

Ile Asn Ala Val Leu Ile Thr Trp Ile Ile Lys His Arg His Leu Pro
40 45 50 55

Ser Cys Thr Ile Ala Tyr Asn Leu Asp Lys Lys Thr Asn Glu Thr Ser
60 65 70

Cys Leu Gly Arg Asn Ile Thr Trp Ala Ser Thr Pro Asp His Ser Pro
75 80 85

Glu Leu Gln Ile Ser Ala Val Ala Leu Gln His Glu Gly Thr Tyr Thr
90 95 100

Cys Glu Ile Val Thr Pro Glu Gly Asn Leu Glu Lys Val Tyr Asp Leu
105 110 115

Gln Val Leu Val Pro Pro Glu Val Thr Tyr Phe Pro Gly Lys Asn Arg
120 125 130 135

Thr Ala Val Cys Glu Ala Met Ala Gly Lys Pro Ala Ala Gln Ile Ser
140 145 150

Trp Thr Pro Asp Gly Asp Cys Val Thr Lys Ser Glu Ser His Ser Asn

155                 160                 165

Gly Thr Val Thr Val Arg Ser Thr Cys His Trp Glu Gln Asn Asn Val
        170                 175                 180

Ser Val Val Ser Cys Leu Val Ser His Ser Thr Gly Asn Gln Ser Leu
    185                 190                 195

Ser Ile Glu Leu Ser Gln Gly Thr Met Thr Thr Pro Arg Ser Leu Leu
200                 205                 210                 215

Thr Ile Leu Tyr Val Lys Met Ala Leu Leu Val Ile Ile Leu Leu Asn
                220                 225                 230

Val Gly Phe Ala Phe Phe Gln Lys Arg Asn Phe Ala Arg Thr
            235                 240                 245

<210> 24
<211> 810
<212> DNA
<213> reverse translation

<220>
<221> misc_feature
<222> (1)..(810)
<223> n may be a, c, g, or t

<400> 24
atgcaygcny tnggnmgnat hccnacnytn acnytnytna thttyathaa yathttygtn 60

wsnggnwsnw sntgyacnga ygaraaycar acnathcara aygaywsnws nwsnwsnytn 120

acncargtna ayacnacnat gwsngtncar atggayaara argcnytnyt ntgytgytty 180

wsnwsnccny tnathaaygc ngtnytnath acntggatha thaarcaymg ncayytnccn 240

wsntgyacna thgcntayaa yytngayaar aaracnaayg aracnwsntg yytnggnmgn 300

aayathacnt gggcnwsnac nccngaycay wsnccngary tncarathws ngcngtngcn 360

ytncarcayg arggnacnta yacntgygar athgtnacnc cngarggnaa yytngaraar 420

gtntaygayy tncargtnyt ngtnccnccn gargtnacnt ayttyccngg naaraaymgn 480

acngcngtnt gygargcnat ggcnggnaar ccngcngcnc arathwsntg gacnccngay 540

ggngaytgyg tnacnaarws ngarwsncay wsnaayggna cngtnacngt nmgnwsnacn 600

tgycaytggg arcaraayaa ygtnwsngtn gtnwsntgyy tngtnwsnca ywsnacnggn 660

aaycarwsny tnwsnathga rytnwsncar ggnacnatga cnacnccnmg nwsnytnytn 720

acnathytnt aygtnaarat ggcnytnytn gtnathathy tnytnaaygt nggnttygcn 780

ttyttycara armgnaaytt ygcnmgnacn                                  810

## Description

### FIELD OF THE INVENTION

The present invention relates to compositions and methods for affecting mammalian physiology, including immune system function. In particular, it provides reagents or methods which may regulate development and/or the immune system. Diagnostic and therapeutic uses of these materials are also described.

### BACKGROUND OF THE INVENTION

The OX2 antigen (OX2) is a cell surface protein identified on a variety of cells including thymocytes, B lymphocytes, activated T lymphocytes, neurons, endothelial cells, and follicular dendritic cells. Barclay (1981) Immunology 44:727-736. Sequence analysis indicates that it is a transmembrane protein containing two extracellular immunoglobulin-like (Ig-like) domains and a short cytoplasmic domain. Clark, et al. (1985) EMBO J. 4:113-118. This domain organization is common and found in many different leukocyte surface proteins. Barclay, et al. (1997) Leucocyte Antigens Factsbook (2d. ed.) Academic Press, London. These types of proteins often interact with other proteins on the surfaces of other cells, also having Ig-like domains.

The distribution of the OX2 antigen is consistent with a hypothesis that OX2 relays a signal through a binding partner, e.g., the OX2 receptor (OX2R), to cells within the leukocyte lineage including macrophages, which express the receptor (Preston, et al. (1997) Eur. J. Immunol. 27:1911-1918) and possibly other cells of the monocyte-macrophage lineage. Also, the OX2 has been implicated in regulation of various functions of macrophages. In this scenario, for instance, expression of OX2 on neurons could establish a direct means of communication to the resident macrophages of the brain called microglia that might express OX2R, since they originate from the monocyte-macrophage lineage. Perry and Gordon (1988) Trends Neurosci. 11:273-277.

Generally, defective or exaggerated activation of macrophages contributes to pathogenesis of a wide range of immunological and other diseases. See, e.g., McGee, et al. (eds. 1992) Oxford Textbook of Pathology Oxford University Press, Oxford; Lewis and McGee (eds. 1992) The Macrophage IRL Press, Oxford; and Bock and Goode (eds. 1997) The Molecular Basis of Cellular Defence Mechanisms Wiley & Sons.

Also, identification of the OX2 interacting proteins, e.g., the OX2R for the OX2 antigen, is difficult because the affinities of the interactions are often very low. This means that the binding of recombinant forms of cell surface proteins, e.g., OX2, to their binding partners, the interacting proteins, e.g., OX2R, is insufficiently stable to allow detection by normal methods. Thus, the interaction between CD48 and CD2, of which both partners contain two Ig-like domains in their extracellular regions, has a half-life of a fraction of a second. See Van der Merwe, et al. (1993) Biochem. Soc. Trans. 21:340S; and Van der Merwe and Barclay (1994) Trends Biochem. Sci. 19:354-358.

Recombinant forms of cell surface proteins such as OX2 can be made multivalent by a number of methods and used to detect novel proteins. An OX2 has been engineered to include a tag of two Ig-like domains from CD4. The recombinant soluble proteins are expressed by conventional expression methods in eukaryotic cells. In an earlier study, an interaction was observed between the multivalent recombinant OX2 protein on fluorescent beads and mouse macrophages. Preston, et al. (1997) Eur. J. Immunol. 27:1911-1918.

Despite the above, attempts to identify OX2R on mouse macrophages through use of a blocking antibody OX89 were not successful. Preston, et al. (1997) Eur. J. Immunol. 27:1911-1918.

From the foregoing, it is evident that the discovery, identification, and understanding of novel receptors for OX2-like molecules would be highly advantageous. The present invention provides new receptor homologs for OX2 ligands and related compounds, and methods for their use.

### SUMMARY OF THE INVENTION

The present invention is directed to novel receptor homologs, for the ligand designated OX2, e.g., rodent and primate embodiments. These have been designated generically OX2 receptor homologs (OX2RH), with embodiments from various rodent and primate species. Two have been established as actually binding to the respective species OX2. In particular, it provides description of homologs designated OX2RH1, OX2RH2, OX2RH3, and OX2RH4. It includes nucleic acids encoding the polypeptides themselves and methods for their production and use. The nucleic acids of the invention are characterized, in part, by their homology to cloned complementary DNA (cDNA) sequences enclosed herein.

The present inventors have produced a new monoclonal antibody (mAb), designated OX102, for OX2R on rat macrophages which blocks the interaction between OX2 and OX2R. They have also isolated and characterised the rat OX2R gene and polypeptide. Sequences for the rat OX2R nucleic acid molecule and polypeptide (predicted amino acid sequence) are provided herein. By analogy with similar proteins, the inventors teach that the nucleotide and amino acid sequences of human OX2R will be at least 50% homologous with the corresponding rat OX2R sequences. The availability of the rat OX2R cDNA and a predicted OX2R polypeptide sequence enable identification of the equivalent human OX2R sequences, either through screening of known human sequences or the isolation of human nucleic acids by hybridisation or PCR technology.

The presence of a large cytoplasmic sequence in the OX2R polypeptide indicates that OX2R has a role in macrophage function, either in signalling or through interactions with components of the cytoplasm. Thus, the present invention provides for reagents based on OX2R that either mimic or recognise OX2R polypeptide or nucleic acid sequences, e.g., small molecular entities designed to react with OX2R binding sites, mAbs raised against OX2R or antisense sequences, which reagents constitute therapeutically useful compounds for modifying the function of cells carrying OX2 and/or OX2R cell surface proteins (e.g., the function of cells such as macrophages, activated lymphocytes, neurons, endothelial cells, dendritic cells, thymocytes and B lymphocytes), either by enhancing or inhibiting cell activity. Thus, reagents based on OX2R that either mimic or recognise OX2R polypeptide or nucleic acid sequences have potential applications for controlling the wide range of functions of macrophages, including responses to bacterial infections, autoimmune diseases, etc.

Since the extracellular domain of OX2R is believed to be responsible for interacting with OX2, the present inventors provide a means of screening candidate compounds for an ability to affect (positively or negatively) binding between OX2 and OX2R. Thus OX2R as provided by the present invention can, e.g., be used to detect compounds which inhibit the interaction between OX2 and OX2R, and hence which are likely to affect the interaction between macrophages and other cells of the immune system, such as lymphocytes or follicular dendritic cells.

The nucleic acid and amino acid sequences for rat OX2R are shown in Table 1. Various aspects of the invention are stated below. Other aspects are clear from the detailed description.

Hence, in a first aspect, the present invention provides a substance comprising a polypeptide having the amino acid sequence set out in Table 1.

In a further aspect, the present invention provides a substance comprising a polypeptide having at least 50% amino acid sequence identity with the amino acid sequence set out in Table 1.

In a further aspect, the present invention provides a polypeptide which is a mutant, variant, derivative or allele of an above polypeptide and which has a characteristic property of full-length OX2R, e.g., an ability to bind with an OX2 or with an antibody for full-length OX2R.

In a further aspect, the present invention provides a substance which is a fragment of an above polypeptide (e.g., a fragment of a polypeptide having the amino acid sequence set out in Table 1), which fragment exhibits a characteristic property of full-length OX2R protein. For example, the fragment may bind with an OX2 protein or with an antibody for full-length OX2R protein. In one embodiment, the fragment includes part or all of the cytoplasmic domain of OX2R or an active portion of that domain. In another embodiment, the fragment includes part or all of the extracellular domain of OX2R or an active portion of that domain. Since the extracellular domain is believed to be responsible for interacting with OX2, such fragments according to the present invention including part or all of the extracellular domain, can be used to screen candidate compounds for an ability to interfere with the binding between OX2 and OX2R.

Accordingly, the present invention provides methods and materials for screening candidate compounds likely to have the ability to interfere with the OX2/OX2R interaction between macrophages and other cells, including thymocytes, B lymphocytes, activated T lymphocytes, neurons, endothelial cells and follicular dendritic cells.

Polypeptides and fragments as above may be recombinant and/or isolated polypeptides.

In a further aspect, the present invention provides a substance comprising a nucleic acid having the nucleotide sequence of Table 1. The present invention also provides a substance which comprises a nucleic acid molecule encoding an above polypeptide or fragment. Thus, Table 1 shows the cDNA sequence of an exemplary nucleic acid molecule coding for an OX2R polypeptide. The nucleic acid molecule may have at least 50% sequence homology with the nucleic acid sequence of Table 1.

The invention also provides a substance comprising a nucleic acid molecule having part of a coding nucleotide sequence of Table 1. Where the substance comprises a part of a coding nucleotide sequence of Table 1, it will be a part which is characteristic of an OX2R gene. Thus, the part may encode a polypeptide fragment as stated above, which binds with OX2 or an antibody for full-length OX2R. Alternatively, the part may comprise at least 4 to 7 contiguous codons, often at least 7 to 9 contiguous codons, typically at least 9 to 13 contiguous codons and, most preferably, at least 20 to 30 contiguous codons of a nucleotide sequence of Table 1. Alternatively, the part may encode at least 4 to 7 contiguous amino acids, often at least 7 to 9 contiguous amino acids, typically at least about 9 to 13 contiguous amino acids and, most preferably, at least about 20 to 30 contiguous amino acids of a polypeptide sequence of Table 1.

Nucleic acid molecules as above may be recombinant and/or isolated.

In further aspects, the present invention provides vectors comprising an OX2R nucleic acid as herein provided, e.g., expression vectors in which an OX2R nucleic acid sequence is operably linked to control sequences to direct its expression. Also provided are host cells transformed with such vectors. The present invention further includes a method of producing OX2R polypeptides, comprising culturing such host cells and isolating OX2R polypeptide produced.

In a further aspect, the present invention provides a method of expressing OX2R in host cells, the method including the steps of inserting a nucleic acid molecule as above into a host cell and providing conditions for expression of said nucleic acid molecule in the host cell. The method may employ an expression vector.

In a further aspect, the present invention provides a composition comprising a soluble form of an OX2R polypeptide or fragment as above, the composition also optionally including an adjuvant, pharmaceutical carrier, or excipient. The composition can be used, e.g., to generate an antibody response to an OX2R polypeptide.

In further aspects, the present invention provides above OX2R polypeptides and nucleic acid molecules for use in screening candidate compounds likely to be useful as therapeutics. The present invention provides the use of an OX2R polypeptide or fragment as above in the screening for substances likely to be useful for the treatment of bacterial infections, autoimmune diseases, and the like.

The present invention also provides the use of OX2R polypeptides, polypeptide fragments, and nucleic acids for the identification of ligands for OX2R other than OX2. The present invention also provides the use of OX2R polypeptides, polypeptide fragments, and nucleic acids for the design of mimetics of OX2.

In a further aspect, the present invention provides antibodies capable of specifically binding to OX2R polypeptides, polypeptide fragments, and nucleic acids as above, and compositions comprising such antibodies. These antibodies can be used in assays to detect and quantify the presence of OX2R, as well as in methods of purifying OX2R. The antibodies may be polyclonal. Preferably, the antibodies are IgG antibodies, more preferably monoclonal IgG antibodies.

In a further aspect, the present invention provides the use of OX2R polypeptides, polypeptide fragments, and nucleic acid molecules as provided herein to produce binding molecules, such as substances with one or more antibody domains, which can block the interaction between OX2 and OX2R. These may be included in a composition likely to be useful in the preparation of medicaments for the treatment of bacterial infections, autoimmune diseases, and the like. Where the binding molecules are antibodies, they may be IgG antibodies, preferably monoclonal IgG antibodies.

In a further aspect, the present invention provides the use of OX2R nucleic acids as defined above in the design of antisense oligonucleotides to restrict OX2R expression in a population of macrophage cells, e.g., phosphorothiolated or cholesterol-linked oligonucleotides which can facilitate internalization and stabilization of the oligonucleotides.

In a further aspect, the present invention provides a method of amplifying a nucleic acid test sample, which comprises priming a nucleic acid polymerase reaction with primer oligonucleotides obtainable from the sequence information provided herein. The nucleic acid test sample may be of a human, such that nucleic acid coding for a human OX2R is amplified using such a method.

In a further aspect, the present invention provides a method of obtaining a nucleic acid molecule coding for part or all of an OX2R from a species other than rat, e.g., human OX2R, which comprises probing a nucleic acid test sample from the species of interest with a nucleic acid probe obtainable from the sequence information provided herein.

In a further aspect, the present invention provides a method of obtaining an OX2R polypeptide sequence from a species other than rat, e.g., a human OX2R, which comprises searching databases for polypeptide sequences at least 50% homologous with an OX2R amino acid sequence as provided herein (Table 1). Similarly, OX2R nucleic acid sequences from species other than rat, e.g., human OX2R, can be obtained by searching databases for nucleotide sequences at least 50% homologous with an OX2R nucleotide sequence as provided herein.

The present invention also provides the use of the nucleic acid sequence information provided herein in the search for mutations in OX2R genes, e.g., using techniques such as single stranded conformation polymorphism (SSCP).

The present invention provides a composition of matter selected from: a substantially pure or recombinant polypeptide comprising at least three distinct nonoverlapping segments of at least four amino acids identical to segments of SEQ ID NO: 2; a substantially pure or recombinant polypeptide comprising at least two distinct nonoverlapping segments of at least five amino acids identical to segments of SEQ ID NO: 2; a natural sequence rodent OX2RH1 polypeptide comprising mature SEQ ID NO: 2; a fusion polypeptide comprising rat OX2RH1 sequence; a substantially pure or recombinant polypeptide comprising at least three distinct nonoverlapping segments of at least four amino acids identical to segments of SEQ ID NO: 4; a substantially pure or recombinant polypeptide comprising at least two distinct nonoverlapping segments of at least five amino acids identical to segments of SEQ ID NO: 4; a natural sequence rodent OX2RH1 polypeptide comprising mature SEQ ID NO: 4; a fusion polypeptide comprising human OX2RH1 sequence; a substantially pure or recombinant polypeptide comprising at least three distinct nonoverlapping segments of at least four amino acids identical to segments of SEQ ID NO: 6; a substantially pure or recombinant polypeptide comprising at least two distinct nonoverlapping segments of at least five amino acids identical to segments of SEQ ID NO: 6; a natural sequence rodent OX2RH1 polypeptide comprising mature SEQ ID NO: 6; a fusion polypeptide comprising mouse OX2RH1 sequence; a substantially pure or recombinant polypeptide comprising at least three distinct nonoverlapping segments of at least four amino acids identical to segments of SEQ ID NO: 8; a substantially pure or recombinant polypeptide comprising at least two distinct nonoverlapping segments of at least five amino acids identical to segments of SEQ ID NO: 8; a natural sequence rodent OX2RH1 polypeptide comprising mature SEQ ID NO: 8; a fusion polypeptide comprising human OX2RH2 sequence; a substantially pure or recombinant polypeptide comprising at least three distinct nonoverlapping segments of at least four amino acids identical to segments of SEQ ID NO: 10; a substantially pure or recombinant polypeptide comprising at least two distinct nonoverlapping segments of at least five amino acids identical to segments of SEQ ID NO: 10; a natural sequence rodent OX2RH2 polypeptide comprising mature SEQ ID NO: 10; a fusion polypeptide comprising mouse OX2RH2 sequence; a substantially pure or recombinant polypeptide comprising at least three distinct nonoverlapping segments of at least four amino acids identical to segments of SEQ ID NO: 12; a substantially pure or recombinant polypeptide comprising at least two distinct nonoverlapping segments of at least five amino acids identical to segments of SEQ ID NO: 12; a natural sequence rodent OX2RH3 comprising mature SEQ ID NO: 12; a fusion polypeptide comprising mouse OX2RH3 sequence; a substantially pure or recombinant polypeptide comprising at least three distinct nonoverlapping segments of at least four amino acids identical to segments of SEQ ID NO: 20; a substantially pure or recombinant polypeptide comprising at least two distinct nonoverlapping segments of at least five amino acids identical to segments of SEQ ID NO: 20; a natural sequence primate OX2RH1.2 polypeptide comprising mature SEQ ID NO: 20; a fusion polypeptide comprising primate OX2RH1.2 sequence; a substantially pure or recombinant polypeptide comprising at least three distinct nonoverlapping segments of at least four amino acids identical to segments of SEQ ID NO: 23; a substantially pure or recombinant polypeptide comprising at least two distinct nonoverlapping segments of at least five amino acids identical to segments of SEQ ID NO: 23; a natural sequence rodent OX2RH4 polypeptide comprising mature SEQ ID NO: 23; or a fusion polypeptide comprising mouse OX2RH4 sequence. Some preferred embodiments include wherein the distinct nonoverlapping segments of identity: include one of at least eight amino acids; include one of at least four amino acids and a second of at least five amino acids; include at least three segments of at least four, five, and six amino acids, or include one of at least twelve amino acids. Other preferred embodiment include those wherein the: a) OX2RH1 polypeptide: comprises a mature sequence of Tables 1 or 2; is an unglycosylated form of OX2RH polypeptide; is from a primate, such as a human; is from a rodent, such as a rat or mouse; comprises at least seventeen amino acids of SEQ ID NO: 2, 4, 6, or 20; exhibits at least four nonoverlapping segments of at least seven amino acids of SEQ ID NO: 2, 4, 6, or 20; is a natural allelic variant of OX2RH1; has a length at least about 30 amino acids; exhibits at least two non-overlapping epitopes which are specific for a primate or rodent OX2RH1; is glycosylated; has a molecular weight of at least 30 kD with natural glycosylation; is a synthetic polypeptide; is attached to a solid substrate; is conjugated to another chemical moiety; is 5-fold or less substituted from natural sequence; or is a deletion or insertion variant from a natural sequence; b) OX2RH2 polypeptide: comprises a mature sequence of Table 2; is an unglycosylated form of OX2RH2 polypeptide; is from a primate, such as a human; is from a rodent, such as a mouse; comprises at least seventeen amino acids of SEQ ID NO: 8 or 10; exhibits at least four nonoverlapping segments of at least seven amino acids of SEQ ID NO: 8 or 10; is a natural allelic variant of OX2RH2; has a length at least about 30 amino acids; exhibits at least two non-overlapping epitopes which are specific for a primate or rodent OX2RH2; is glycosylated; has a molecular weight of at least 30 kD with natural glycosylation; is a synthetic polypeptide; is attached to a solid substrate; is conjugated to another chemical moiety; is a 5-fold or less substitution from natural sequence; or is a deletion or insertion variant from a natural sequence; c) OX2RH3 polypeptide: comprises a mature sequence of Table 3; is an unglycosylated form of OX2RH3; is from a rodent, such as a mouse; comprises at least seventeen amino acids of SEQ ID NO: 12; exhibits at least four nonoverlapping segments of at least seven amino acids of SEQ ID NO: 12; is a natural allelic variant of OX2RH3; has a length at least about 30 amino acids; exhibits at least two non-overlapping epitopes which are specific for a rodent OX2PH3; is glycosylated; has a molecular weight of at least 30 kD with natural glycosylation; is a synthetic polypeptide; is attached to a solid substrate; is conjugated to another chemical moiety; is 5-fold or less substituted from natural sequence; or is a deletion or insertion variant from a natural sequence; or d) OX2RH4 polypeptide: comprises a mature sequence of Table 2; is an unglycosylated form of OX2RH4; is from a rodent, such as a mouse; comprises at least seventeen amino acids of SEQ ID NO: 23; exhibits at least four nonoverlapping segments of at least seven amino acids of SEQ ID NO: 23; is a natural allelic variant of OX2RH4; has a length at least about 30 amino acids; exhibits at least two non-overlapping epitopes which are specific for a rodent OX2RH4; is glycosylated; has a molecular weight of at least 30 kD with natural glycosylation; is a synthetic polypeptide; is attached to a solid substrate; is conjugated to another chemical moiety; is 5-fold or less substituted from natural sequence; or is a deletion or insertion variant from a natural sequence. In yet other embodiments, the invention provides a composition comprising: a1) a substantially pure OX2RH1 and another Ig superfamily member; a2) a substantially pure OX2RH2 and: another Ig superfamily member, DAP12, or DAP10; a3) a substantially pure OX2RH3 and: another Ig superfamily member, DAP12, or DAP10; a4) a substantially pure OX2RH4 and: another Ig superfamily member, DAP12, or DAP10; or a sterile OX2RH1 polypeptide; a sterile OX2RH2 polypeptide; a sterile OX2RH3 polypeptide; a sterile OX2RH4 polypeptide; the OX2RH1, OX2RH2, OX2RH3, or OX2RH4 polypeptide and a carrier, wherein the carrier is an aqueous compound, including water, saline, and/or buffer; and/or formulated for oral, rectal, nasal, topical, or parenteral administration.

Fusion polypeptides are also provided, e.g., comprising: mature protein sequence of Tables 1-3; a detection or purification tag, including a FLAG, His6, or Ig sequence; or sequence of another Ig superfamily protein. Kits are also provided, e.g., comprising an OX2RH polypeptide and: a compartment comprising the protein or polypeptide; or instructions for use or disposal of reagents in the kit.

The invention also embraces various antibody like reagents, including antibodies derived from different species. It provides, e.g., a binding compound comprising an antigen binding site from an antibody, which specifically binds to a natural OX2RH polypeptide, e.g., OX2RH1, OX2RH2, OX2RH3, and/or OX2RH4, wherein: the binding compound is in a container; the OX2RH polypeptide is from a rodent or primate; the binding compound is an Fv, Fab, or Fab2 fragment; the binding compound is conjugated to another chemical moiety; or the antibody: is raised against a peptide sequence of a mature polypeptide of Tables 1-3; is raised against a mature OX2RH; is raised to a purified mammalian OX2RH; is immunoselected; is a polyclonal antibody; binds to a denatured OX2RH; exhibits a Kd to antigen of at least 30 µM; is attached to a solid substrate, including a bead or plastic membrane; is in a sterile composition; or is detectably labeled, including a radioactive or fluorescent label. Kits are thereby provided, e.g., comprising such binding compounds and: a compartment comprising the binding compound; or instructions for use or disposal of reagents in the kit. Methods are also provided, e.g., producing an antigen:binding compound or antigen:antibody complex, comprising contacting under appropriate conditions a mammalian OX2RH polypeptide with an antibody, thereby allowing the complex to form. Preferably, in this method: the complex is purified from other cytokine or Ig superfamily receptors; the complex is purified from other antibody; the contacting is with a sample comprising a mammalian OX2; the contacting allows quantitative detection of the antigen; the contacting is with a sample comprising the antibody; or the contacting allows quantitative detection of the antibody. Related compositions are made available, e.g., comprising: a sterile binding compound, or the binding compound and a carrier, wherein the carrier is: an aqueous compound, including water, saline, and/or buffer; and/or formulated for oral, rectal, nasal, topical, or parenteral administration.

The present invention further provides nucleic acids, e.g., an isolated or recombinant nucleic acid encoding a OX2RH polypeptide wherein the: OX2RH is from a mammal; or the nucleic acid: encodes an antigenic peptide sequence of Tables 1-3; encodes a plurality of antigenic peptide sequences of Tables 1-3; exhibits identity over at least thirteen nucleotides to a natural cDNA encoding the segment; is an expression vector; further comprises an origin of replication; is from a natural source; comprises a detectable label; comprises synthetic nucleotide sequence; is less than 6 kb, preferably less than 3 kb; is from a primate or rodent; comprises a natural full length coding sequence; is a hybridization probe for a gene encoding the OX2RH; further encodes DAP12 or DAP10; or is a PCR primer, PCR product, or mutagenesis primer. Cells comprising the recombinant nucleic acid are also provided, e.g., wherein the cell is: a prokaryotic cell; a eukaryotic cell; a bacterial cell; a yeast cell; an insect cell; a mammalian cell; a mouse cell; a primate cell; or a human cell. Kits comprising the nucleic acid are provided, e.g., with a compartment comprising the nucleic acid; with a compartment further comprising a mammalian OX2RH polypeptide; or with instructions for use or disposal of reagents in the kit.

Alternatively, the invention provides a nucleic acid which: hybridizes under wash conditions of 30 minutes at 40° C and less than 2M salt to the coding portion of SEQ ID NO: 1, 3, 5, 7, 9, 11, 19, or 22; or exhibits identity over a stretch of at least about 30 nucleotides to a primate or rodent OX2RH cDNA. Preferably, the wash conditions are at: 50° C and/or 500 mM salt; or 60° C and/or 150 mM salt; the stretch is at least 55 nucleotides or 75 nucleotides; or the nucleic acid further encodes a DAP12 or DAP10 peptide.

Other methods are further embraced, e.g., a method of modulating physiology or development of a cell or tissue culture cells comprising contacting the cell with an agonist or antagonist of a mammalian OX2RH. Often, the cell is transformed with a nucleic acid encoding an OX2RH.

### DETAILED DESCRIPTION OF THE PREFERRED EMBODIMENTS

### OUTLINE

I. General
II. Activities
III. Nucleic acids
   A. encoding fragments, sequence, probes
   B. mutations, chimeras, fusions
   C. making nucleic acids
   D. vectors, cells comprising
IV. Proteins, Peptides
   A. fragments, sequence, immunogens, antigens
   B. muteins
   C. agonists/antagonists, functional equivalents
   D. making proteins
V. Making nucleic acids, proteins
   A. synthetic
   B. recombinant
   C. natural sources
VI. Antibodies
   A. polyclonals
   B. monoclonal
   C. fragments; Kd
   D. anti-idiotypic antibodies
   E. hybridoma cell lines
VII. Kits and Methods to quantify OX2RHs
   A. ELISA
   B. assay mRNA encoding
   C. qualitative/quantitative
   D. kits
VIII. Therapeutic compositions, methods
   A. combination compositions
   B. unit dose
   C. administration
IX. Screening
X. Ligands

### I. General

The present invention provides amino acid sequences and DNA sequences of mammalian, herein primate and rodent, receptor-like subunit molecules, these designated OX2 receptor homologs (OX2RH). These genes have particular defined properties, either or both structural and biological. Various cDNAs encoding these molecules were obtained from mammal, e.g., human and rodent, cDNA sequence libraries. Other mammalian, e.g., primate, rodent, or other, counterparts would also be desired.

The OX2 antigen was first characterized in rat, using a monoclonal antibody (mAb) MRC OX2. See, e.g., McMaster and Williams (1979) Eur. J. Immunol. 9:426-433; Barclay (1981) Immunology 44:727-736; Barclay (1981) Immunology 42:593-600; Bukovsky, et al. (1984) Immunology 52:631-640; and Webb and Barclay (1984) J. Neurochem. 43:1061-1067. Using this antibody in immunohistochemical (IHC) staining of tissue sections or cell suspensions for flow cytometry revealed that the OX2 antigen was expressed by a wide variety of cells, e.g. neurons, vascular endothelium, B cells, activated T cells, follicular dendritic cells, smooth muscle cells and trophoblasts. Furthermore, human OX2 is known to be expressed in normal brain and by B cells. McCaughan, et al. (1987) Immunogenetics 25:329-335. Characterization of the rat protein recognized by MRC OX2 (Clark, et al. (1985) EMBO J. 4:113-118) revealed that OX2 consists of about 248 amino acids comprising two extracellular immunoglobulin (Ig) domains, a transmembrane domain and a short C-terminal cytoplasmic tail. The molecule is glycosylated through 6 N-linked glycosylation sites, three of which are present in the N-terminal V-like Ig domain and the others reside in the membrane proximal C2-like Ig domain. This places OX2 in the Ig superfamily (IgSF), forming a sub-group of small IgSF molecules with molecules like CD2, CD48, CD58, CD80, CD86, CD90, and CD147, which are characterized structurally, e.g., by the existence of the immunoglobulin-like domains corresponding to Ig variable and constant domains, a transmembrane segment, an intracellular domain, and characteristic cysteine and tryptophan residue spacings. See, e.g., Campbell, et al. (1979) Nature 282:341-342. Interestingly, CD90 is also highly expressed by neurons. Williams, et al. (1977) Cold Spring Harb. Symp. Quant. Biol. 41 Pt 1:51-61. Furthermore, it was shown that OX2 was a structural homologue of CD80 and CD86 (Borriello, et al. (1997) J. Immunol. 158:4548-4554) and that the OX2 gene was closely linked to those coding for CD80 and CD86 on chromosome 16 in the mouse. Borriello, et al. (1998) Mamm. Genome 9:114-118. Both CD80 and CD86 serve as ligands in a process known as co-stimulation, and therefore it is likely that OX2 would act as a ligand as well. The OX2 antigen will be referred hereafter as the OX2 protein or ligand OX2. The binding partner will be referred to as the OX2 receptor.

To identify the receptor for OX2 (OX2R), a multivalent reagent was prepared using rat OX2-rat CD4 fusion protein bound to fluorescent beads. This reagent was shown to bind to mouse and rat peritoneal macrophages, and this binding could be blocked by the mAb MRC OX88. Preston, et al. (1997) Eur. J. Immunol. 27:1911-1918. This mAb was shown to bind to macrophages isolated from both peritoneum and spleen and in IHC on spleen sections staining was found in areas known to contain high proportions of macrophages.

A second monoclonal antibody raised by the Barclay group, designated OX102, was shown to bind macrophages in the rat species and also to prevent specifically the binding of the OX2 molecule to rat peritoneal macrophages. Isolation of material binding to the OX102 molecule and N-terminal sequencing showed the putative OX2 receptor (OX2R) to be a novel molecule. This was cloned as described herein. That the protein recognized by the OX102 antibody was indeed the receptor was supported by the demonstration of a longer cytoplasmic tail on this molecule relative to the OX2 molecule itself (the ligand). Clark, et al. (1985) EMBO J. 4:113-118. Preliminary analysis of the OX2R did not reveal obvious motifs consistent with known signaling molecules although this does not exclude the potential role of this molecule in mediating OX2-delivered signals.

Then, a mouse homolog was identified, designated OX2RH1. Because the terminology OX2R should be reserved for those proteins which have been verified to actually bind to the OX2, the initial designation applied is a receptor homolog of the group 1. The nucleotide and amino acid sequences of this molecule are described herein.

Further analysis of available sequence databases revealed the presence of another distinct form of OX2RH, a molecule that showed significant homology in the putative extracellular Ig-domain structures with OX2RH1 but with a different transmembrane and cytoplasmic sequence. These forms have herein been designated OX2RH2, and both human and mouse embodiments have been identified. Of particular note is the presence of a lysine (K) moiety at positions 224 (human) and 170 (mouse) that lies within the transmembrane portion of the molecule. Such a residue suggests that this molecule will associate with molecular partners such as DAP12 known to express motifs capable of signaling for cellular activation. See, e.g., Lanier, et al. (1998) Nature 391:703-707; Colonna(1998) Nature 391:642-3; Campbell, et al. (1999) Int. J. Biochem. Cell. Biol. 31:631-636; and Lopez-Botet, et al. (1999) Curr. Opin. Immunol. 11:301-307. Moreover, such suggests various signaling pathways and associated biochemistry. See, e.g., Lanier, et al. (1998) Immunity 8:693-701; Smith, et al. (1998) J. Immunol. 161:7-10; Gosselin, et al. (1999) J. Leukoc. Biol. 66:165-171; Tomasello, et al.(1998) J. Biol. Chem. 273:34115-34119; and McVicar, et al. (1998) J. Biol. Chem. 273:32934-32942. But, a full length mouse or human OX2RH2 form is yet to be isolated.

There is high homology between the mouse and rat extracellular regions of the OX2RH1 molecule, both of which have been confirmed to bind to their respective species OX2. Thus, the rat and mouse OX2RH1 embodiments are properly also referred to functionally as OX2R. Both contain typical extracellular, transmembrane, and intracellular domain structures. Human OX2RH1 embodiments were discovered. Additionally, soluble forms of the rat and mouse OX2RH1 may exist.

Related homologs, designated OX2RH2 and OX2RH4, have also been described, various embodiments originating in mouse and human. OX2RH2, H3, and H4 embodiments exhibit a charged lysine residue in the transmembrane segment. The human OX2RH2 embodiment lacks a signal sequence and shows some genomic sequence earmarks, suggesting that the functional form of the natural human OX2RH2 should be closely related but slightly different from the sequence provided. The functional relationship of the mouse and human homologs 2 and 4 remain to be confirmed.

A further OX2R homolog was also found in the mouse. Although its homology is much more divergent, it exhibits some similarities in sequence. In particular, it has a lysine residue in the transmembrane region. Thus, like the other OX2RH2, H3, and H4 molecules exhibiting this feature, it would be expected to signal via an associating molecule such as DAP12. This embodiment is herein designated OX2RH3 from rodent, e.g., mouse.

Ongoing analysis of the expression patterns of the OX2RH1 indicates that in rat, mouse, and human leukocytes, OX2RH1 (as determined by flow cytometric staining with the OX102 antibody and/or analysis of mRNA expression by PCR techniques) is expressed most strongly by monocytes, granulocytes, and mast cells, marginally by B cells, and weakly by T cells. This is consistent with the preferential binding of the ligand OX2 to macrophages in earlier studies. In the normal rat central nervous system, a proportion of resident macrophage (or microglial cells) also express the OX2R, but at a low level.

Some applicable standard methods are described or referenced, e.g., in Maniatis, et al. (1982) Molecular Cloning, A Laboratory Manual, Cold Spring Harbor Laboratory, Cold Spring Harbor Press; Sambrook, et al. (1989) Molecular Cloning: A Laboratory Manual, (2d ed.), vols. 1-3, CSH Press, NY; Ausubel, et al., Biology, Greene Publishing Associates, Brooklyn, NY; or Ausubel, et al. (1987 and periodic supplements) Current Protocols in Molecular Biology, Greene/Wiley, New York; each of which is incorporated herein by reference.

Nucleotide (SEQ ID NO: 1) and corresponding amino acid sequence (SEQ ID NO: 2) of a rodent, e.g., rat, OX2 receptor homolog 1 (OX2RH1) coding segment is shown in Table 1. Similarly, further embodiments, primate, e.g., human, and rodent, e.g., mouse, are described, designated OX2RH1, 1.2, 2, and 4. The nucleic acid sequences are SEQ ID NO: 3, 19, 5, 7, 9, and 22; the corresponding amino acid sequences are SEQ ID NO: 4, 20, 6, 8, 10, and 23 which are presented in Table 2. Table 3 provides the sequence of other rodent, e.g., mouse, OX2RH3 (SEQ ID NO: 11 and 12).

Reverse translation nucleic acid sequences are provided in Table 4 (SEQ ID NO: 13-14, 21, 15-17, 24, and 18). Table 5 provides alignment and numeric comparison of polypeptide sequences.

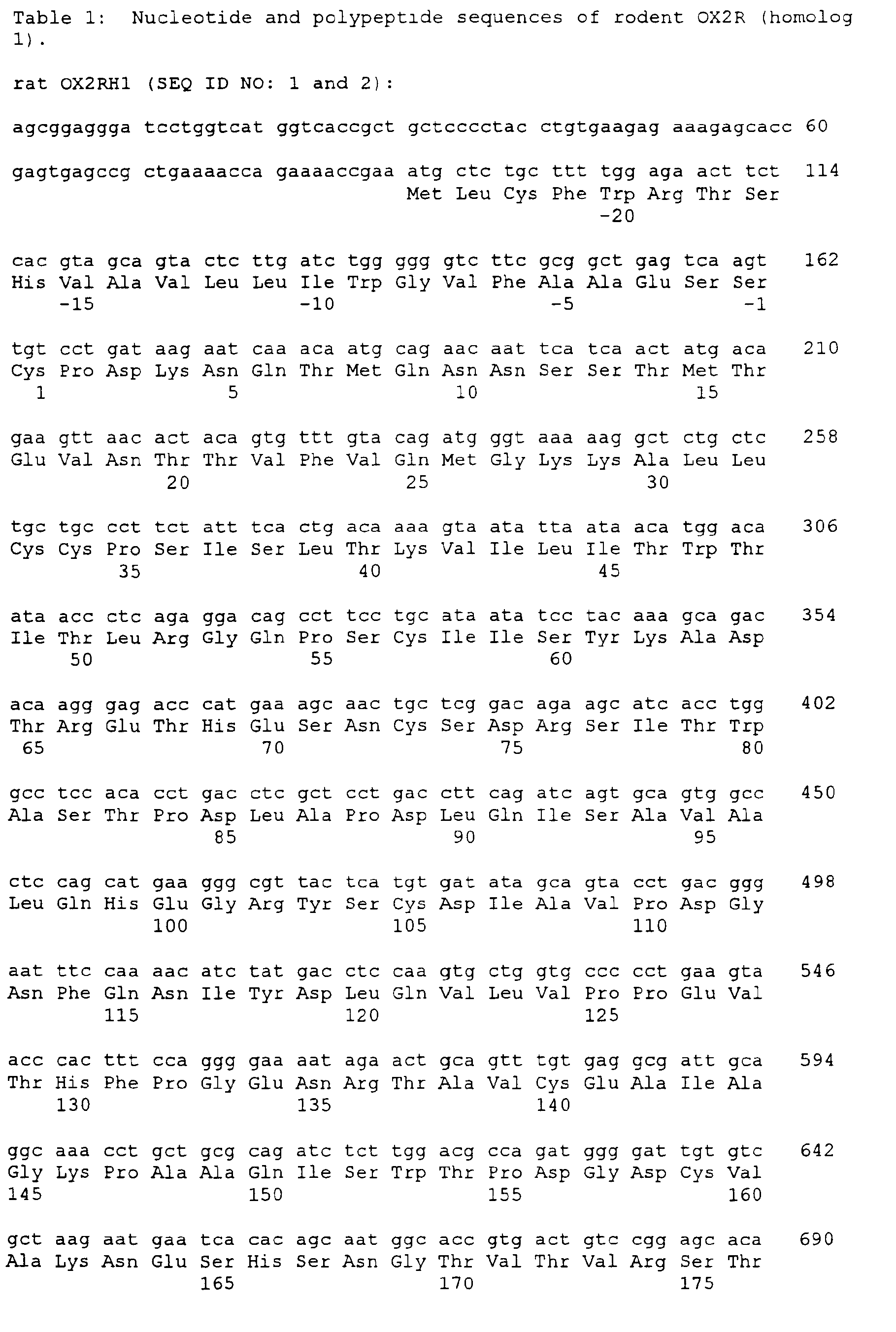

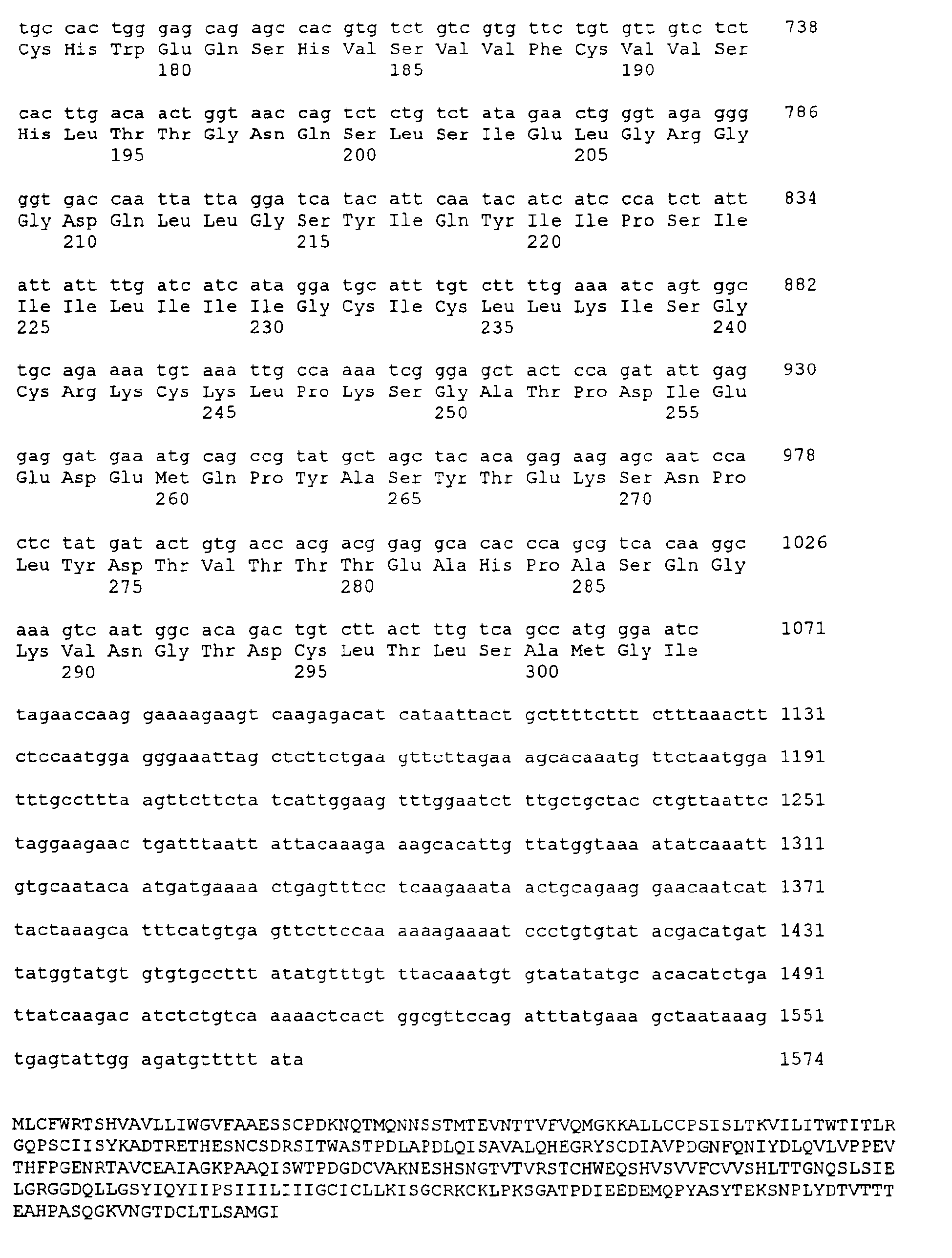

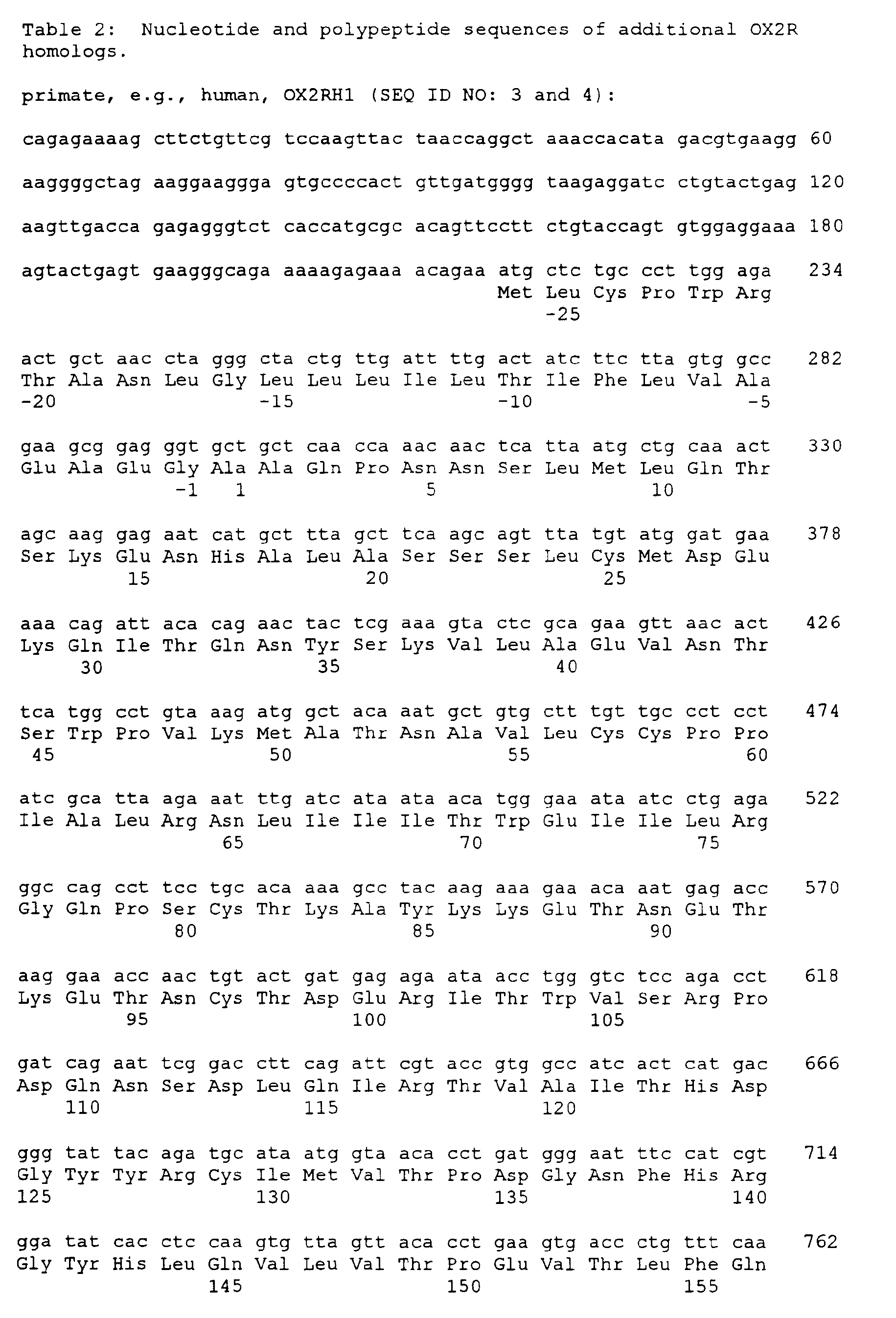

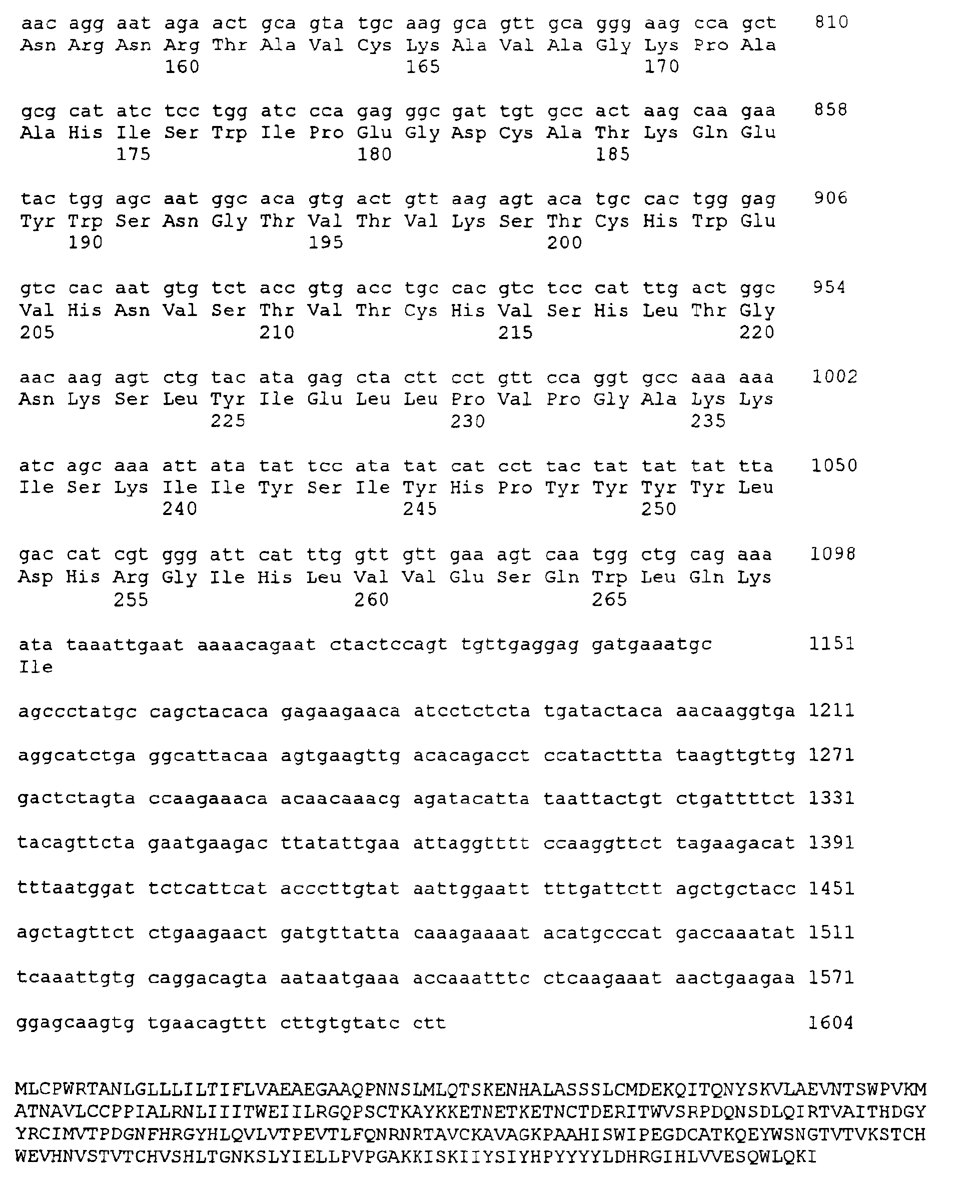

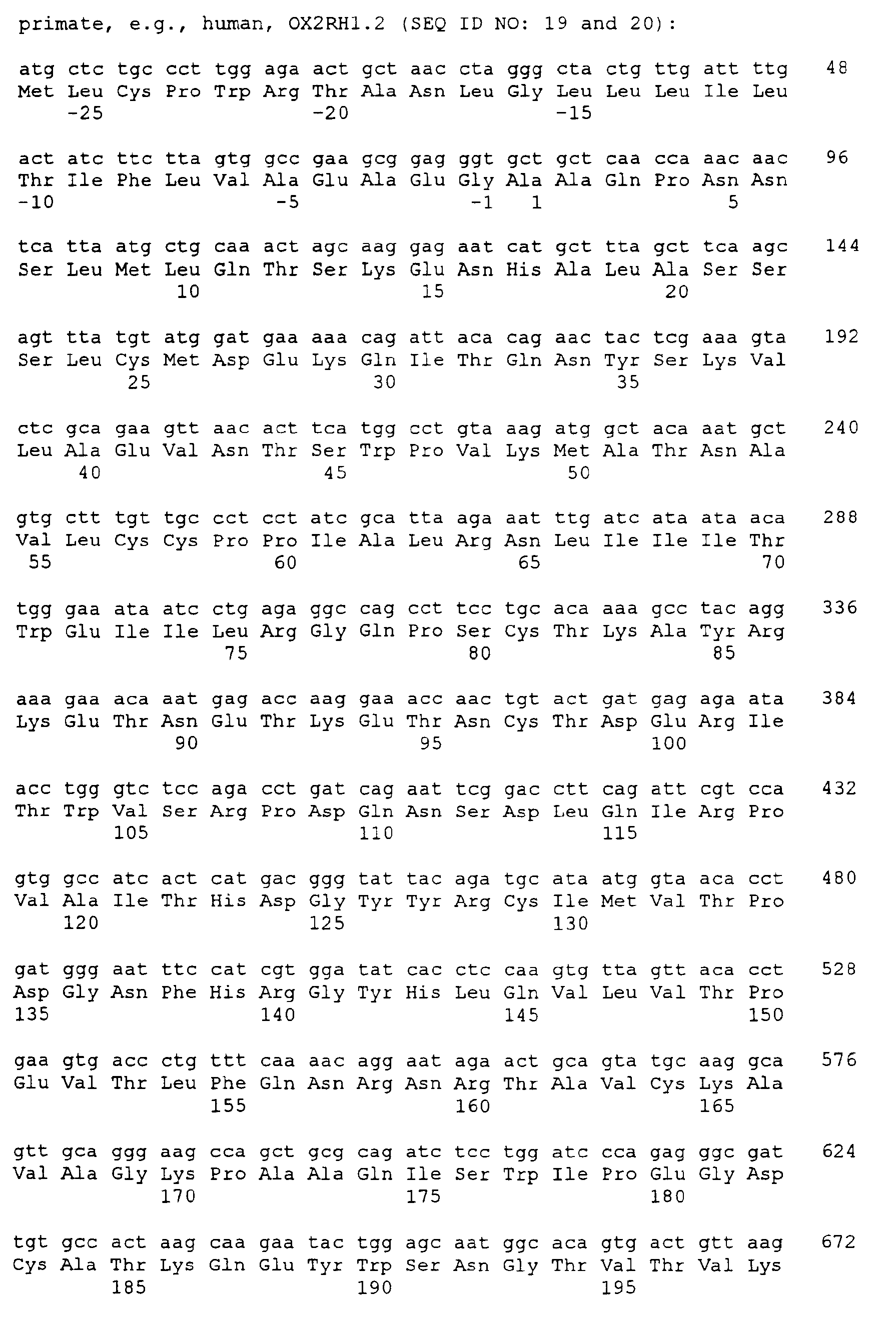

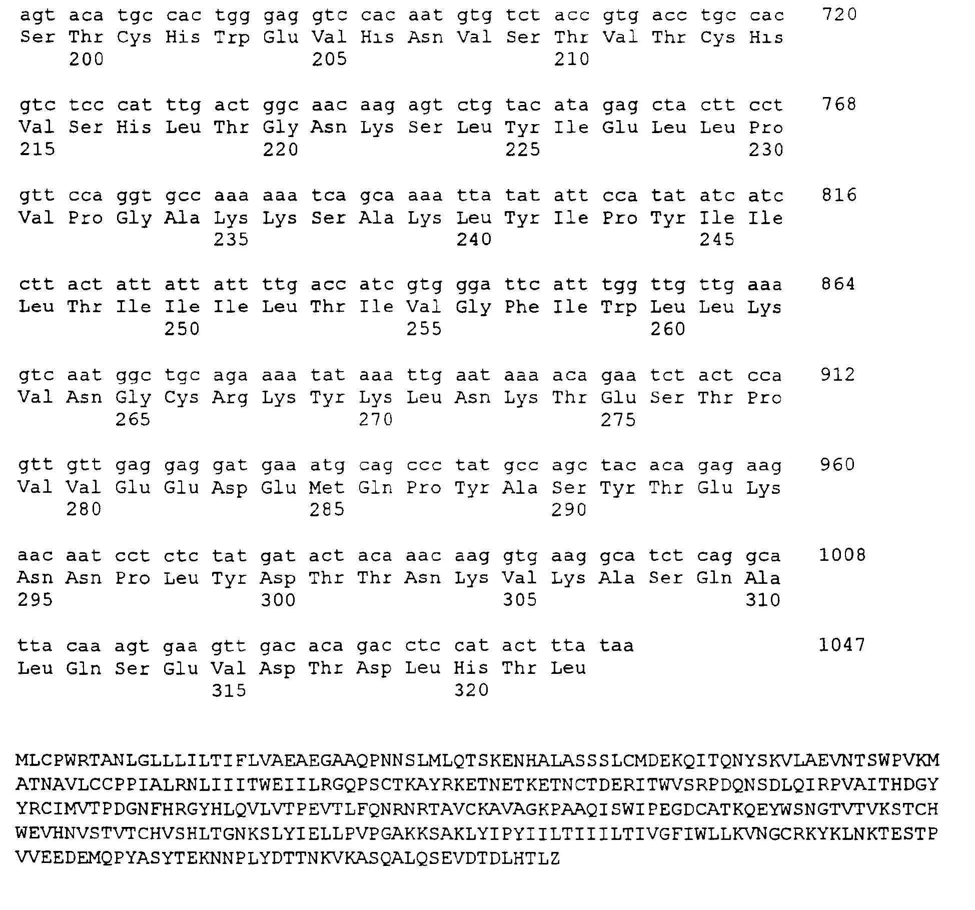

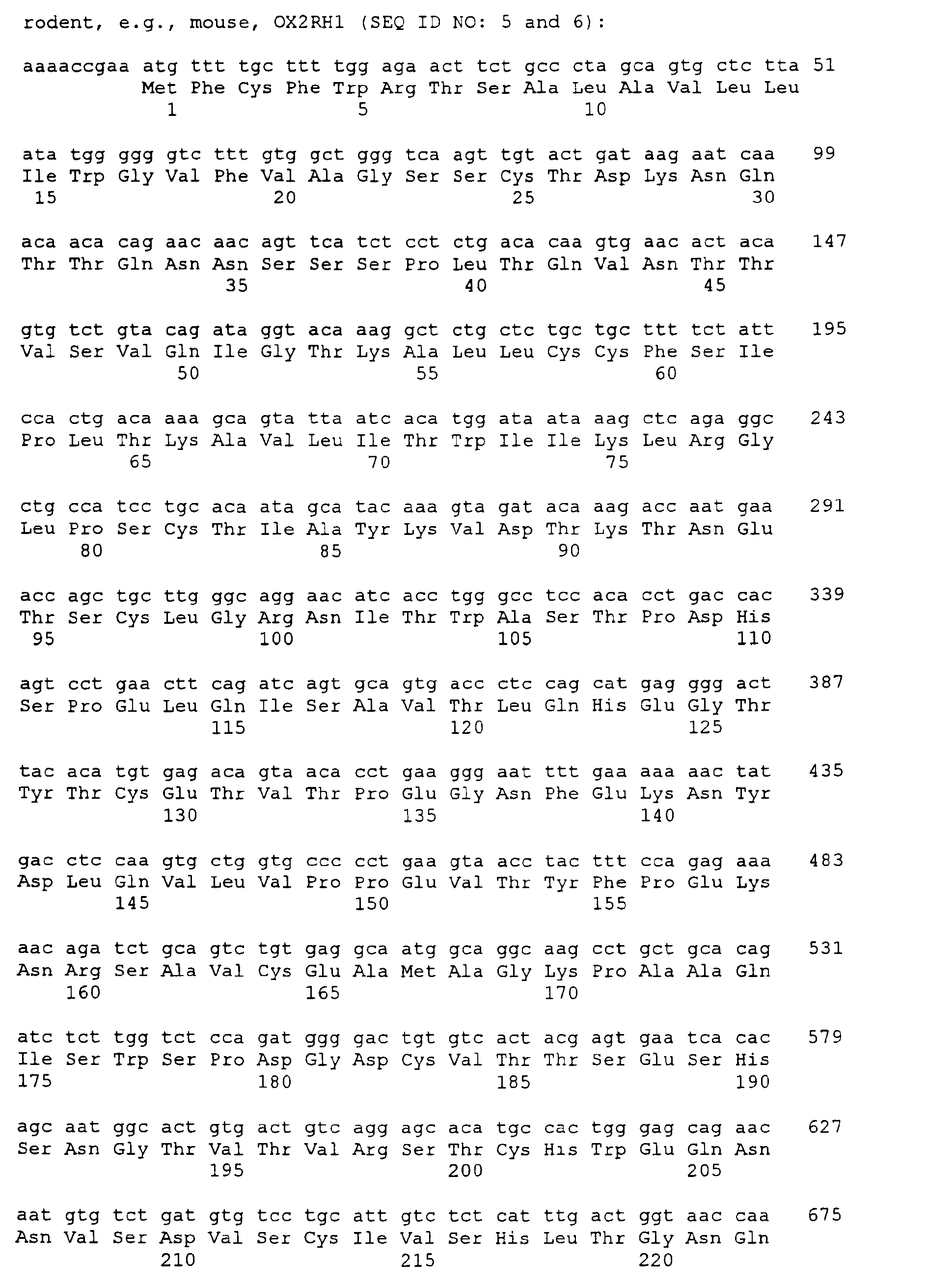

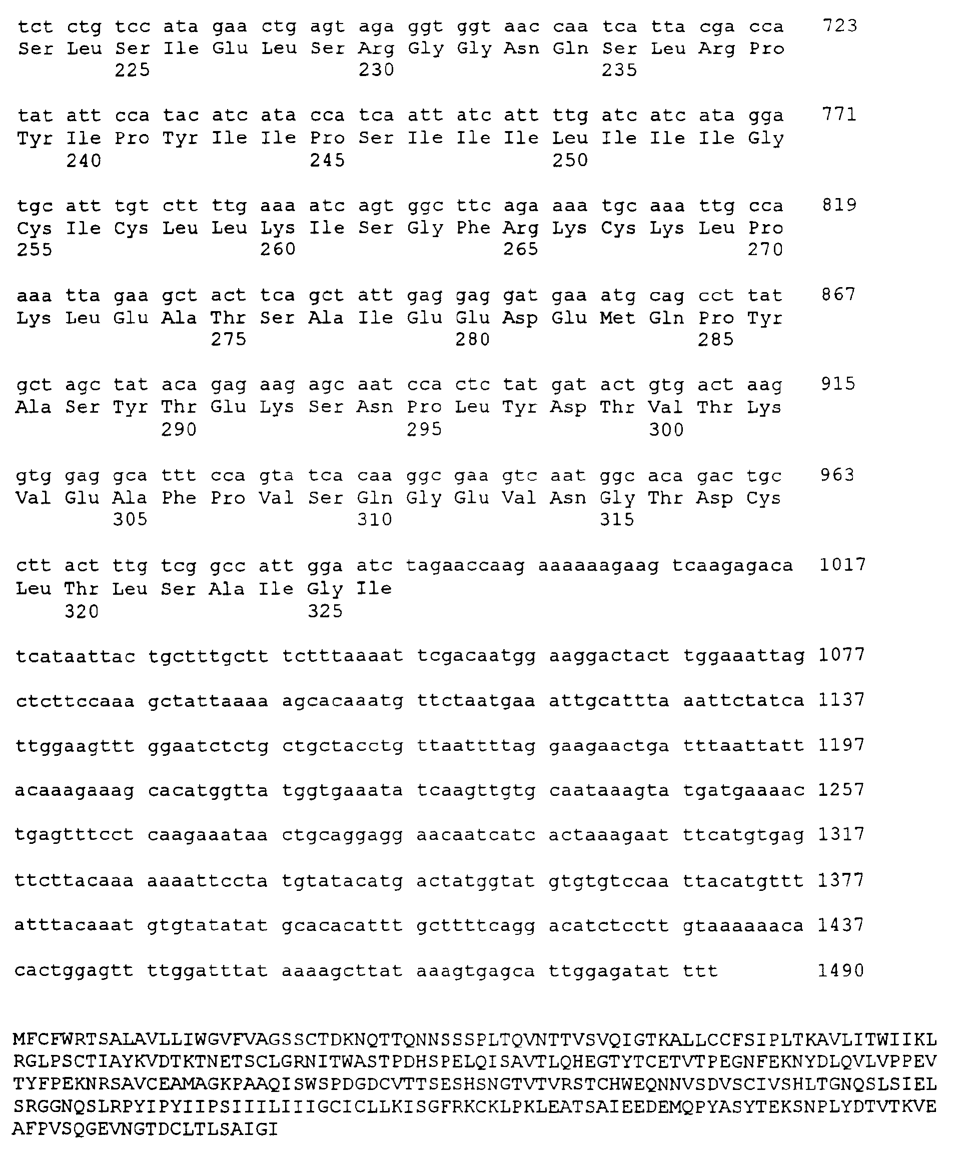

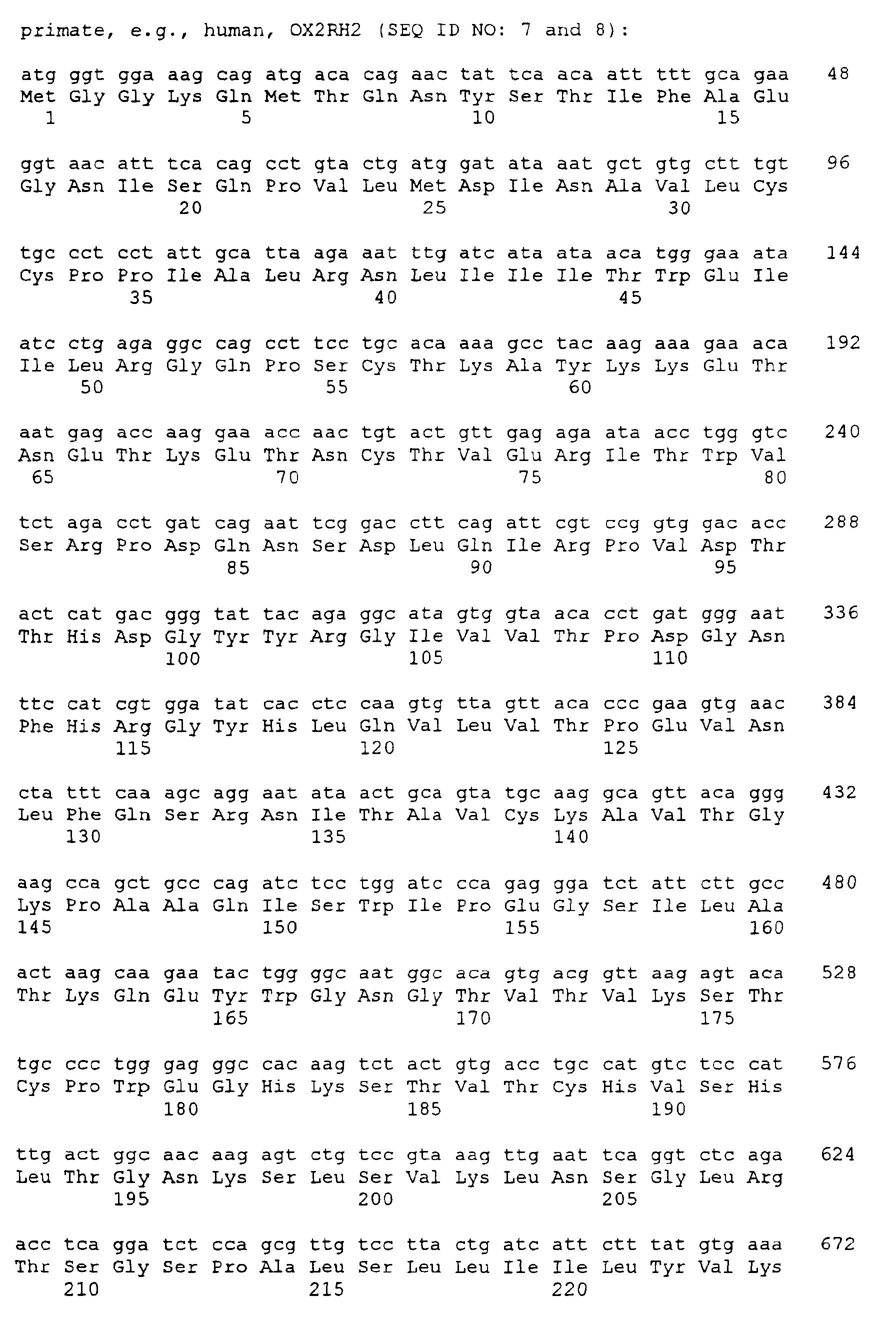

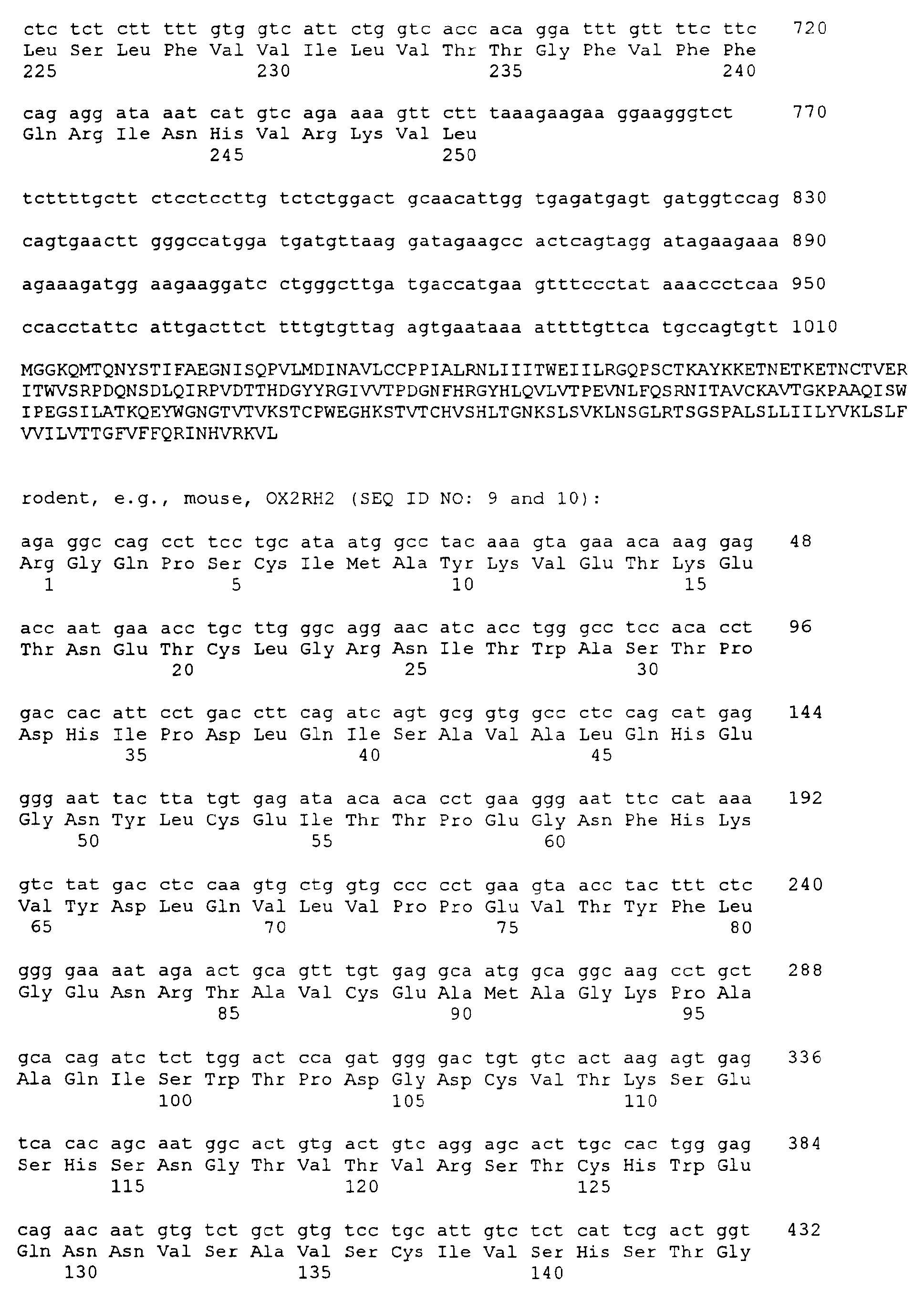

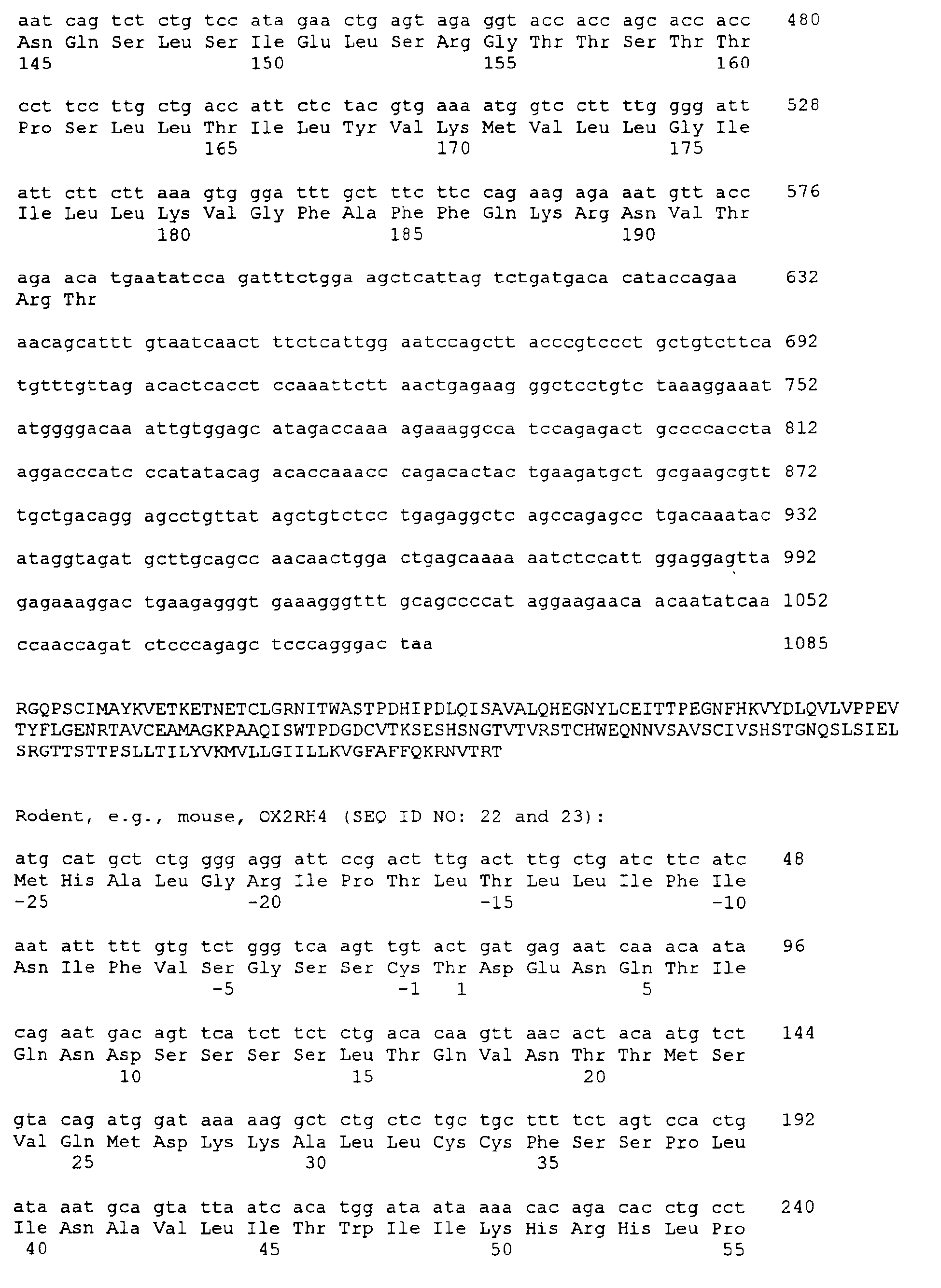

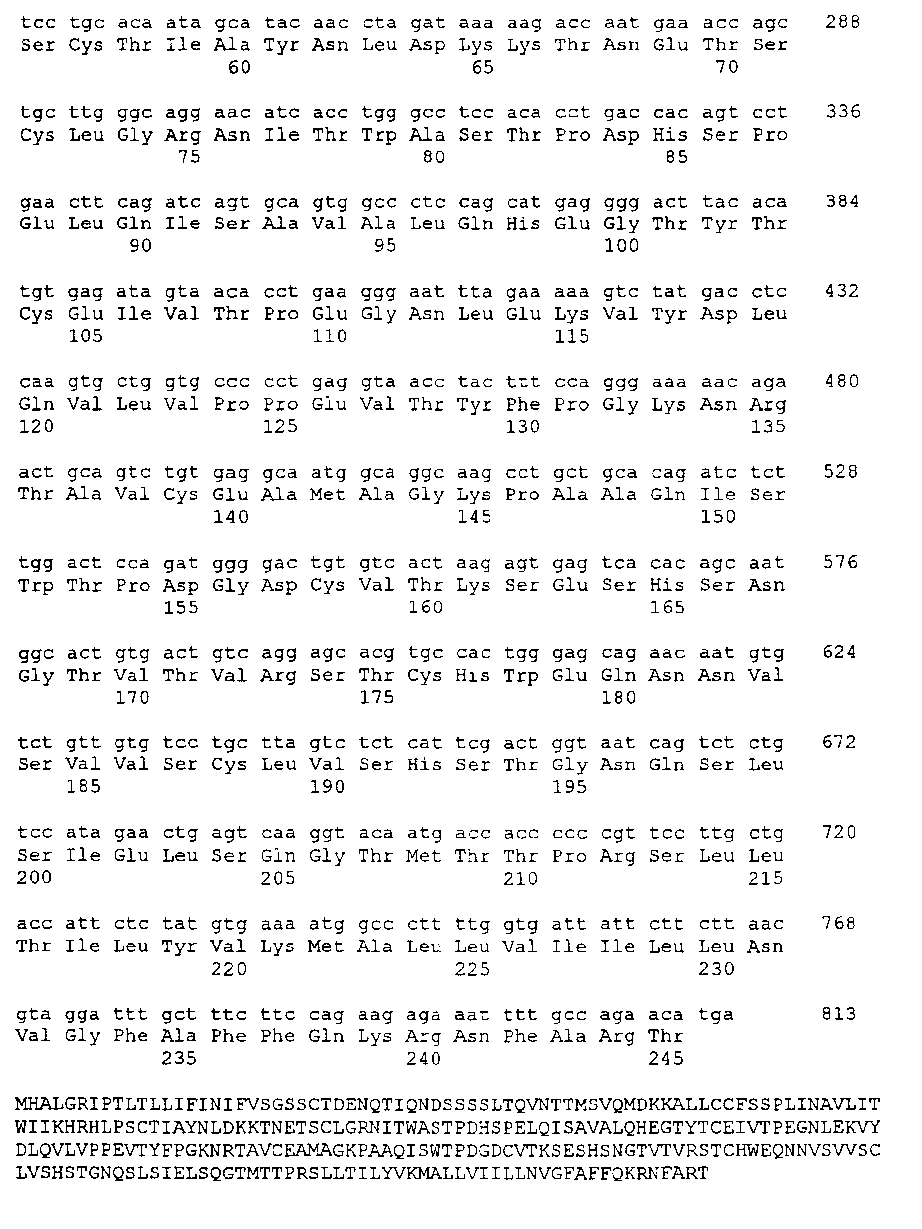

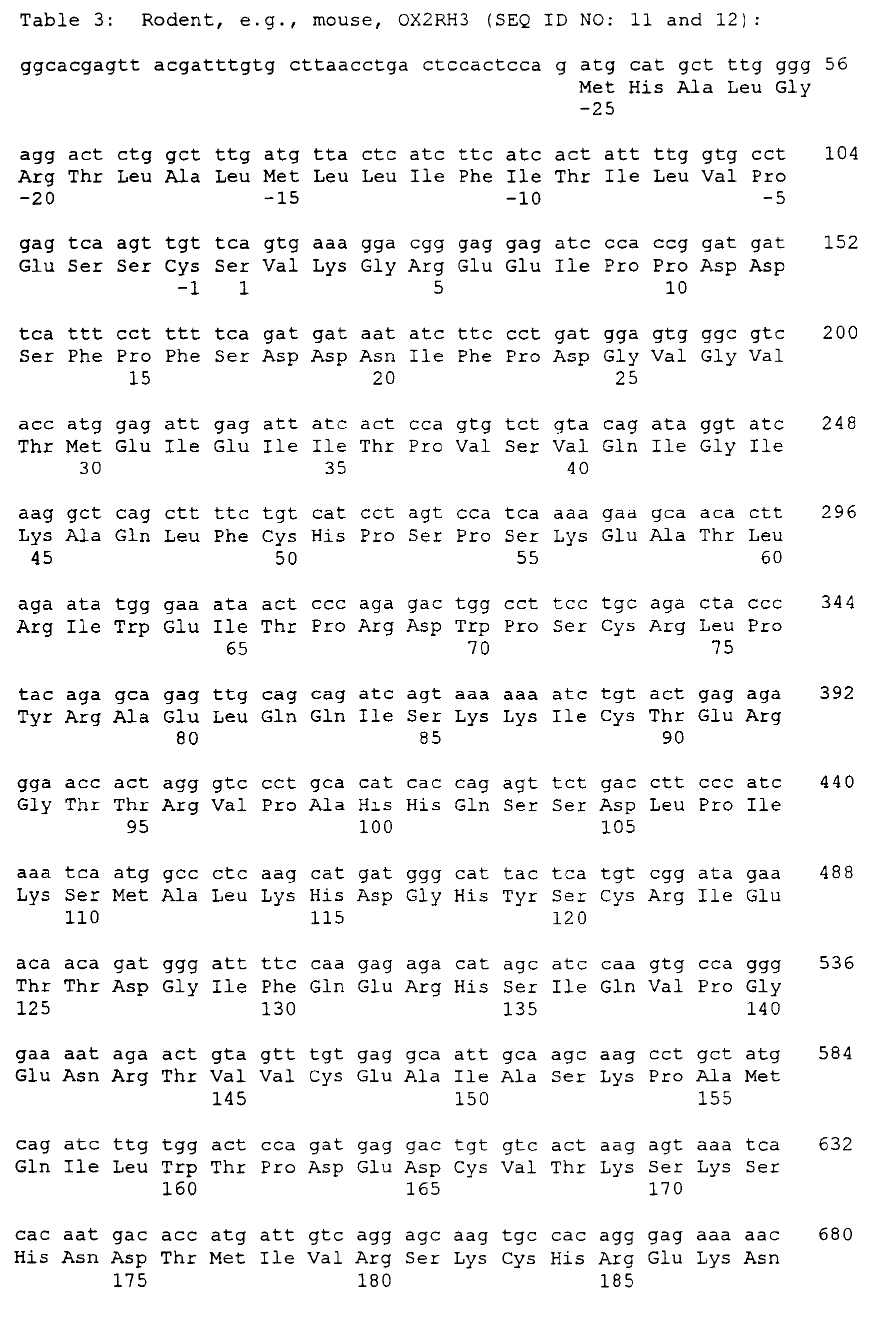

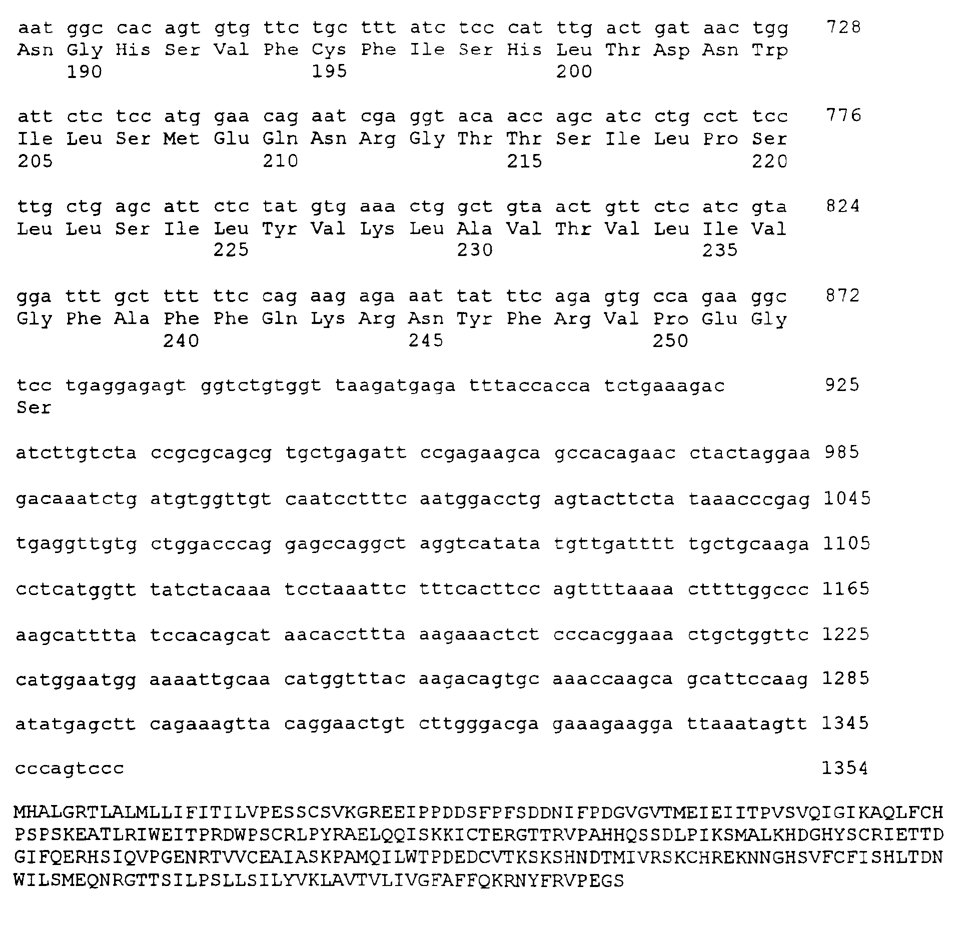

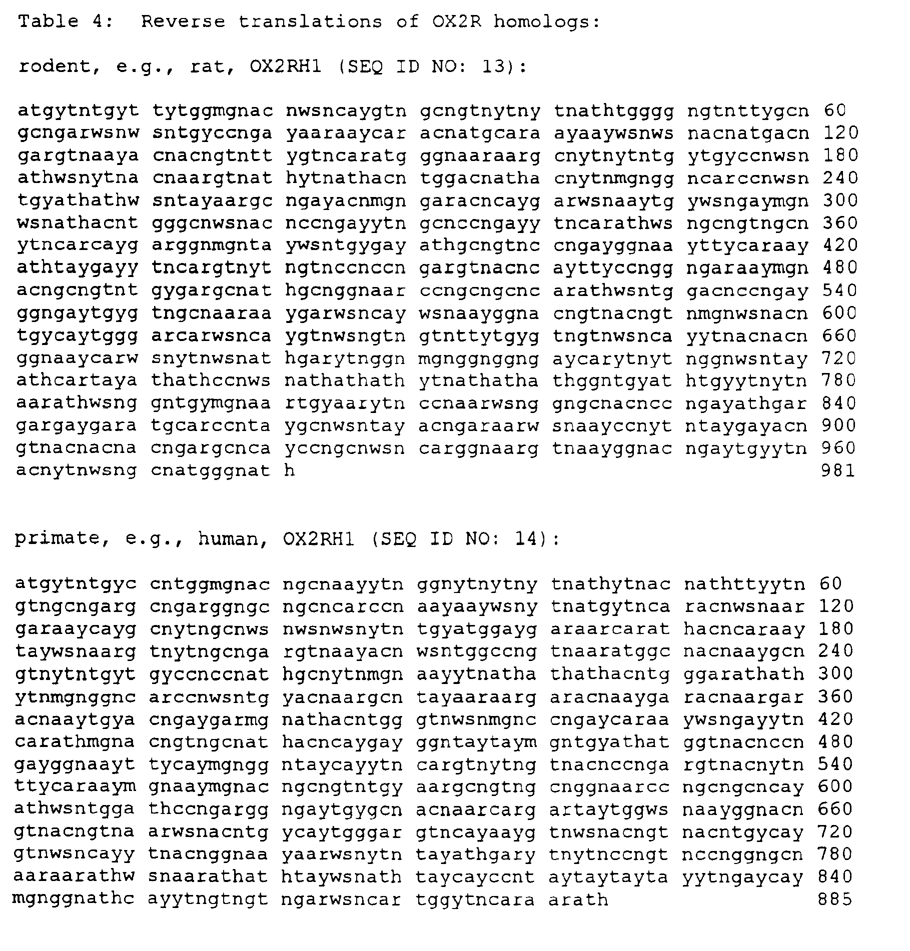

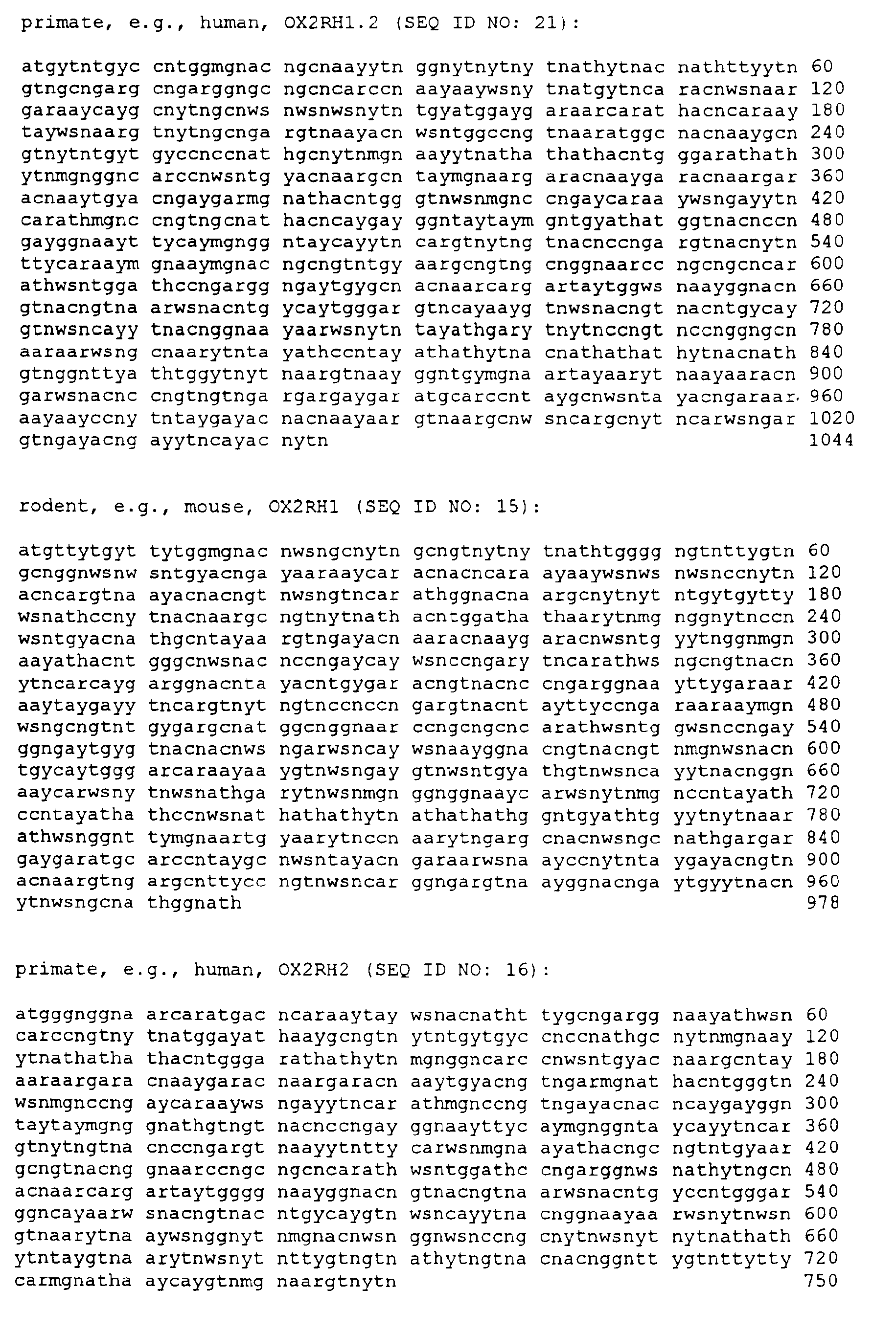

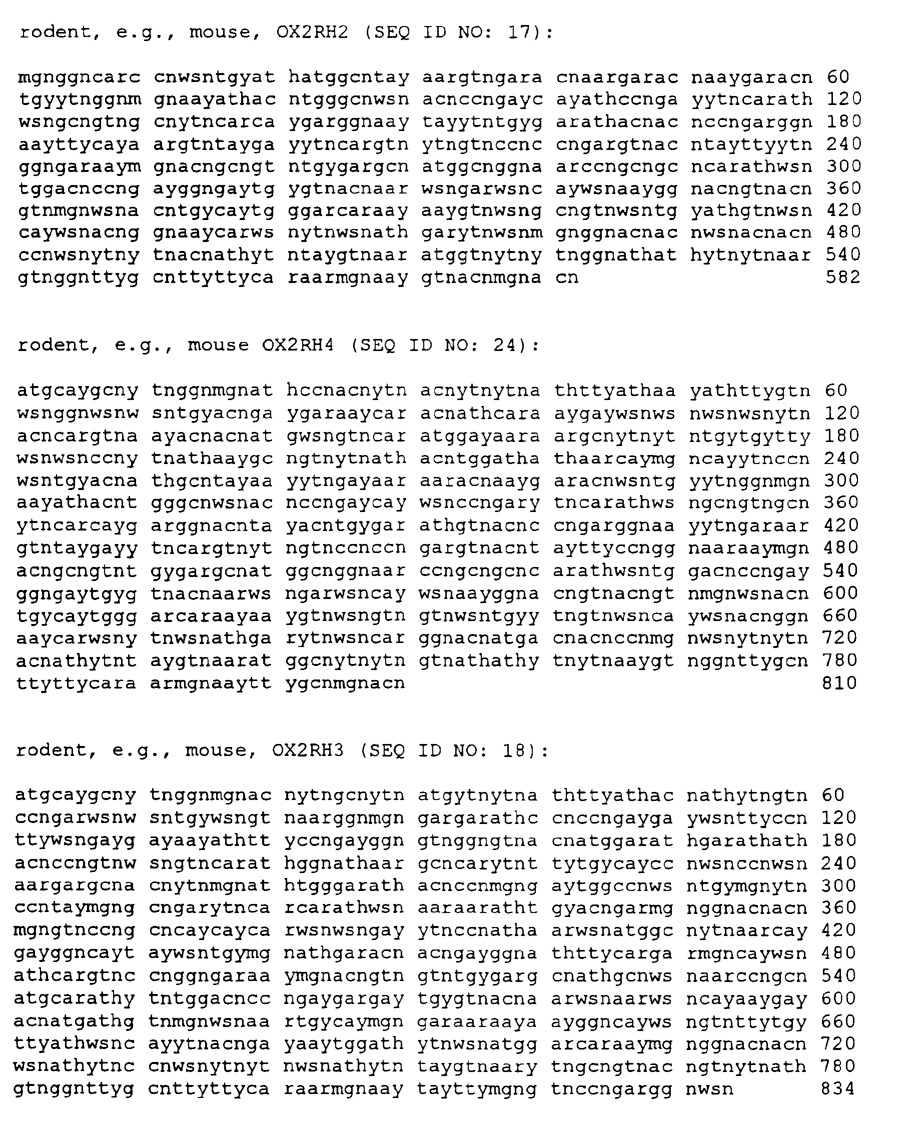

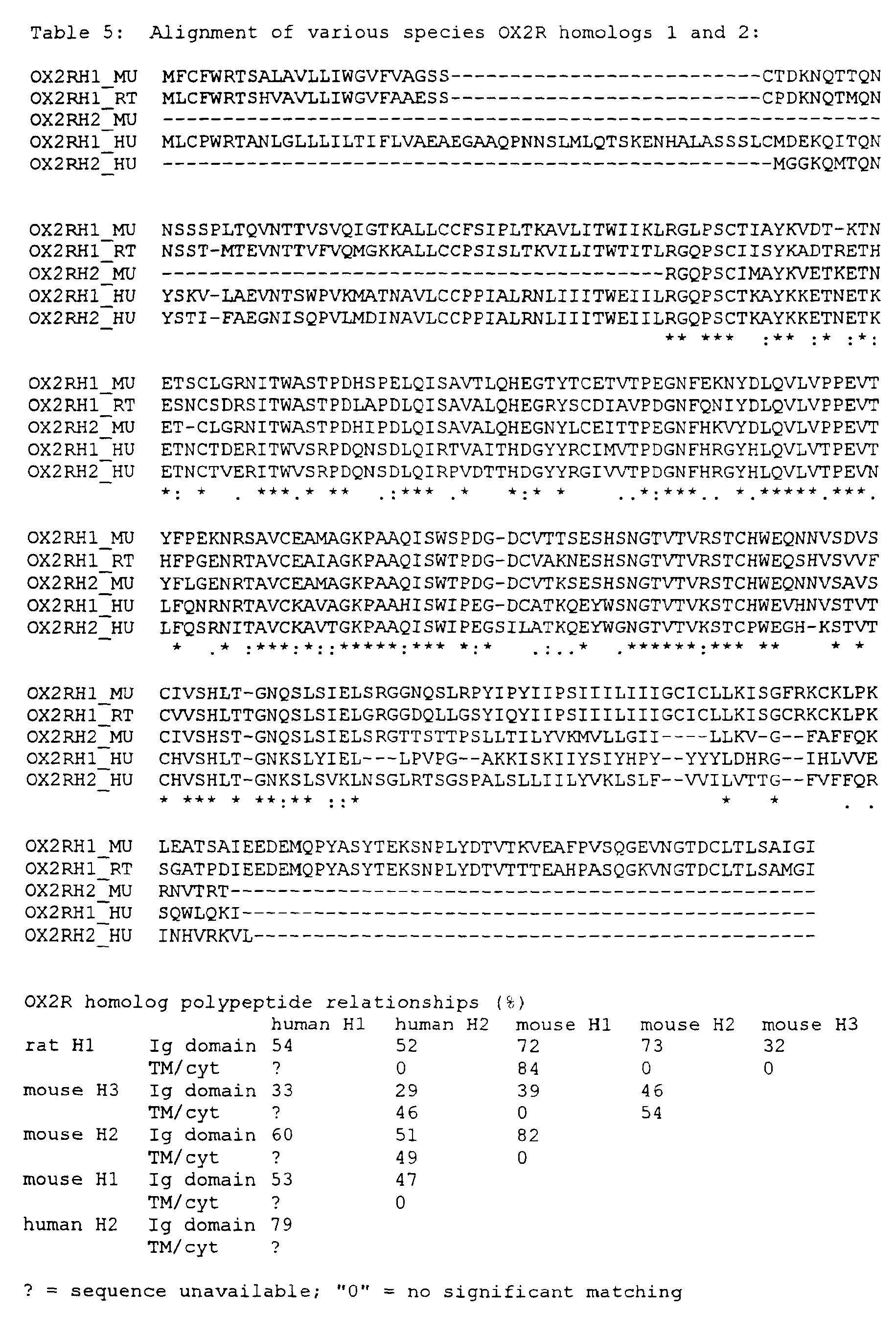

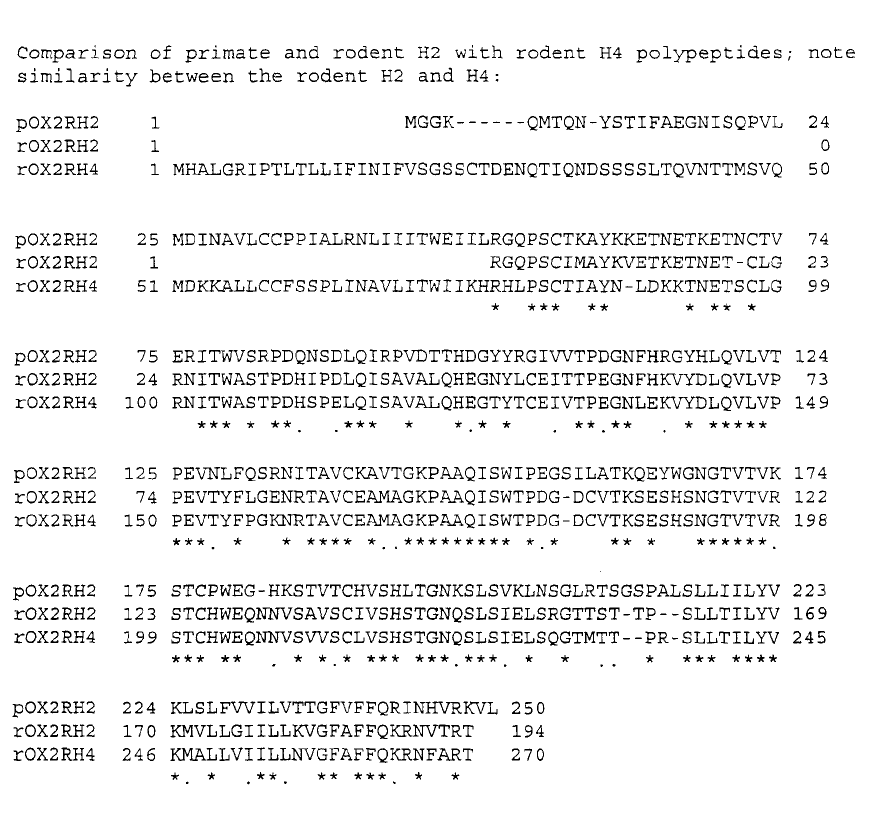

The OX2RH1 and 2 embodiments show particular similarity to one another, see, e.g., Table 5. Particular regions or positions of interest are, for the rat H1: boundaries adjacent to (before, at, or after) cys2, leu33, cys35, ile46, trp48, arg53, pro56, cys58, tyr62, cys74, thr80, trp81, leu91, ile93, his100, gly102, tyr104, gly113, phe115, leu122, va1123, pro127, asn136, ala139, va1140, cys141, ala143, lys147, pro148, ala149, ile152, trp154, pro156, asn169, thr171, va1174, ser176, cys178, glu181, ser186, va1188, cys190, ser193, his194, thr196, asn198, leu202, gly215, tyr217, leu237, lys238, and ile304. Many of the residues are conserved across the H1 and H2 classes. Likewise with H2 and H4. See Table 5. Particular domains of interest in rat OX2RH1 are the C2 domain from about cys2 to pro127, the C2 domain from about glu128-g1y215, the TM segment from about tyr217-leu237, and the intracellular domain from about lys238-ile304. Corresponding segments in mouse H1 are about ser24-pro150, glu151-gly231, tyr239-leu259, and lys260-ile326. In the mouse H2, the segments correspond, in available sequence, from about argl-pro74, glu75-gly155, pro161-g1y182, and phe183-thr194. For human H2, the transmembrane segment is about ala214-va1233, and thr234-leu250, and in mouse H3, about pro119-gly237, with the intramembrane lys228, and phe238-gly252. Table 5 also indicates alignment of the H2 snd H4 embodiments. Additional positions of interest, e.g., as boundaries for fragments, will be those conserved across homolog groups with the rat OX2RH1 or various subsets of the family members.

Functionally, the rat and mouse H1 have been shown to bind to the OX2. This has not yet been confirmed for the human H1, but can be easily tested. Ligand matching for the H2, H4, and H3 groups is described below.

The rodent H3 has been shown to associate with DAP12, as predicted. Recombinantly expressed epitope tagged DAP12 is not membrane associated in the absence of coexpression of a chaparone partner. see, e.g., Bakker, et al. (1999) Proc. Nat'l Acad. Sci. USA 96:9792-9796. Mouse H3 can serve as the chaparone partner. However, the signal pathway through DAP12 requires binding to the H3 ligand, which has not yet been identified, but can be found using appropriate screening strategies, e.g., biochemical or physical methods. Sequence similarity of H2 and rodent H4 suggest a similar association with either the DAP12, or possibly the DAP10.

The mouse H2 and H4 and human H2 are likely also to possess such properties, e.g., association with DAP12 (activating) or DAP10. The signaling pathways have been determined with some of the related receptors on NK cells. See, e.g., Lanier, et al. (1998) Immunity 8:693-701; Smith, et al. (1998) J. Immunol . 161:7-10; Gosselin, et al. (1999) J. Leukoc. Biol. 66:165-171; Tomasello, et al.(1998) J. Biol. Chem. 273:34115-34119; and McVicar, et al. (1998) J. Biol. Chem. 273:32934-32942. Because of the similarity of the extracellular domains with the H1 members, OX2-like genes, particularly OX2, are likely ligands.

As used herein, the term OX2RH1, OX2RH2, or OX2RH4 shall be used to describe a protein comprising amino acid sequences shown, e.g., in Tables 1-2. In many cases, a substantial fragment thereof will be functionally or structurally equivalent, including, e.g., an extracellular or intracellular domain. The invention also includes protein variants of the respective OX2RH alleles whose sequences are provided, e.g., muteins or soluble extracellular constructs. Typically, such agonists or antagonists will exhibit less than about 10% sequence differences, and thus will often have between 1 and 11 residue substitutions, e.g., 2, 3, 5, 7, and others. It also encompasses allelic and other variants, e.g., natural polymorphic, of the proteins described. Typically, the receptor will bind to a corresponding biological ligand with high affinity, e.g., at least about 100 nM, usually better than about 30 nM, preferably better than about 10 nM, and more preferably at better than about 3 nM. The term shall also be used herein to refer to related naturally occurring forms, e.g., alleles, polymorphic variants, and metabolic variants of the mammalian proteins. Preferred forms of the receptor complexes will bind the appropriate ligand with an affinity and selectivity appropriate for a ligand-receptor interaction.

This invention also encompasses combinations of proteins or peptides having substantial amino acid sequence identity with the amino acid sequences in Tables 1-3. It will include sequence variants with relatively few substitutions, e.g., preferably less than about 3-5.

A substantial polypeptide "fragment", or "segment", is a stretch of amino acid residues of at least about 8 amino acids, generally at least 10 amino acids, more generally at least 12 amino acids, often at least 14 amino acids, more often at least 16 amino acids, typically at least 18 amino acids, more typically at least 20 amino acids, usually at least 22 amino acids, more usually at least 24 amino acids, preferably at least 26 amino acids, more preferably at least 28 amino acids, and, in particularly preferred embodiments, at least about 30 or more amino acids. Sequences of segments of different proteins can be compared to one another over appropriate length stretches. In many situations, fragments may exhibit functional properties of the intact subunits, e.g., the extracellular domain of the transmembrane receptor may retain the ligand binding features, and may be used to prepare a soluble receptor-like complex.

Amino acid sequence homology, or sequence identity, is determined by optimizing residue matches. In some comparisons, gaps may be introduced, as required. See, e.g., Needleham, et al. (1970) J. Mol. Biol. 48:443-453; Sankoff, et al. (1983) chapter one in Time Warps, String Edits, and Macromolecules: The Theory and Practice of Sequence Comparison, Addison-Wesley, Reading, MA; and software packages from IntelliGenetics, Mountain View, CA; and the University of Wisconsin Genetics Computer Group (GCG), Madison, WI; each of which is incorporated herein by reference. This changes when considering conservative substitutions as matches. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine. Homologous amino acid sequences are intended to include natural allelic and interspecies variations in the receptor homolog sequence. Typical homologous proteins or peptides will have from 50-100% homology (if gaps can be introduced), to 60-100% homology (if conservative substitutions are included) with an amino acid sequence segment of Tables 1-3. Homology measures will be at least about 70%, generally at least 76%, more generally at least 81%, often at least 85%, more often at least 88%, typically at least 90%, more typically at least 92%, usually at least 94%, more usually at least 95%, preferably at least 96%, and more preferably at least 97%, and in particularly preferred embodiments, at least 98% or more. The degree of homology will vary with the length of the compared segments. Homologous proteins or peptides, such as the allelic variants, will share most biological activities with the embodiments described in Tables 1-3.

As used herein, the term "biological activity" is used to describe, without limitation, effects on inflammatory responses, innate immunity, and/or morphogenic development by receptor-like proteins. For example, these receptors are likely to mediate their effects through phosphatase or phosphorylase activities, which activities are easily measured by standard procedures. See, e.g., Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738. The receptor homologs, or portions thereof, may be useful as phosphate labeling enzymes to label general or specific substrates. The subunits may also be functional immunogens to elicit recognizing antibodies, or antigens capable of binding antibodies.

The terms ligand, agonist, antagonist, and analog of, e.g., an OX2RH, include molecules that modulate the characteristic cellular responses to binding of OX2 proteins, as well as molecules possessing the more standard structural binding competition features of ligand-receptor interactions, e.g., where the antagonist is a soluble extracellular domain of a receptor homolog or an antibody. The cellular responses likely are typically mediated through receptor tyrosine kinase pathways.

Also, a receptor homolog may be a molecule which serves either as a natural receptor to which said ligand, or an analog thereof, binds, or a molecule which is a functional analog of the natural receptor. The functional analog may be a receptor homolog with structural modifications, or may be a wholly unrelated molecule which has a molecular shape which interacts with the appropriate ligand binding determinants. The ligands may serve as agonists or antagonists, see, e.g., Goodman, et al. (eds. 1990) Goodman & Gilman's: The Pharmacological Bases of Therapeutics, Pergamon Press, New York.

Rational drug design may also be based upon structural studies of the molecular shapes of a receptor homolog, antibody, or other effectors or receptor homolog associated entities. See, e.g., Herz, et al. (1997) J. Recent. Signal Transduct. Res. 17:671-776; and Chaiken, et al. (1996) Trends Biotechnol. 14:369-375. Effectors may be other proteins which mediate other functions in response to ligand binding, or other proteins which normally interact with the receptor homolog. One means for determining which sites interact with specific other proteins is a physical structure determination, e.g., x-ray crystallography or 2 dimensional NMR techniques. These will provide guidance as to which amino acid residues form molecular contact regions. For a detailed description of protein structural determination, see, e.g., Blundell and Johnson (1976) Protein Crystallography, Academic Press, New York, which is hereby incorporated herein by reference.

### II. Activities

The receptor-like proteins will have a number of different biological activities, e.g., modulating cell proliferation, or in phosphate metabolism, being added to or removed from specific substrates, typically proteins. Such will generally result in modulation of an inflammatory function, other innate immunity response, or a morphological effect. The receptor homolog will probably have a specific low affinity binding to the ligand, as described.

The OX2RH1 has motifs suggestive of a receptor signaling through a receptor tyrosine kinase pathway. See, e.g., Ihle, et al. (1997) Stem Cells 15(suppl. 1):105-111; Silvennoinen, et al. (1997) APMIS 105:497-509; Levy (1997) Cytokine Growth Factor Review 8:81-90; Winston and Hunter (1996) Current Biol. 6:668-671; Barrett (1996) Baillieres Clin. Gastroenterol. 10:1-15; and Briscoe, et al. (1996) Philos. Trans. R. Soc. Lond. B. Biol. Sci. 351:167-171.

The biological activities of the OX2R homologs will likely be related to addition or removal of phosphate moieties to substrates, typically in a specific manner, but occasionally in a non specific manner. Substrates may be identified, or conditions for enzymatic activity may be assayed by standard methods, e.g., as described in Hardie, et al. (eds. 1995) The Protein Kinase FactBook vols. I and II, Academic Press, San Diego, CA; Hanks, et al. (1991) Meth. Enzymol. 200:38-62; Hunter, et al. (1992) Cell 70:375-388; Lewin (1990) Cell 61:743-752; Pines, et al. (1991) Cold Spring Harbor Symp. Quant. Biol. 56:449-463; and Parker, et al. (1993) Nature 363:736-738.

A receptor homolog may combine with one or more other proteins to form functional complexes, e.g., which may be useful for binding ligand or preparing antibodies. These will have substantial diagnostic uses, including detection or quantitation.

### III. Nucleic Acids

This invention contemplates use of isolated nucleic acid or fragments, e.g., which encode these or closely related proteins, or fragments thereof, e.g., to encode a corresponding polypeptide, preferably one which is biologically active. In addition, this invention covers isolated or recombinant DNAs which encode combinations of such proteins or polypeptides having characteristic sequences, e.g., of the homologs. Typically, the nucleic acid is capable of hybridizing, under appropriate conditions, with a nucleic acid sequence segment shown in Tables 1-3, but preferably not with a corresponding segment of other known Ig superfamily receptors. Said biologically active protein or polypeptide can be a full length protein, or fragment, and will typically have a segment of amino acid sequence highly homologous, e.g., exhibiting significant stretches of identity, to one shown in Tables 1-3. Further, this invention covers the use of isolated or recombinant nucleic acid, or fragments thereof, which encode proteins having fragments which are equivalent to the OX2RH proteins. The isolated nucleic acids can have the respective regulatory sequences in the 5' and 3' flanks, e.g., promoters, enhancers, poly-A addition signals, and others from the natural gene.

An "isolated" nucleic acid is a nucleic acid, e.g., an RNA, DNA, or a mixed polymer, which is substantially pure, e.g., separated from other components which naturally accompany a native sequence, such as ribosomes, polymerases, and flanking genomic sequences from the originating species. The term embraces a nucleic acid sequence which has been removed from its naturally occurring environment, and includes recombinant or cloned DNA isolates, which are thereby distinguishable from naturally occurring compositions, and chemically synthesized analogs or analogs biologically synthesized by heterologous systems. A substantially pure molecule includes isolated forms of the molecule, either completely or substantially pure.

An isolated nucleic acid will generally be a homogeneous composition of molecules, but will, in some embodiments, contain heterogeneity, preferably minor. This heterogeneity is typically found at the polymer ends or portions not critical to a desired biological function or activity.

A "recombinant" nucleic acid is typically defined either by its method of production or its structure. In reference to its method of production, e.g., a product made by a process, the process is use of recombinant nucleic acid techniques, e.g., involving human intervention in the nucleotide sequence. Typically this intervention involves in vitro manipulation, although under certain circumstances it may involve more classical animal breeding techniques. Alternatively, it can be a nucleic acid made by generating a sequence comprising fusion of two fragments which are not naturally contiguous to each other, but is meant to exclude products of nature, e.g., naturally occurring mutants as found in their natural state. Thus, for example, products made by transforming cells with an unnaturally occurring vector is encompassed, as are nucleic acids comprising sequence derived using most any synthetic oligonucleotide process. Such a process is often done to replace a codon with a redundant codon encoding the same or a conservative amino acid, while typically introducing or removing a restriction enzyme sequence recognition site. Alternatively, the process is performed to join together nucleic acid segments of desired functions to generate a single genetic entity comprising a desired combination of functions not found in the commonly available natural forms, e.g., encoding a fusion protein. Restriction enzyme recognition sites are often the target of such artificial manipulations, but other site specific targets, e.g., promoters, DNA replication sites, regulation sequences, control sequences, or other useful features may be incorporated by design. A similar concept is intended for a recombinant, e.g., fusion, polypeptide. This will include a dimeric repeat. Specifically included are synthetic nucleic acids which, by genetic code redundancy, encode equivalent polypeptides to fragments of OX2 receptor homologs and fusions of sequences from various different related molecules, e.g., other Ig superfamily members.

A "fragment" in a nucleic acid context is a contiguous segment of at least about 17 nucleotides, generally at least 21 nucleotides, more generally at least 25 nucleotides, ordinarily at least 30 nucleotides, more ordinarily at least 35 nucleotides, often at least 39 nucleotides, more often at least 45 nucleotides, typically at least 50 nucleotides, more typically at least 55 nucleotides, usually at least 60 nucleotides, more usually at least 66 nucleotides, preferably at least 72 nucleotides, more preferably at least 79 nucleotides, and in particularly preferred embodiments will be at least 85 or more nucleotides. Typically, fragments of different genetic sequences can be compared to one another over appropriate length stretches, particularly defined segments such as the domains described below.

A nucleic acid which codes for OX2R homologs will be particularly useful to identify genes, mRNA, and cDNA species which code for itself or closely related proteins, as well as DNAs which code for polymorphic, allelic, or other genetic variants, e.g., from different individuals or related species. Preferred probes for such screens are those regions of the receptor homolog which are conserved between different polymorphic variants or which contain nucleotides which lack specificity, and will preferably be full length or nearly so. In other situations, polymorphic variant specific sequences will be more useful. Quantitation or specific sequence analysis may be useful as markers for disease or medical conditions, or in selecting particular therapeutic treatments.

This invention further covers recombinant nucleic acid molecules and fragments having a nucleic acid sequence identical to or highly homologous to the isolated DNA set forth herein. In particular, the sequences will often be operably linked to DNA segments which control transcription, translation, and/or DNA replication. These additional segments typically assist in expression of the desired nucleic acid segment.

Homologous, or highly identical, nucleic acid sequences, when compared to one another, e.g., OX2RH sequences, exhibit significant similarity. The standards for homology in nucleic acids are either measures for homology generally used in the art by sequence comparison or based upon hybridization conditions. Comparative hybridization conditions are described in greater detail below.

Substantial identity in the nucleic acid sequence comparison context means either that the segments, or their complementary strands, when compared, are identical when optimally aligned, with appropriate nucleotide insertions or deletions, in at least about 60% of the nucleotides, generally at least 66%, ordinarily at least 71%, often at least 76%, more often at least 80%, usually at least 84%, more usually at least 88%, typically at least 91%, more typically at least about 93%, preferably at least about 95%, more preferably at least about 96 to 98% or more, and in particular embodiments, as high at about 99% or more of the nucleotides, including, e.g., segments encoding structural domains such as the segments described below. Alternatively, substantial identity will exist when the segments will hybridize under selective hybridization conditions, to a strand or its complement, typically using a sequence derived from Tables 1-3. Typically, selective hybridization will occur when there is at least about 55% homology over a stretch of at least about 14 nucleotides, more typically at least about 65%, preferably at least about 75%, and more preferably at least about 90%. See, Kanehisa (1984) Nucl. Acids Res. 12:203-213, which is incorporated herein by reference. The length of homology comparison, as described, may be over longer stretches, and in certain embodiments will be over a stretch of at least about 17 nucleotides, generally at least about 20 nucleotides, ordinarily at least about 24 nucleotides, usually at least about 28 nucleotides, typically at least about 32 nucleotides, more typically at least about 40 nucleotides, preferably at least about 50 nucleotides, and more preferably at least about 75 to 100 or more nucleotides. This includes, e.g., 125, 150, 175, 200, 225, 246, 273, 300, 325, 350, 400, 450, 500, 550, 600, 650, 700, 750, 800, 850, 900, and other lengths.

Stringent conditions, in referring to homology in the hybridization context, will be stringent combined conditions of salt, temperature, organic solvents, and other parameters typically controlled in hybridization reactions. Stringent temperature conditions will usually include temperatures in excess of about 30° C, more usually in excess of about 37° C, typically in excess of about 45° C, more typically in excess of about 50° C, e.g., 55° C or 60° C, preferably in excess of about 65° C, and more preferably in excess of about 70° C. Stringent salt conditions will ordinarily be less than about 1 M, more ordinarily less than about 500 mM, usually less than about 400 mM, more usually less than about 300 mM, typically less than about 200 mM, preferably less than about 100 mM, and more preferably less than about 80 mM, e.g., less than 50 mM, even down to less than about 20 mM. However, the combination of parameters is much more important than the measure of any single parameter. See, e.g., Wetmur and Davidson (1968) J. Mol. Biol. 31:349-370, which is hereby incorporated herein by reference.

The isolated DNA can be readily modified by nucleotide substitutions, nucleotide deletions, nucleotide insertions, and inversions of nucleotide stretches. These modifications generally result in novel DNA sequences which encode this protein or its derivatives. These modified sequences can be used to produce mutant proteins (muteins) or to enhance the expression of variant species. Enhanced expression may involve gene amplification, increased transcription, increased translation, and other mechanisms. Such mutant OX2R homolog derivatives include predetermined or site-specific mutations of the protein or its fragments, including silent mutations using genetic code degeneracy. "Mutant OX2 receptor homolog" as used herein encompasses a polypeptide otherwise falling within the definition of the OX2 receptor homologs as set forth above, but having an amino acid sequence which differs from that of other Ig superfamily as found in nature, whether by way of deletion, substitution, or insertion. In particular, "site specific mutant OX2 receptor homolog" encompasses a protein having substantial sequence identity with a protein of Tables 1-3, and typically shares some or most of the biological activities or effects, e.g., immunogenicity, of the forms disclosed herein.

Although site specific mutation sites are predetermined, mutants need not be site specific. Mammalian OX2 receptor homolog mutagenesis can be achieved by making amino acid insertions or deletions in the gene, coupled with expression. Substitutions, deletions, insertions, or many combinations may be generated to arrive at a final construct. Insertions include amino- or carboxy- terminal fusions. Random mutagenesis can be conducted at a target codon and the expressed mammalian OX2RH mutants can then be screened for the desired activity, providing some aspect of a structure-activity relationship. Methods for making substitution mutations at predetermined sites in DNA having a known sequence are well known in the art, e.g., by M13 primer mutagenesis. See also Sambrook, et al. (1989) and Ausubel, et al. (1987 and periodic Supplements).

The mutations in the DNA normally should not place coding sequences out of reading frames and preferably will not create complementary regions that could hybridize to produce secondary mRNA structure such as loops or hairpins.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Polymerase chain reaction (PCR) techniques can often be applied in mutagenesis. Alternatively, mutagenesis primers are commonly used methods for generating defined mutations at predetermined sites. See, e.g., Innis, et al. (eds. 1990) PCR Protocols: A Guide to Methods and Applications Academic Press, San Diego, CA; and Dieffenbach and Dveksler (1995; eds.) PCR Primer: A Laboratory Manual Cold Spring Harbor Press, CSH, NY.

Certain embodiments of the invention are directed to combination compositions comprising the receptor homolog or ligand sequences described. In other embodiments, functional portions of the sequences may be joined to encode fusion proteins. In other forms, variants of the described sequences may be substituted.

### IV. Proteins, Peptides

As described above, the present invention encompasses mammalian OX2RH polypeptides, e.g., whose sequences are disclosed in Tables 1-3, and described above. Allelic and other variant polypeptides are also contemplated, including, e.g., fusion proteins combining portions of such sequences with others, including, e.g., epitope tags, functional domains, and DAP12 or DAP10 sequences.

The present invention also provides recombinant proteins, e.g., heterologous fusion proteins using segments from these primate or rodent proteins. A heterologous fusion protein is a fusion of proteins or segments which are naturally not normally fused in the same manner. Thus, the fusion product of two OX2RHs is a continuous protein molecule having sequences fused in a typical peptide linkage, typically made as a single translation product and exhibiting properties, e.g., sequence or antigenicity, derived from each source peptide. A similar concept applies to heterologous nucleic acid sequences. Combinations of various designated proteins into complexes are also provided.

In addition, new constructs may be made from combining similar functional or structural domains from other related proteins, e.g., other ITIM, ITAM, or YxxM motif containing receptors, including species variants. For example, ligand-binding or other segments may be "swapped" between different new fusion polypeptides or fragments. See, e.g., Cunningham, et al. (1989) Science 243:1330-1336; and O'Dowd, et al. (1988) J. Biol. Chem. 263:15985-15992, each of which is incorporated herein by reference. Thus, new chimeric polypeptides exhibiting new combinations of specificities will result from the functional linkage of receptor-binding specificities. For example, the ligand binding domains from other related receptor homolog molecules may be added or substituted for other domains of this or related proteins. The resulting protein will often have hybrid function and properties. For example, a fusion protein may include a targeting domain which may serve to provide sequestering of the fusion protein to a particular subcellular organelle.

Candidate fusion partners and sequences can be selected from various sequence data bases, e.g., GenBank, c/o IntelliGenetics, Mountain View, CA; and BCG, University of Wisconsin Biotechnology Computing Group, Madison, WI, which are each incorporated herein by reference. In particular, combinations of polypeptide sequences provided in Tables 1-3 are particularly preferred. Variant forms of the proteins may be substituted in the described combinations.

The present invention particularly provides muteins which bind OX2-like ligands, and/or which are affected in signal transduction. Structural alignment of various members of the OX2 receptor homolog family show conserved features/residues. See, e.g., Table 5. Alignment of the OX2R homolog sequences indicates various structural and functionally shared features. See also, Bazan, et al. (1996) Nature 379:591; Lodi, et al. (1994) Science 263:1762-1766; Sayle and Milner-White (1995) TIBS 20:374-376; and Gronenberg, et al. (1991) Protein Engineering 4:263-269.

Substitutions with either mouse sequences or human sequences are particularly preferred. Conversely, conservative substitutions away from the ligand binding interaction regions will probably preserve most signaling activities; and conservative substitutions away from the intracellular domains will probably preserve most ligand binding properties.

"Derivatives" of a mammalian OX2RH include amino acid sequence mutants, glycosylation variants, metabolic derivatives and covalent or aggregative conjugates with other chemical moieties. Covalent derivatives can be prepared by linkage of functionalities to groups which are found in the OX2RH amino acid side chains or at the N- or C- termini, e.g., by means which are well known in the art. These derivatives can include, without limitation, aliphatic esters or amides of the carboxyl terminus, or of residues containing carboxyl side chains, O-acyl derivatives of hydroxyl group-containing residues, and N-acyl derivatives of the amino terminal amino acid or amino-group containing residues, e.g., lysine or arginine. Acyl groups are selected from the group of alkyl-moieties, including C3 to C18 normal alkyl, thereby forming alkanoyl aroyl species.

In particular, glycosylation alterations are included, e.g., made by modifying the glycosylation patterns of a polypeptide during its synthesis and processing, or in further processing steps. Particularly preferred means for accomplishing this are by exposing the polypeptide to glycosylating enzymes derived from cells which normally provide such processing, e.g., mammalian glycosylation enzymes. Deglycosylation enzymes are also contemplated. Also embraced are versions of the same primary amino acid sequence which have other minor modifications, including phosphorylated amino acid residues, e.g., phosphotyrosine, phosphoserine, or phosphothreonine.

A major group of derivatives are covalent conjugates of the receptor homologs or fragments thereof with other proteins of polypeptides. These derivatives can be synthesized in recombinant culture such as N- or C-terminal fusions or by the use of agents known in the art for their usefulness in cross-linking proteins through reactive side groups. Preferred derivatization sites with cross-linking agents are at free amino groups, carbohydrate moieties, and cysteine residues.

Fusion polypeptides between the receptor homologs and other homologous or heterologous proteins are also provided. Homologous polypeptides may be fusions between different receptors, resulting in, for instance, a hybrid protein exhibiting binding specificity for multiple different OX2 related ligands, or a receptor which may have broadened or weakened specificity of substrate effect. Likewise, heterologous fusions may be constructed which would exhibit a combination of properties or activities of the derivative proteins. Typical examples are fusions of a reporter polypeptide, e.g., luciferase, with a segment or domain of a receptor, e.g., a ligand-binding segment, so that the presence or location of a desired ligand may be easily determined. See, e.g., Dull, et al., U.S. Patent No. 4,859,609, which is hereby incorporated herein by reference. Other gene fusion partners include glutathione-S-transferase (GST), bacterial β-galactosidase, trpE, Protein A, β-lactamase, alpha amylase, alcohol dehydrogenase, and yeast alpha mating factor. See, e.g., Godowski, et al. (1988) Science 241:812-816. Labeled proteins will often be substituted in the described combinations of proteins.

The phosphoramidite method described by Beaucage and Carruthers (1981) Tetra. Letts. 22:1859-1862, will produce suitable synthetic DNA fragments. A double stranded fragment will often be obtained either by synthesizing the complementary strand and annealing the strand together under appropriate conditions or by adding the complementary strand using DNA polymerase with an appropriate primer sequence.

Such polypeptides may also have amino acid residues which have been chemically modified by phosphorylation, sulfonation, biotinylation, or the addition or removal of other moieties, particularly those which have molecular shapes similar to phosphate groups. In some embodiments, the modifications will be useful labeling reagents, or serve as purification targets, e.g., affinity ligands.

Fusion proteins will typically be made by either recombinant nucleic acid methods or by synthetic polypeptide methods. Techniques for nucleic acid manipulation and expression are described generally, for example, in Sambrook, et al. (1989) Molecular Cloning: A Laboratory Manual (2d ed.), Vols. 1-3, Cold Spring Harbor Laboratory, and Ausubel, et al. (eds. 1987 and periodic supplements) Current Protocols in Molecular Biology, Greene/Wiley, New York, which are each incorporated herein by reference. Techniques for synthesis of polypeptides are described, for example, in Merrifield (1963) J. Amer. Chem. Soc. 85:2149-2156; Merrifield (1986) Science 232:341-347; and Atherton, et al. (1989) Solid Phase Peptide Synthesis: A Practical Approach, IRL Press, Oxford; each of which is incorporated herein by reference. See also Dawson, et al. (1994) Science 266:776-779 for methods to make larger polypeptides.

This invention also contemplates the use of derivatives of an OX2RH other than variations in amino acid sequence or glycosylation. Such derivatives may involve covalent or aggregative association with chemical moieties. These derivatives generally fall into three classes: (1) salts, (2) side chain and terminal residue covalent modifications, and (3) adsorption complexes, e.g., with cell membranes. Such covalent or aggregative derivatives are useful as immunogens, as reagents in immunoassays, or in purification methods such as for affinity purification of a receptor or other binding molecule, e.g., an antibody. For example, an OX2 ligand can be immobilized by covalent bonding to a solid support such as cyanogen bromide-activated Sepharose, by methods which are well known in the art, or adsorbed onto polyolefin surfaces, with or without glutaraldehyde cross-linking, for use in the assay or purification of an OX2RH, antibodies, or other similar molecules. The ligand can also be labeled with a detectable group, for example radioiodinated by the chloramine T procedure, covalently bound to rare earth chelates, or conjugated to another fluorescent moiety for use in diagnostic assays.

A combination, e.g., including an OX2RH, of this invention can be used as an immunogen for the production of antisera or antibodies specific, e.g., capable of distinguishing between other OX2 receptor homologs, or for desired combination specificity. The OX2RHs can be used to screen monoclonal antibodies or antigen-binding fragments prepared by immunization with various forms of impure preparations containing the protein. In particular, the term "antibodies" also encompasses antigen binding fragments of natural antibodies, e.g., Fab, Fab2, Fv, etc. The purified OX2RH can also be used as a reagent to detect antibodies generated in response to the presence of elevated levels of expression, or immunological disorders which lead to antibody production to the endogenous receptor homolog. Additionally, OX2RH fragments may also serve as immunogens to produce the antibodies of the present invention, as described immediately below. For example, this invention contemplates antibodies having binding affinity to or being raised against the amino acid sequences shown in Tables 1-3, fragments thereof, or various homologous peptides or subsets. In particular, this invention contemplates antibodies having binding affinity to, or having been raised against, specific fragments which are predicted to be, or actually are, exposed at the exterior protein surface of the native OX2RH. Complexes of combinations of proteins will also be useful, and antibody preparations thereto can be made.

The blocking of physiological response to the receptor ligands may result from the inhibition of binding of the ligand to the receptor, likely through competitive inhibition, or perhaps down-regulation of receptor expression due to antibody binding. Thus, in vitro assays of the present invention will often use antibodies or antigen binding segments of these antibodies, or fragments attached to solid phase substrates. These assays will also allow for the diagnostic determination of the effects of either ligand binding region mutations and modifications, or other mutations and modifications, e.g., which affect signaling or enzymatic function.

This invention also contemplates the use of competitive drug screening assays, e.g., where neutralizing antibodies to the receptor complexes or fragments compete with a test compound for binding to a ligand or other antibody. In this manner, the neutralizing antibodies or fragments can be used to detect the presence of a polypeptide which shares one or more binding sites to a receptor and can also be used to occupy binding sites on a receptor that might otherwise bind a ligand.

### V. Making Nucleic Acids and Protein

DNA which encodes the protein or fragments thereof can be obtained by chemical synthesis, screening cDNA libraries, or by screening genomic libraries prepared from a wide variety of cell lines or tissue samples. Natural sequences can be isolated using standard methods and the sequences provided herein, e.g., in Tables 1-3. Reverse translation sequences are provided in Table 4. Other species counterparts can be identified by hybridization techniques, or by various PCR techniques, combined with or by searching in sequence databases, e.g., GenBank. Antibodies may be used in expression cloning efforts on species counterparts.

This DNA can be expressed in a wide variety of host cells for the synthesis of a full-length receptor or fragments which can, e.g., be used to generate polyclonal or monoclonal antibodies; for binding studies; for construction and expression of modified ligand binding or kinase/phosphatase domains; and for structure/function studies. Variants or fragments can be expressed in host cells that are transformed or transfected with appropriate expression vectors. These molecules can be substantially free of protein or cellular contaminants, other than those derived from the recombinant host, and therefore are particularly useful in pharmaceutical compositions when combined with a pharmaceutically acceptable carrier and/or diluent. The protein, or portions thereof, may be expressed as fusions with other proteins. Combinations of the described proteins, or nucleic acids encoding them, are particularly interesting.

Expression vectors are typically self-replicating DNA or RNA constructs containing the desired receptor homolog gene or its fragments, usually operably linked to suitable genetic control elements that are recognized in a suitable host cell. These control elements are capable of effecting expression within a suitable host. The multiple genes may be coordinately expressed, and may be on a polycistronic message. The specific type of control elements necessary to effect expression will depend upon the eventual host cell used. Generally, the genetic control elements can include a prokaryotic promoter system or a eukaryotic promoter expression control system, and typically include a transcriptional promoter, an optional operator to control the onset of transcription, transcription enhancers to elevate the level of mRNA expression, a sequence that encodes a suitable ribosome binding site, and sequences that terminate transcription and translation. Expression vectors also usually contain an origin of replication that allows the vector to replicate independently of the host cell.

The vectors of this invention include those which contain DNA which encodes a combination of proteins, as described, or a biologically active equivalent polypeptide. The DNA can be under the control of a viral promoter and can encode a selection marker. This invention further contemplates use of such expression vectors which are capable of expressing eukaryotic cDNAs coding for such proteins in a prokaryotic or eukaryotic host, where the vector is compatible with the host and where the eukaryotic cDNAs are inserted into the vector such that growth of the host containing the vector expresses the cDNAs in question. Usually, expression vectors are designed for stable replication in their host cells or for amplification to greatly increase the total number of copies of the desirable gene per cell. It is not always necessary to require that an expression vector replicate in a host cell, e.g., it is possible to effect transient expression of the protein or its fragments in various hosts using vectors that do not contain a replication origin that is recognized by the host cell. It is also possible to use vectors that cause integration of the protein encoding portions into the host DNA by recombination.

Vectors, as used herein, comprise plasmids, viruses, bacteriophage, integratable DNA fragments, and other vehicles which enable the integration of DNA fragments into the genome of the host. Expression vectors are specialized vectors which contain genetic control elements that effect expression of operably linked genes. Plasmids are the most commonly used form of vector but all other forms of vectors which serve an equivalent function and which are, or become, known in the art are suitable for use herein. See, e.g., Pouwels, et al. (1985 and Supplements) Cloning Vectors: A Laboratory Manual, Elsevier, N.Y., and Rodriguez, et al. (eds. 1988) Vectors: A Survey of Molecular Cloning Vectors and Their Uses, Buttersworth, Boston, which are incorporated herein by reference.

Transformed cells are cells, preferably mammalian, that have been transformed or transfected with vectors constructed using recombinant DNA techniques. Transformed host cells usually express the desired proteins, but for purposes of cloning, amplifying, and manipulating its DNA, do not need to express the subject proteins. This invention further contemplates culturing transformed cells in a nutrient medium, thus permitting the proteins to accumulate. The proteins can be recovered, either from the culture or, in certain instances, from the culture medium.

For purposes of this invention, nucleic acid sequences are operably linked when they are functionally related to each other. For example, DNA for a presequence or secretory leader is operably linked to a polypeptide if it is expressed as a preprotein or participates in directing the polypeptide to the cell membrane or in secretion of the polypeptide. A promoter is operably linked to a coding sequence if it controls the transcription of the polypeptide; a ribosome binding site is operably linked to a coding sequence if it is positioned to permit translation. Usually, operably linked means contiguous and in reading frame, however, certain genetic elements such as repressor genes are not contiguously linked but still bind to operator sequences that in turn control expression.

Suitable host cells include prokaryotes, lower eukaryotes, and higher eukaryotes. Prokaryotes include both gram negative and gram positive organisms, e.g., E. coli and B. subtilis. Lower eukaryotes include yeasts, e.g., S. cerevisiae and Pichia, and species of the genus Dictyostelium. Higher eukaryotes include established tissue culture cell lines from animal cells, both of non-mammalian origin, e.g., insect cells, and birds, and of mammalian origin, e.g., human, primates, and rodents.

Prokaryotic host-vector systems include a wide variety of vectors for many different species. E. coli and its vectors will be described, but equivalent vectors and hosts can generally be substituted. A representative vector for amplifying DNA is pBR322 or many of its derivatives. Vectors that can be used to express the receptor homolog or its fragments include, but are not limited to, such vectors as those containing the lac promoter (pUC-series); trp promoter (pBR322-trp); Ipp promoter (the pIN-series); lambda-pP or pR promoters (pOTS); or hybrid promoters such as ptac (pDR540). See Brosius, et al. (1988) "Expression Vectors Employing Lambda-, trp-, lac-, and Ipp-derived Promoters", in Vectors: A Survey of Molecular Cloning Vectors and Their Uses, (eds. Rodriguez and Denhardt), Buttersworth, Boston, Chapter 10, pp. 205-236, which is incorporated herein by reference.

Lower eukaryotes, e.g., yeasts and Dictyostelium, may be transformed with OX2RH sequence containing vectors. The most popular lower eukaryotic host is the baker's yeast, Saccharomyces cerevisiae. It will be used to exemplify lower eukaryotes, though many other strains and species are also available. Yeast vectors typically consist of a replication origin (unless of the integrating type), a selection gene, a promoter, DNA encoding the receptor homolog or its fragments, and sequences for translation termination, polyadenylation, and transcription termination. Suitable expression vectors for yeast include such constitutive promoters as 3-phosphoglycerate kinase and various other glycolytic enzyme gene promoters or such inducible promoters as the alcohol dehydrogenase 2 promoter or metallothionine promoter. Suitable vectors include derivatives of the following types: self-replicating low copy number (such as the YRp-series), self-replicating high copy number (such as the YEp-series); integrating types (such as the YIp-series), or mini-chromosomes (such as the YCp-series).

Higher eukaryotic tissue culture cells are normally the preferred host cells for expression of functionally active OX2RH proteins. In principle, many higher eukaryotic tissue culture cell lines are workable, e.g., insect baculovirus expression systems, whether from an invertebrate or vertebrate source. However, mammalian cells are preferred. Transformation or transfection and propagation of such cells has become a routine procedure. Examples of useful cell lines include HeLa cells, Chinese hamster ovary (CHO) cell lines, baby rat kidney (BRK) cell lines, insect cell lines, bird cell lines, and monkey (COS) cell lines. Expression vectors for such cell lines usually include an origin of replication, a promoter, a translation initiation site, RNA splice sites (if genomic DNA is used), a polyadenylation site, and a transcription termination site. These vectors also usually contain a selection gene or amplification gene. Suitable expression vectors may be plasmids, viruses, or retroviruses carrying promoters derived, e.g., from such sources as from adenovirus, SV40, parvoviruses, vaccinia virus, or cytomegalovirus. Representative examples of suitable expression vectors include pCDNA1; pCD, see Okayama, et al. (1985) Mol. Cell Biol. 5:1136-1142; pMC1neo PolyA, see Thomas, et al. (1987) Cell 51:503-512; and a baculovirus vector such as pAC 373 or pAC 610.

For secreted proteins and some membrane proteins, an open reading frame usually encodes a polypeptide that consists of a mature or secreted product covalently linked at its N-terminus to a signal peptide. The signal peptide is cleaved prior to secretion of the mature, or active, polypeptide. The cleavage site can be predicted with a high degree of accuracy from empirical rules, e.g., von-Heijne (1986) Nucleic Acids Research 14:4683-4690, and Nielsen, et al. (1997) Protein Eng. 10:1-12. And the precise amino acid composition of the signal peptide often does not appear to be critical to its function, e.g., Randall, et al. (1989) Science 243:1156-1159; Kaiser, et al. (1987) Science 235:312-317. The mature proteins of the invention can be readily determined using standard methods.

It will often be desired to express these polypeptides in a system which provides a specific or defined glycosylation pattern. In this case, the usual pattern will be that provided naturally by the expression system. However, the pattern will be modifiable by exposing the polypeptide, e.g., an unglycosylated form, to appropriate glycosylating proteins introduced into a heterologous expression system. For example, the OX2RH gene may be co-transformed with one or more genes encoding mammalian or other glycosylating enzymes. Using this approach, certain mammalian glycosylation patterns will be achievable in prokaryote or other cells. Expression in prokaryote cells will typically lead to unglycosylated forms of protein.

The source of OX2RH can be a eukaryotic or prokaryotic host expressing recombinant polypeptide, such as is described above. The source can also be a cell line, but other mammalian cell lines are also contemplated by this invention, with the preferred cell line being from the human species.

Now that the sequences are known, the primate OX2RH, fragments, or derivatives thereof can be prepared by conventional processes for synthesizing peptides. These include processes such as are described in Stewart and Young (1984) Solid Phase Peptide Synthesis, Pierce Chemical Co., Rockford, IL; Bodanszky and Bodanszky (1984) The Practice of Peptide Synthesis, Springer-Verlag, New York; and Bodanszky (1984) The Principles of Peptide Synthesis, Springer-Verlag, New York; all of each which are incorporated herein by reference. For example, an azide process, an acid chloride process, an acid anhydride process, a mixed anhydride process, an active ester process (for example, p-nitrophenyl ester, N-hydroxysuccinimide ester, or cyanomethyl ester), a carbodiimidazole process, an oxidative-reductive process, or a dicyclohexylcarbodiimide (DCCD)/additive process can be used. Solid phase and solution phase syntheses are both applicable to the foregoing processes. Similar techniques can be used with partial OX2RH sequences.

The OX2RH proteins, fragments, or derivatives are suitably prepared in accordance with the above processes as typically employed in peptide synthesis, generally either by a so-called stepwise process which comprises condensing an amino acid to the terminal amino acid, one by one in sequence, or by coupling peptide fragments to the terminal amino acid. Amino groups that are not being used in the coupling reaction typically must be protected to prevent coupling at an incorrect location.

If a solid phase synthesis is adopted, the C-terminal amino acid is bound to an insoluble carrier or support through its carboxyl group. The insoluble carrier should have a binding capability to a reactive carboxyl group, e.g., halomethyl resins, such as chloromethyl resin or bromomethyl resin, hydroxymethyl resins, phenol resins, tert-alkyloxycarbonylhydrazidated resins, and the like.

An amino group-protected amino acid is bound in sequence through condensation of its activated carboxyl group and the reactive amino group of the previously formed peptide or chain, to synthesize the peptide step by step. After synthesizing the complete sequence, the peptide is split off from the insoluble carrier to produce the peptide. This solid-phase approach is generally described by Merrifield, et al. (1963) in J. Am. Chem. Soc. 85:2149-2156, which is incorporated herein by reference.

The prepared protein and fragments thereof can be isolated and purified from the reaction mixture by means of peptide separation, e.g., by extraction, precipitation, electrophoresis, various forms of chromatography, and the like. The receptor homologs of this invention can be obtained in varying degrees of purity depending upon desired uses. Purification can be accomplished using standard protein purification techniques or the antibodies herein described in immunoabsorbant affinity chromatography methods. Typically, affinity chromatography is carried out by first linking the antibodies to a solid support and then contacting the linked antibodies with solubilized lysates of appropriate cells, lysates of other cells expressing the OX2RH, or lysates or supernatants of cells producing the protein as a result of DNA techniques, see below.

Generally, the purified protein will be at least about 40% pure, ordinarily at least about 50% pure, usually at least about 60% pure, typically at least about 70% pure, more typically at least about 80% pure, preferable at least about 90% pure and more preferably at least about 95% pure, and in particular embodiments, 97%-99% or more. Purity will usually be on a weight basis, but can also be on a molar basis. Different assays will be applied as appropriate. Individual proteins may be purified and thereafter combined.

### VI. Antibodies

Antibodies can be raised to the various mammalian, e.g., primate, OX2RH proteins and fragments thereof, both in naturally occurring native forms and in their denatured forms. Antibodies raised to native OX2RH are more likely to recognize epitopes which are only present in the native conformations. Denatured antigen detection can also be useful in, e.g., Western analysis. Anti-idiotypic antibodies are also contemplated, which would be useful, e.g., diagnostic reagents.

Antibodies, including binding fragments and single chain versions, against predetermined fragments of the protein can be raised by immunization of animals with conjugates of the fragments with immunogenic proteins. Monoclonal antibodies are prepared from cells secreting the desired antibody. These antibodies can be screened for binding to normal or defective protein, or screened for agonistic or antagonistic activity. These monoclonal antibodies will usually bind with at least a K_{D} of about 1 mM, more usually at least about 300 µM, typically at least about 100µM, more typically at least about 30 µM, preferably at least about 10 µM, and more preferably at least about 3 µM or better.

The antibodies, including antigen binding fragments, of this invention can have significant diagnostic or therapeutic value. They can be potent antagonists that bind to a receptor homolog and inhibit binding to ligand or inhibit the ability of the receptor homolog to elicit a biological response, e.g., act on its substrate. They also can be useful as non-neutralizing antibodies and can be coupled to toxins or radionuclides to bind OX2RH producing cells. Further, these antibodies can be conjugated to drugs or other therapeutic agents, either directly or indirectly, e.g., by means of a linker.

The antibodies of this invention can also be useful in diagnostic applications. As capture or non-neutralizing antibodies, they might bind to the OX2RH without inhibiting ligand or substrate binding. As neutralizing antibodies, they can be useful in competitive binding assays. They will also be useful in detecting or quantifying ligand. They may be used as reagents for Western blot analysis, or for immunoprecipitation or immunopurification of the respective protein. Likewise, nucleic acids and proteins may be immobilized to solid substrates for affinity purification or detection methods. The substrates may be, e.g., solid resin beads, sheets of plastic, or derivatized glass.

Protein fragments may be joined to other materials, particularly polypeptides, as fused or covalently joined polypeptides, to be used as immunogens. Mammalian OX2RH polypeptides and fragments may be fused or covalently linked to a variety of immunogens, such as keyhole limpet hemocyanin, bovine serum albumin, tetanus toxoid, etc. See Microbiology, Hoeber Medical Division, Harper and Row, 1969; Landsteiner (1962) Specificity of Serological Reactions, Dover Publications, New York; and Williams, et al. (1967) Methods in Immunology and Immunochemistry, Vol. 1, Academic Press, New York; each of which are incorporated herein by reference. A typical method involves hyperimmunization of an animal with an antigen. The blood of the animal is then collected shortly after the repeated immunizations and serum or gamma globulin is isolated.

In some instances, it is desirable to prepare monoclonal antibodies from various mammalian hosts, such as mice, rodents, primates, humans, etc. See, e.g., Stites, et al. (eds.) Basic and Clinical Immunology (4th ed.), Lange Medical Publications, Los Altos, CA, and references cited therein; Harlow and Lane (1988) Antibodies: A Laboratory Manual, CSH Press; Goding (1986) Monoclonal Antibodies: Principles and Practice (2d ed.) Academic Press, New York; and particularly Kohler and Milstein (1975) Nature 256:495-497, each of these references is incorporated herein by reference. Briefly, an immunogen is injected into an animal to induce an immune response. The animal is then sacrificed and cells taken from its spleen, which are fused with myeloma cells to produce a hybridoma. The population of hybridomas is then screened to isolate an individual clone which secretes an antibody which binds to the immunogen.

Other suitable techniques involve in vitro exposure of lymphocytes to the antigenic polypeptides or alternatively to selection of libraries of antibodies in phage or similar vectors. See, Huse, et al. (1989) "Generation of a Large Combinatorial Library of the Immunoglobulin Repertoire in Phage Lambda," Science 246:1275-1281; and Ward, et al. (1989) Nature 341:544-546, each of which is hereby incorporated herein by reference. Chimeric or humanized antibodies may be produced, see Cabilly, U.S. Patent No. 4,816,567; or made in transgenic mice, see Mendez, et al. (1997) Nature Genetics 15:146-156. These references are incorporated herein by reference.

Polypeptides and antibodies will often be labeled. A wide variety of labels and conjugation techniques are known and are reported extensively in both the scientific and patent literature. Suitable labels include radionuclides, enzymes, substrates, cofactors, inhibitors, fluorescent moieties, chemiluminescent moieties, magnetic particles, and the like. Patents teaching the use of such labels include, e.g., U.S. Patent Nos. 3,817,837; 3,850,752; 3,939,350; 3,996,345; 4,277,437; 4,275,149; and 4,366,241.

The antibodies of this invention can also be used for affinity chromatography in isolating the OX2RH proteins or peptides. Columns can be prepared where the antibodies are linked to a solid support, e.g., particles, such as agarose, Sephadex, or the like, where a cell lysate may be passed through the column, the column washed, followed by increasing concentrations of a mild denaturant, whereby the purified protein will be released. Alternatively, the protein may be used to purify antibody. Appropriate cross absorptions or depletions may be applied.

The antibodies may also be used to screen expression libraries for particular expression products. Usually the antibodies used in such a procedure will be labeled with a moiety allowing easy detection of presence of antigen by antibody binding.

Antibodies raised against an OX2RH will also be used to raise anti-idiotypic antibodies. These will be useful in detecting or diagnosing various immunological conditions related to expression of the protein or cells which express the protein. They also will be useful as agonists or antagonists of the ligand, which may be competitive inhibitors or substitutes for naturally occurring ligands.

A receptor homolog protein that specifically binds to or that is specifically immunoreactive with an antibody generated against a defined immunogen, such as an immunogen consisting of the amino acid sequence of SEQ ID NO: 2, is typically determined in an immunoassay. The immunoassay typically uses a polyclonal antiserum which was raised, e.g., to a protein of SEQ ID NO: 2. This antiserum is selected to have low crossreactivity against other Ig superfamily receptor members, e.g., NKG2D, preferably from the same species, and any such crossreactivity is removed by immunoabsorption prior to use in the immunoassay.

In order to produce antisera for use in an immunoassay, the protein, e.g., of SEQ ID NO: 2, is isolated as described herein. For example, recombinant protein may be produced in a mammalian cell line. An appropriate host, e.g., an inbred strain of mice such as Balb/c, is immunized with the selected protein, typically using a standard adjuvant, such as Freund's adjuvant, and a standard mouse immunization protocol (see Harlow and Lane, supra). Alternatively, a synthetic peptide derived from the sequences disclosed herein and conjugated to a carrier protein can be used an immunogen. Polyclonal sera are collected and titered against the immunogen protein in an immunoassay, e.g., a solid phase immunoassay with the immunogen immobilized on a solid support. Polyclonal antisera with a titer of 10⁴ or greater are selected and tested for their cross reactivity against other Ig superfamily receptor members using a competitive binding immunoassay such as the one described in Harlow and Lane, supra, at pages 570-573. Preferably at least two receptor family members are used in this determination. These receptor family members can be produced as recombinant proteins and isolated using standard molecular biology and protein chemistry techniques as described herein.

Immunoassays in the competitive binding format can be used for the crossreactivity determinations. For example, the protein of SEQ ID NO: 2 can be immobilized to a solid support. Proteins added to the assay compete with the binding of the antisera to the immobilized antigen. The ability of the above proteins to compete with the binding of the antisera to the immobilized protein is compared to the proteins. The percent crossreactivity for the above proteins is calculated, using standard calculations. Those antisera with less than 10% crossreactivity with each of the proteins listed above are selected and pooled. The cross-reacting antibodies are then removed from the pooled antisera by immunoabsorption with the above-listed proteins.

The immunoabsorbed and pooled antisera are then used in a competitive binding immunoassay as described above to compare a second protein to the immunogen protein (e.g., the OX2RH1 like protein of SEQ ID NO: 2). In order to make this comparison, the two proteins are each assayed at a wide range of concentrations and the amount of each protein required to inhibit 50% of the binding of the antisera to the immobilized protein is determined. If the amount of the second protein required is less than twice the amount of the protein of the selected protein or proteins that is required, then the second protein is said to specifically bind to an antibody generated to the immunogen.

It is understood that these OX2 receptor homolog proteins are members of a family of homologous proteins that comprise at least 6 so far identified genes. For a particular gene product, such as the OX2RH1, the term refers not only to the amino acid sequences disclosed herein, but also to other proteins that are allelic, non-allelic, or species variants. It is also understood that the terms include nonnatural mutations introduced by deliberate mutation using conventional recombinant technology such as single site mutation, or by excising short sections of DNA encoding the respective proteins, or by substituting new amino acids, or adding new amino acids. Such minor alterations typically will substantially maintain the immunoidentity of the original molecule and/or its biological activity. Thus, these alterations include proteins that are specifically immunoreactive with a designated naturally occurring OX2RH protein. The biological properties of the altered proteins can be determined by expressing the protein in an appropriate cell line and measuring the appropriate effect, e.g., upon transfected lymphocytes. Particular protein modifications considered minor would include conservative substitution of amino acids with similar chemical properties, as described above for the receptor homolog family as a whole. By aligning a protein optimally with the protein of the receptor homologs and by using the conventional immunoassays described herein to determine immunoidentity, one can determine the protein compositions of the invention.

### VII. Kits and quantitation

Both naturally occurring and recombinant forms of the receptor like molecules of this invention are particularly useful in kits and assay methods. For example, these methods would also be applied to screening for binding activity, e.g., ligands for these proteins. Several methods of automating assays have been developed in recent years so as to permit screening of tens of thousands of compounds per year. See, e.g., a BIOMEK automated workstation, Beckman Instruments, Palo Alto, California, and Fodor, et al. (1991) Science 251:767-773, which is incorporated herein by reference. The latter describes means for testing binding by a plurality of defined polymers synthesized on a solid substrate. The development of suitable assays to screen for a ligand or agonist/antagonist homologous proteins can be greatly facilitated by the availability of large amounts of purified, soluble receptors in an active state such as is provided by this invention.

Purified OX2RH can be coated directly onto plates for use in the aforementioned ligand screening techniques. However, non-neutralizing antibodies to these proteins can be used as capture antibodies to immobilize the respective receptor homolog on the solid phase, useful, e.g., in diagnostic uses.

This invention also contemplates use of OX2RH, fragments thereof, peptides, and their fusion products in a variety of diagnostic kits and methods for detecting the presence of the protein or its ligand. Alternatively, or additionally, antibodies against the molecules may be incorporated into the kits and methods and may be used in quantitating the OX2RH or cells expressing them. Typically the kit will have a compartment containing either an OX2RH peptide or gene segment or a reagent which recognizes one or the other. Typically, recognition reagents, in the case of peptide, would be a receptor homolog or antibody, or in the case of a gene segment, would usually be a hybridization probe.

A preferred kit for determining the concentration of OX2RH in a sample would typically comprise a labeled compound, e.g., ligand or antibody, having known binding affinity for OX2RH, a source of OX2RH (naturally occurring or recombinant) as a positive control, and a means for separating the bound from free labeled compound, for example a solid phase for immobilizing the OX2RH in the test sample. Compartments containing reagents, and instructions, will normally be provided. Appropriate nucleic acid or protein containing kits are also provided.

Antibodies, including antigen binding fragments, specific for mammalian OX2RH or a peptide fragment, or receptor homolog fragments are useful in diagnostic applications to detect the presence of elevated levels of homolog and/or its fragments. Diagnostic assays may be homogeneous (without a separation step between free reagent and antibody-antigen complex) or heterogeneous (with a separation step). Various commercial assays exist, such as radioimmunoassay (RIA), enzyme-linked immunosorbent assay (ELISA), enzyme immunoassay (EIA), enzyme-multiplied immunoassay technique (EMIT), substrate-labeled fluorescent immunoassay (SLFIA) and the like. For example, unlabeled antibodies can be employed by using a second antibody which is labeled and which recognizes the antibody to a receptor homolog or to a particular fragment thereof. These assays have also been extensively discussed in the literature. See, e.g., Harlow and Lane (1988) Antibodies: A Laboratory Manual, CSH., and Coligan (ed. 1991 and periodic supplements) Current Protocols In Immunology Greene/Wiley, New York.

Anti-idiotypic antibodies may have similar use to serve as agonists or antagonists of the receptor homologs. These should be useful as therapeutic reagents under appropriate circumstances.

Frequently, the reagents for diagnostic assays are supplied in kits, so as to optimize the sensitivity of the assay. For the subject invention, depending upon the nature of the assay, the protocol, and the label, either labeled or unlabeled antibody, or labeled ligand is provided. This is usually in conjunction with other additives, such as buffers, stabilizers, materials necessary for signal production such as substrates for enzymes, and the like. Preferably, the kit will also contain instructions for proper use and disposal of the contents after use. Typically the kit has compartments for each useful reagent, and will contain instructions for proper use and disposal of reagents. Desirably, the reagents are provided as a dry lyophilized powder, where the reagents may be reconstituted in an aqueous medium having appropriate concentrations for performing the assay.

The aforementioned constituents of the diagnostic assays may be used without modification or may be modified in a variety of ways. For example, labeling may be achieved by covalently or non-covalently joining a moiety which directly or indirectly provides a detectable signal. In many of these assays, a test compound, receptor homolog, or antibodies thereto can be labeled either directly or indirectly. Possibilities for direct labeling include label groups: radiolabels such as ¹²⁵I, enzymes (U.S. Pat. No. 3,645,090) such as peroxidase and alkaline phosphatase, and fluorescent labels (U.S. Pat. No. 3, 940, 475) capable of monitoring the change in fluorescence intensity, wavelength shift, or fluorescence polarization. Both of the patents are incorporated herein by reference. Possibilities for indirect labeling include biotinylation of one constituent followed by binding to avidin coupled to one of the above label groups. There are also numerous methods of separating the bound from the free ligand, or alternatively the bound from the free test compound. The receptor homolog can be immobilized on various matrixes followed by washing. Suitable matrices include plastic such as an ELISA plate, filters, and beads. Methods of immobilizing the receptor homolog to a matrix include, without limitation, direct adhesion to plastic, use of a capture antibody, chemical coupling, and biotin-avidin. The last step in this approach involves the precipitation of antibody/antigen complex by any of several methods including those utilizing, e.g., an organic solvent such as polyethylene glycol or a salt such as ammonium sulfate. Other suitable separation techniques include, without limitation, the fluorescein antibody magnetizable particle method described in Rattle, et al. (1984) Clin. Chem. 30(9):1457-1461, and the double antibody magnetic particle separation as described in U.S. Pat. No. 4,659,678, each of which is incorporated herein by reference.

The methods for linking protein or fragments to various labels have been extensively reported in the literature and do not require detailed discussion here. Many of the techniques involve the use of activated carboxyl groups either through the use of carbodiimide or active esters to form peptide bonds, the formation of thioethers by reaction of a mercapto group with an activated halogen such as chloroacetyl, or an activated olefin such as maleimide, for linkage, or the like. Fusion proteins will also find use in these applications.

Another diagnostic aspect of this invention involves use of oligonucleotide or polynucleotide sequences taken from the sequence of a receptor homolog. These sequences can be used as probes for detecting levels of the respective receptor homolog in patients suspected of having an immunological disorder. The preparation of both RNA and DNA nucleotide sequences, the labeling of the sequences, and the preferred size of the sequences has received ample description and discussion in the literature. Normally an oligonucleotide probe should have at least about 14 nucleotides, usually at least about 18 nucleotides, and the polynucleotide probes may be up to several kilobases. Various labels may be employed, most commonly radionuclides, particularly ³²P. However, other techniques may also be employed, such as using biotin modified nucleotides for introduction into a polynucleotide. The biotin then serves as the site for binding to avidin or antibodies, which may be labeled with a wide variety of labels, such as radionuclides, fluorescers, enzymes, or the like. Alternatively, antibodies may be employed which can recognize specific duplexes, including DNA duplexes, RNA duplexes, DNA-RNA hybrid duplexes, or DNA-protein duplexes. The antibodies in turn may be labeled and the assay carried out where the duplex is bound to a surface, so that upon the formation of duplex on the surface, the presence of antibody bound to the duplex can be detected. The use of probes to the novel anti-sense RNA may be carried out in conventional techniques such as nucleic acid hybridization, plus and minus screening, recombinational probing, hybrid released translation (HRT), and hybrid arrested translation (HART). This also includes amplification techniques such as polymerase chain reaction (PCR).

Diagnostic kits which also test for the qualitative or quantitative presence of other markers are also contemplated. Diagnosis or prognosis may depend on the combination of multiple indications used as markers. Thus, kits may test for combinations of markers. See, e.g., Viallet, et al. (1989) Progress in Growth Factor Res. 1:89-97.

### VIII. Therapeutic Utility

This invention provides reagents with significant therapeutic value. See, e.g., Levitzki (1996) Curr. Opin. Cell Biol. 8:239-244. The receptor homologs (naturally occurring or recombinant), fragments thereof, mutein receptors, and antibodies, along with compounds identified as having binding affinity to the receptor homologs or antibodies, should be useful in the treatment of conditions wherein modulation of function of myeloid lineage cells particularly is desirable. Such abnormality will typically be manifested by immunological disorders, but also by conditions in which myeloid cell activities impact physiological processes, e.g., CNS maturation or development, etc. Additionally, this invention should provide therapeutic value in various diseases or disorders associated with abnormal expression or abnormal triggering of response to the ligand.

In cases where leukocytes, including macrophage/myeloid lineage cells, expressing the OX2R are involved in pathologies and contribute to the disease process, it may be desirable to inhibit the function of these cells. This may be achieved by appropriate stimulation of an OX2R, such that the cell-inhibitory activities of receptor signalling are mobilized. This may be achieved using, e.g., a ligand OX2 agonist or an antibody to the OX2R that has agonistic activities for the receptor. Suitable conditions would be where the animal exhibits signs or symptoms of an inflammatory, leukoproliferative, neurodegenerative, or post-traumatic condition. Preferred embodiments include where the sign or symptom is in neural tissue; lymphoid tissue; myeloid tissue; pancreas; gastrointestinal tissue; thyroid tissue; muscle tissue; or skin or collagenous tissue. Certain embodiments include where the animal is experiencing signs or symptoms of autoimmunity; an inflammatory condition; tissue specific autoimmunity; degenerative autoimmunity; rheumatoid arthritis; atherosclerosis; multiple sclerosis; vasculitides; delayed hypersensitivities; skin grafting; a transplant; spinal injury; stroke; neurodegeneration; or ischemia. The administering agent may be in combination with: an anti-inflammatory cytokine agonist or antagonist; an analgesic; an anti-inflammatory agent; or a steroid.

By contrast, in cases where leukocytes, including macrophage/myeloid lineage cells, expressing the OX2R are involved in processes of immunization and vaccination, repair mechanisms, limiting pathologies, or controlling infection, particularly of bacterial infections, it may be desirable to enhance the function of these cells. This may be achieved therapeutically by appropriate stimulation of an OX2R, such that the cell-activation activities of receptor signalling are mobilized, or by blocking OX2-OX2R interactions completely should this enable cell-activation to proceed. The latter occurs in ligand OX2 gene knockout mice where the lack of ligand OX2 leads to myeloid cell activation. This may be achieved using, e.g., a ligand OX2 antagonist (such as an antibody against ligand OX2), an antibody to the OX2R that prevents OX2-OX2R interactions, antisense nucleic acids which may prevent OX2R expression, an Ig-OX2R fusion protein that, e.g., by competitive binding, blocks the capacity of cell-bound OX2 to interact with cell-bound OX2R, or a small molecule antagonist. That this modality has applications in vivo in promotion of myeloid cell functions has been demonstrated by experiment, e.g., where i.v. injection to mice of an adenovirus construct producing a human IgG-mouse OX2RH1 fusion protein known to bind mouse OX2 resulted in accelerated onset of the autoimmune disease experimental autoimmune encephalomyelitis (EAE) in these mice, as compared to mice receiving an adenovirus construct producing only the backbone human IgG-fusion protein. The degree of disease acceleration was comparable to that seen in mice in which a ligand for OX2R, namely OX2, had been inactivated by gene targeting.

Alternatively, if the various OX2R molecules described herein have activating vs. inhibitory function, specific activation of the OX2R that induces cellular activation may be appropriate. This may be achieved, e.g., by the use of specific antibody with agonistic activities of a given OX2R. In various embodiments, the method is applied where the animal experiences signs or symptoms of wound healing or clot formation in which enhanced macrophage activation may be desirable, or where an animal experiences a bacterial infection where enhanced phagocytic activity by granulocytes and/or macrophages is desirable. The administering will often be in combination with: an angiogenic factor; a growth factor, including FGF or PDGF; an antibiotic; or a clotting factor.

Recombinant receptors, muteins, agonist or antagonist antibodies thereto, or antibodies can be purified and then administered to a patient. These reagents can be combined for therapeutic use with additional active ingredients, e.g., in conventional pharmaceutically acceptable carriers or diluents, along with physiologically innocuous stabilizers and excipients. These combinations can be sterile, e.g., filtered, and placed into dosage forms as by lyophilization in dosage vials or storage in stabilized aqueous preparations. This invention also contemplates use of antibodies or binding fragments thereof which are not complement binding.

Ligand screening using receptor or fragments thereof can be performed to identify molecules having binding affinity to the receptors. Subsequent biological assays can then be utilized to determine if a putative ligand can provide competitive binding, which can block intrinsic stimulating activity. Receptor fragments can be used as a blocker or antagonist in that it blocks the activity of ligand. Likewise, a compound having intrinsic stimulating activity can activate the receptor and is thus an agonist in that it simulates the activity of ligand, e.g., inducing signaling. This invention further contemplates the therapeutic use of antibodies to receptors as antagonists.

The quantities of reagents necessary for effective therapy will depend upon many different factors, including means of administration, target site, reagent physiological life, pharmacological life, physiological state of the patient, and other medicants administered. Thus, treatment dosages should be titrated to optimize safety and efficacy. Typically, dosages used in vitro may provide useful guidance in the amounts useful for in situ administration of these reagents. Animal testing of effective doses for treatment of particular disorders will provide further predictive indication of human dosage. Various considerations are described, e.g., in Gilman, et al. (eds. 1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Penn.; each of which is hereby incorporated herein by reference. Methods for administration are discussed therein and below, e.g., for oral, intravenous, intraperitoneal, or intramuscular administration, transdermal diffusion, and others. Pharmaceutically acceptable carriers will include water, saline, buffers, and other compounds described, e.g., in the Merck Index, Merck & Co., Rahway, New Jersey. Dosage ranges would ordinarily be expected to be in amounts lower than 100 mM concentrations, typically less than about 1 mM concentrations, usually less than about 100 µM, preferably less than about 1 µM, and most preferably less than about 10 nM, with an appropriate carrier. Slow release formulations, or slow release apparatus will often be utilized for continuous administration.

Receptor homologs, fragments thereof, and antibodies or its fragments, antagonists, and agonists, may be administered directly to the host to be treated or, depending on the size of the compounds, it may be desirable to conjugate them to carrier proteins such as ovalbumin or serum albumin prior to their administration. Therapeutic formulations may be administered in many conventional dosage formulations. While it is possible for the active ingredient to be administered alone, it is preferable to present it as a pharmaceutical formulation. Formulations comprise at least one active ingredient, as defined above, together with one or more acceptable carriers thereof. Each carrier must be both pharmaceutically and physiologically acceptable in the sense of being compatible with the other ingredients and not injurious to the patient. Formulations include those suitable for oral, rectal, nasal, or parenteral (including subcutaneous, intramuscular, intravenous and intradermal) administration. The formulations may conveniently be presented in unit dosage form and may be prepared by methods well known in the art of pharmacy. See, e.g., Gilman, et al. (eds. 1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Penn.; Avis, et al. (eds. 1993) Pharmaceutical Dosage Forms: Parenteral Medications Dekker, NY; Lieberman, et al. (eds. 1990) Pharmaceutical Dosage Forms: Tablets Dekker, NY; and Lieberman, et al. (eds. 1990) Pharmaceutical Dosage Forms: Disperse Systems Dekker, NY. The therapy of this invention may be combined with or used in association with other therapeutic agents, particularly agonists or antagonists of other receptor family members.

### IX. Screening

Drug screening using OX2RH or fragments thereof can be performed to identify compounds having binding affinity to the receptor homolog, including isolation of associated components. Subsequent biological assays can then be utilized to determine if the compound has intrinsic stimulating activity and is therefore a blocker or antagonist in that it blocks the activity of the ligand. Likewise, a compound having intrinsic stimulating activity can activate the receptor and is thus an agonist in that it simulates the activity of a ligand, e.g., OX2. This invention further contemplates the therapeutic use of antibodies to the receptor as agonists or antagonists.

Similarly, complexes comprising multiple proteins may be used to screen for ligands or reagents capable of recognizing the complex. Some receptors comprise at least two subunits, which may be the same, or distinct. Alternatively, the transmembrane receptor may bind to a complex comprising a ligand associated with another soluble protein serving, e.g., as a second receptor subunit.

One method of drug screening utilizes eukaryotic or prokaryotic host cells which are stably transformed with recombinant DNA molecules expressing the OX2RH in combination with another receptor subunit. Cells may be isolated which express a receptor in isolation from other functional receptors. Such cells, either in viable or fixed form, can be used for standard antibody/antigen or ligand/receptor binding assays. See also, Parce, et al. (1989) Science 246:243-247; and Owicki, et al. (1990) Proc. Nat'l Acad. Sci. USA 87:4007-4011, which describe sensitive methods to detect cellular responses. Competitive assays are particularly useful, where the cells (source of putative ligand) are contacted and incubated with a labeled receptor or antibody having known binding affinity to the ligand, such as ¹²⁵I-antibody, and a test sample whose binding affinity to the binding composition is being measured. The bound and free labeled binding compositions are then separated to assess the degree of ligand binding. The amount of test compound bound is inversely proportional to the amount of labeled receptor binding to the known source. Many techniques can be used to separate bound from free ligand to assess the degree of ligand binding. This separation step could typically involve a procedure such as adhesion to filters followed by washing, adhesion to plastic followed by washing, or centrifugation of the cell membranes. Viable cells could also be used to screen for the effects of drugs on OX2 mediated functions, e.g., second messenger levels, i.e., Ca⁺⁺; cell proliferation; inositol phosphate pool changes; and others. Some detection methods allow for elimination of a separation step, e.g., a proximity sensitive detection system. Calcium sensitive dyes will be useful for detecting Ca⁺⁺ levels, with a fluorimeter or a fluorescence cell sorting apparatus.

### X. Ligands

The descriptions of the OX2RHs herein provides means to identify ligands, as described above. Such ligand should bind specifically to the respective receptor with reasonably high affinity. Various constructs are made available which allow either labeling of the receptor to detect its ligand. For example, directly labeling receptor, fusing onto it markers for secondary labeling, e.g., FLAG or other epitope tags, etc., will allow detection of receptor. This can be histological, as an affinity method for biochemical purification, or labeling or selection in an expression cloning approach. A two-hybrid selection system may also be applied making appropriate constructs with the available receptor sequences. See, e.g., Fields and Song (1989) Nature 340:245-246.

The broad scope of this invention is best understood with reference to the following examples, which are not intended to limit the inventions to the specific embodiments.

### EXAMPLES

### I. General Methods

Some of the standard methods are described or referenced, e.g., in Maniatis, et al. (1982) Molecular Cloning, A Laboratory Manual, Cold Spring Harbor Laboratory, Cold Spring Harbor Press; Sambrook, et al. (1989) Molecular Cloning: A Laboratory Manual, (2d ed.), vols. 1-3, CSH Press, NY; or Ausubel, et al. (1987 and Supplements) Current Protocols in Molecular Biology, Greene/Wiley, New York. Methods for protein purification include such methods as ammonium sulfate precipitation, column chromatography, electrophoresis, centrifugation, crystallization, and others. See, e.g., Ausubel, et al. (1987 and periodic supplements); Coligan, et al. (ed. 1996) and periodic supplements, Current Protocols In Protein Science Greene/Wiley, New York; Deutscher (1990) "Guide to Protein Purification" in Methods in Enzymology, vol. 182, and other volumes in this series; and manufacturer's literature on use of protein purification products, e.g., Pharmacia, Piscataway, N.J., or Bio-Rad, Richmond, CA. Combination with recombinant techniques allow fusion to appropriate segments, e.g., to a FLAG sequence or an equivalent which can be fused via a protease-removable sequence. See, e.g., Hochuli (1990) "Purification of Recombinant Proteins with Metal Chelate Absorbent" in Setlow (ed.) Genetic Engineering, Principle and Methods 12:87-98, Plenum Press, N.Y.; and Crowe, et al. (1992) OIAexpress: The High Level Expression & Protein Purification System QUIAGEN, Inc., Chatsworth, CA.

Computer sequence analysis is performed, e.g., using available software programs, including those from the GCG (U. Wisconsin) and GenBank sources. Public sequence databases were also used, e.g., from GenBank and others.

Many techniques applicable to IL-10 receptors may be applied to the OX2RHs, as described, e.g., in USSN 08/110,683 (IL-10 receptor), which is incorporated herein by reference.

### II. Monoclonal Antibody which blocks rat OX2/OX2RH interaction on macrophages

A bead assay was set up using recombinant OX2-CD4 protein and rat peritoneal macrophages. See Preston, et al. (1997) Eur. J. Immunol. 27:1911-1918. Macrophages bound to fluorescent beads coated with recombinant OX2-CD4 proteins. Six wk old BALB/c mice were immunized 6 times with either 0.1-0.25 mg crude membrane fraction (Williams and Barclay (1986) in Handbook of Experimental Immunology vol. 1, 22.1-22.24, Blackwell Scientific Publications) or resident rat peritoneal exudate cells (5 million). Mice were screened for high titers of antibodies recognizing macrophages by testing various dilutions of the sera for labeling of macrophages by indirect immunofluorescence and flow cytometry. Mice producing good immune responses to the rat macrophages were finally boosted by injection of peritoneal exudate cells. Four days later, spleens were removed and fused to NS-1 myeloma cells to produce hybridomas. The final injection before screening was intrasplenic. Hybridoma supernatants were screened for the ability to label rat macrophages and for the ability to block the rat OX2 interaction with macrophages. One antibody, designated OX102, was obtained and cloned. This antibody gave clear blocking. This hybridoma was grown in bulk and the antibody was purified by standard procedures.

### III. Purification of the antigen for the OX102 mAb

Purified OX102 mAb was covalently coupled to CNBr activated sepharose-4B (Pharmacia) as recommended by the manufacturer. Membrane proteins were solubilised using Tween 40 and sodium deoxycholate and incubated with the Sepharose beads coupled to OX102 mAb, for 70 hours. Williams and Barclay (1986) in Handbook of Experimental Immunology vol. 1, 22.1-22.24, Blackwell Scientific Publications. The OX102 mAb-coupled Sepharose beads were pelleted by centrifugation and washed in 0.1% sodium dodecyl sulphate (SDS) and finally eluted in 0.5% SDS at 55° C for 15 min The eluted fraction was analysed by SDS polyacrylamide gel electrophoresis (SDS-PAGE).

### IV. N-terminal sequence of the antigen for the OX102 mAb

Amino terminal sequencing was performed using automated Edman degradation in an Applied Biosystems Procise 494A protein sequencer (Perkin-Elmer Ltd., UK). The N-terminal sequence was confirmed, as shown in Table 1. Blank cycles are assumed to be asparagine due to the presence of asparagine modified by N-linked glycosylation. The purified polypeptide was identified as novel by screening known protein databases with the N-terminal 20 amino acids of the antigen for the OX102 mAb. This protein is the rat OX2RH1.

### V. Isolation of cDNA clones coding for the antigen of the OX102 mAb

Total RNA was extracted from rat peritoneal exudate cells using RNAzol B (Biogenesis) and then the poly-A fraction purified using oligo dT beads (Oligotex, QIAGEN) as recommended by the manufacturer. Approximately 50 ng of polyA+ purified mRNA was treated with 200 U of Superscript II reverse transcriptase (GIBCO BRL) in the presence of 1 µM of selected sense and antisense oligonucleotides, 1 mM dNTPs, and 2 mM DTT, 50 mM Tris-HCl pH 8.3, 75 mM KC1, 3 mM MgCl₂, and incubated at 42° C for 1 h.

This cDNA was then used as a template in a PCR reaction, e.g., 40 µl of provided 10x Advantage Taq thermophilic PCR buffer (provided by Clontech); 8 µl of 10 mM dNTPs; 8 µl of Advantage Taq (Clontech); 2 µl of cDNA prepared as described above; 318 µl of distilled water; 16 µl of an antisense, degenerate oligonucleotide corresponding to the N-terminal peptide at 10 µM; and 8 µl 10 µM sense oligonucleotide. Both oligonucleotide primers were synthesized (Genosys) with a 5' terminal phosphate to facilitate cloning.

The PCR mix was aliquoted into 8 x 50 µl samples and subjected to PCR conditions in a Robocycler PCR machine (Stratagene) which allows the operator to vary the annealing temperature in separate samples simultaneously. Example parameters are: 93° C 30 sec; followed by 35 cycles of: 93° C 30 sec; 42-56° C 1 min; 72° C 30 sec; and a final cycle of 72° C for 8 min.

Ten µl of the PCR products were analyzed by agarose gel electrophoresis by standard procedures. PCR products of lengths ranging between 100 and 300 base pairs in the 3 samples which had an annealing temperature of about 42°, 44°, and 46° C were excised from the gel and the nucleic acid purified using QIAquick (QIAGEN). These purified products were ligated at about 16° C for 48 h using standard procedures into PCRScript vector (Stratagene) which had been SmaI digested and phosphatase treated.

Transformants were screened initially by colony PCR and 20 colonies containing appropriately-sized inserts were grown up in LB broth in the presence of 50 µg/ml ampicillin and plasmids purified by a QIAGEN robot. Inserts were sequenced using the BIGDYE fluorescent dideoxy-terminator technology and ABI-PRISM Model 377 (Perkin-Elmer Ltd., UK). Inserts containing nucleotide sequence that coded for the N-terminal sequence of antigen for the OX102 mAb were used to design oligonucleotides for 3' RACE reactions.

The full cDNA sequence of the antigen for OX102 was obtained by 3'RACE PCR (using the same protocol as above) but modified by using appropriate oligonucleotides at a final concentration of 0.2 µM each. PCR conditions, e.g., were: 93° C 30 sec; followed by 30 cycles of: 93° C 30 sec; 51°-65° C 1 min; 72° C 3.5 min; and a final cycle of 72° C for 12 min.

A band of approximately 2.3 Kb was excised from the 65° C PCR reaction, gel purified, digested with NotI and XhoI, and ligated into NotI/XhoI digested vector (PCRScript, Stratagene) using standard procedures. Inserts were sequenced as above.

The cDNA sequence of the OX102 protein, also referred to herein as rat OX2RH1, is shown in Table 1. Full length isolates of the other homolog embodiments are similarly cloned and sequences confirmed. Standard methods are readily applicable.

### VI. Obtaining other OX2RH cDNAs

The knowledge of the rat OX2RH1 nucleotide and predicted amino acid sequences allows one to obtain homologous functional equivalents from other species, including mouse or human OX2RH1, on the basis of sequence similarity and because the rat OX2RH1 provides a tool for the isolation of such equivalents.

Thus, to identify human OX2RH1, one can search existing databases of nucleotide and amino acid sequences for polypeptides of unknown identity (e.g., databases storing sequences obtained from the Human Genome Project) for sequences with homology to the rat OX2RH1 nucleic acid and amino acid sequences provided herein. The databases, which are stored and updated at many sites, including the European Bioinformatics Centre (http://www.ebi.ac.uk/) and National Center for Biotechnology Information (http://www.ncbi.nlm.nih.gov/), can be accessed by widely-used programs such as FASTA or BLAST. These databases include sequences for expressed sequence tags (ESTs) which are short regions of nucleotide sequence sequenced from random cDNA clones. This allows partial cDNA clones for mouse or human OX2RH to be isolated by comparison with the rat OX2RH sequence information provided herein. Full length clones can then be isolated by screening macrophage cDNA or genomic libraries or by primer extension techniques such as those described herein for obtaining the full-length rat OX2RH1 clones.

Mouse and human sequences related to the rat OX2RH1 were identified from genomic sequence database using, e.g., the BLAST server (Altschul, et al. (1994) Nature Genet. 6:119-129). Standard analysis programs may be used to evaluate structure, e.g., PHD (Rost and Sander (1994) Proteins 19:55-72) and DSC (King and Sternberg (1996) Protein Sci. 5:2298-2310). Standard comparison software includes, e.g., Altschul, et al. (1990) J. Mol. Biol. 215:403-10; Waterman (1995) Introduction to Computational Biology: Maps, Sequences, and Genomes Chapman & Hall; Lander and Waterman (eds. 1995) Calculating the Secrets of Life: Applications of the Mathematical Sciences in Molecular Biology National Academy Press; and Speed and Waterman (eds. 1996) Genetic Mapping and DNA Sequencing (IMA Volumes in Mathematics and Its Applications, Vol 81) Springer Verlag.

Nucleic acid sequence for rat and human OX2RH1 will have between 50 and 98% homology, e.g., as can be observed in Table 5. For the other homologs, the similarity may be less, especially with the homolog 3.

As an alternative to database screening, the rat OX2RH1 nucleic acid sequence as provided herein can be used to screen macrophage cDNA or genomic libraries to identify human sequences sufficiently homologous to hybridize under conditions of appropriate stringency. This approach was utilized to isolate the human OX2 gene using rat OX2 nucleic acid as a probe. McCaughan, et al. (1987) Immunogenetics 25:329-335). PCR based methods using templates of cDNA, genomic DNA, cDNA clones, or genomic clones as templates are also widely used, the general approach being exemplified by the isolation of mouse OX2 from cDNA. See Preston, et al. (1997) Eur. J. Immunol. 27:1911-1918.

PCR primers derived from the provided OX2RH sequences are used to probe a human, or other species or tissue, cDNA library. Sequences may be derived, e.g., from Tables 1-4, preferably those adjacent the ends of sequences. Full length cDNAs for primate, rodent, or other species OX2RHs are cloned, e.g., by DNA hybridization screening of λgt10 phage. PCR reactions are conducted using T. aquaticus Taqplus DNA polymerase (Stratagene) under appropriate conditions.

Further, the rat OX2RH1 sequence can be used to isolate the corresponding mouse OX2RH1 sequence, and to identify regions conserved between them. Particularly with the discovery of a group of homologs, regions of similarity may be identified. Conserved-region sequences provide useful reagents for identifying a given gene in a range of species. For instance domain 1 of mouse and rat OX2 are 90% identical at the amino acid level, compared to 77% identity between the same human and rat domains. Preston, et al. (1997) Eur. J. Immunol. 27:1911-1918.

Yet another method is to use antibody reagents to expression clone or identify crossreacting proteins expressed in cDNA libraries from appropriate cell types, e.g., macrophages, or from other species.

### VII. Chromosomal localization

The genes will be mapped. For example, chromosome spreads are prepared. In situ hybridization is performed on chromosome preparations obtained from phytohemagglutinin-stimulated lymphocytes, e.g., from human, cultured for 72 h. 5-bromodeoxyuridine is added for the final seven hours of culture (60 µg/ml of medium), to ensure a posthybridization chromosomal banding of good quality.

A PCR fragment, amplified with the help of primers, is cloned into an appropriate vector. The vector is labeled by nick-translation with ³H. The radiolabeled probe is hybridized to metaphase spreads at final concentration of 200 ng/ml of hybridization solution as described in Mattei, et al. (1985) Hum. Genet. 69:327-331.

After coating with nuclear track emulsion (KODAK NTB₂), slides are exposed. To avoid any slipping of silver grains during the banding procedure, chromosome spreads are first stained with buffered Giemsa solution and metaphase photographed. R-banding is then performed by the fluorochrome-photolysis-Giemsa (FPG) method and metaphases rephotographed before analysis.

Similar appropriate methods are used for other species.

### VIII. Localization of various OX2RH mRNA

While the expected expression patterns of the OX2RH1 are primarily on macrophages, granulocytes, and mast cells, the homologs H2, H3, and/or H4 may not be so closely related functionally. Thus, the distribution of those will be of particular interest. Distribution may be evaluated at the nucleic acid level, e.g., by hybridization or PCR methods, or at the protein level, e.g., by histology or immunochemical methods.

Human multiple tissue (Cat #1, 2) and cancer cell line blots (Cat #7757-1), containing approximately 2 µg of poly(A)⁺ RNA per lane, are purchased from Clontech (Palo Alto, CA). Probes are radiolabeled with [α-³²P] dATP, e.g., using the Amersham Rediprime random primer labeling kit (RPN1633). Prehybridization and hybridizations are performed, e.g., at 65° C in 0.5 M Na₂HPO₄, 7% SDS, 0.5 M EDTA (pH 8.0). High stringency washes are conducted, e.g., at 65° C with two initial washes in 2 x SSC, 0.1% SDS for 40 min followed by a subsequent wash in 0.1 x SSC, 0.1% SDS for 20 min. Membranes are then exposed at -70° C to X-Ray film (Kodak) in the presence of intensifying screens. More detailed studies by cDNA library Southerns are performed with selected appropriate mammalian OX2RH clones to examine their expression in hemopoietic or other cell subsets.

Alternatively, two appropriate primers are selected, e.g., from Tables 1-4. RT-PCR is used on an appropriate mRNA sample selected for the presence of message to produce a cDNA, e.g., a sample which expresses the gene.

Full length clones may be isolated by hybridization of cDNA libraries from appropriate tissues pre-selected by PCR signal. Northern blots can be performed.

Message for genes encoding OX2RHs will be assayed by appropriate technology, e.g., PCR, immunoassay, hybridization, or otherwise. Tissue and organ cDNA preparations are available, e.g., from Clontech, Mountain View, CA. Identification of sources of natural expression are useful, as described.

For mouse distribution, e.g., Southern Analysis can be performed: DNA (5 µg) from a primary amplified cDNA library was digested with appropriate restriction enzymes to release the inserts, run on a 1% agarose gel and transferred to a nylon membrane (Schleicher and Schuell, Keene, NH).

Samples for mouse mRNA isolation may include: resting mouse fibroblastic L cell line (C200); Braf:ER (Braf fusion to estrogen receptor) transfected cells, control (C201); T cells, TH1 polarized (Mel14 bright, CD4+ cells from spleen, polarized for 7 days with IFN-γ and anti IL-4; T200); T cells, TH2 polarized (Mel14 bright, CD4+ cells from spleen, polarized for 7 days with IL-4 and anti-IFN-γ; T201); T cells, highly TH1 polarized (see Openshaw, et al. (1995) J. Exp. Med. 182:1357-1367; activated with anti-CD3 for 2, 6, 16 h pooled; T202); T cells, highly TH2 polarized (see Openshaw, et al. (1995) J. Exp. Med. 182:1357-1367; activated with anti-CD3 for 2, 6, 16 h pooled; T203); CD44-CD25+ pre T cells, sorted from thymus (T204); TH1 T cell clone D1.1, resting for 3 weeks after last stimulation with antigen (T205); TH1 T cell clone D1.1, 10 µg/ml ConA stimulated 15 h (T206); TH2 T cell clone CDC35, resting for 3 weeks after last stimulation with antigen (T207); TH2 T cell clone CDC35, 10 µg/ml ConA stimulated 15 h (T208); Mel14+ naive T cells from spleen, resting (T209); Mel14+ T cells, polarized to Th1 with IFN-γ/IL-12/anti-IL-4 for 6, 12, 24 h pooled (T210); Mel14+ T cells, polarized to Th2 with IL-4/anti-IFN-γ for 6, 13, 24 h pooled (T211); unstimulated mature B cell leukemia cell line A20 (B200); unstimulated B cell line CH12 (B201); unstimulated large B cells from spleen (B202); B cells from total spleen, LPS activated (B203); metrizamide enriched dendritic cells from spleen, resting (D200); dendritic cells from bone marrow, resting (D201); monocyte cell line RAW 264.7 activated with LPS 4 h (M200); bone-marrow macrophages derived with GM and M-CSF (M201); macrophage cell line J774, resting (M202); macrophage cell line J774 + LPS + anti-IL-10 at 0.5, 1, 3, 6, 12 h pooled (M203); macrophage cell line J774 + LPS + IL-10 at 0.5, 1, 3, 5, 12 h pooled(M204); aerosol challenged mouse lung tissue, Th2 primers, aerosol OVA challenge 7, 14, 23 h pooled (see Garlisi, et al. (1995) Clinical Immunology and Immunopathology 75:75-83; X206); Nippostrongulus-infected lung tissue (see Coffman, et al. (1989) Science 245:308-310; X200); total adult lung, normal (0200); total lung, rag-1 (see Schwarz, et al. (1993) Immunodeficiency 4:249-252; 0205); IL-10 K.O. spleen (see Kuhn, et al. (1991) Cell 75:263-274; X201); total adult spleen, normal (0201); total spleen, rag-1 (0207); IL-10 K.O. Peyer's patches (0202); total Peyer's patches, normal (0210); IL-10 K.O. mesenteric lymph nodes (X203); total mesenteric lymph nodes, normal (0211); IL-10 K.O. colon (X203); total colon, normal (0212); NOD mouse pancreas (see Makino, et al. (1980) Jikken Dobutsu 29:1-13; X205); total thymus, rag-1 (0208); total kidney, rag-1 (0209); total heart, rag-1 (0202); total brain, rag-1 (0203); total testes, rag-1 (0204); total liver, rag-1 (0206); rat normal joint tissue (0300); and rat arthritic joint tissue (X300).

Samples for human mRNA isolation may include: peripheral blood mononuclear cells (monocytes, T cells, NK cells, granulocytes, B cells), resting (T100); peripheral blood mononuclear cells, activated with anti-CD3 for 2, 6, 12 h pooled (T101) ; T cell, TH0 clone Mot 72, resting (T102) ; T cell, TH0 clone Mot 72, activated with anti-CD28 and anti-CD3 for 3, 6, 12 h pooled (T103); T cell, TH0 clone Mot 72, anergic treated with specific peptide for 2, 7, 12 h pooled (T104); T cell, TH1 clone HY06, resting (T107); T cell, TH1 clone HY06, activated with anti-CD28 and anti-CD3 for 3, 6, 12 h pooled (T108); T cell, TH1 clone HY06, anergic treated with specific peptide for 2, 6, 12 h pooled (T109); T cell, TH2 clone HY935, resting (T110); T cell, TH2 clone HY935, activated with anti-CD28 and anti-CD3 for 2, 7, 12 h pooled (T111); T cells CD4+CD45RO- T cells polarized 27 days in anti-CD28, IL-4, and anti IFN-γ, TH2 polarized, activated with anti-CD3 and anti-CD28 4 h (T116); T cell tumor lines Jurkat and Hut78, resting (T117); T cell clones, pooled AD130.2, Tc783.12, Tc783.13, Tc783.58, Tc782.69, resting (T118); T cell random γδ T cell clones, resting (T119); Splenocytes, resting (B100); Splenocytes, activated with anti-CD40 and IL-4 (B101); B cell EBV lines pooled WT49, RSB, JY, CVIR, 721.221, RM3, HSY, resting (B102); B cell line JY, activated with PMA and ionomycin for 1, 6 h pooled (B103); NK 20 clones pooled, resting (K100); NK 20 clones pooled, activated with PMA and ionomycin for 6 h (K101); NKL clone, derived from peripheral blood of LGL leukemia patient, IL-2 treated (K106); NK cytotoxic clone 640-A30-1, resting (K107); hematopoietic precursor line TF1, activated with PMA and ionomycin for 1, 6 h pooled (C100); U937 premonocytic line, resting (M100); U937 premonocytic line, activated with PMA and ionomycin for 1, 6 h pooled (M101); elutriated monocytes, activated with LPS, IFNγ, anti-IL-10 for 1, 2, 6, 12, 24 h pooled (M102); elutriated monocytes, activated with LPS, IFNγ, IL-10 for 1, 2, 6, 12, 24 h pooled (M103); elutriated monocytes, activated with LPS, IFNγ, anti-IL-10 for 4, 16 h pooled (M106); elutriated monocytes, activated with LPS, IFNγ, IL-10 for 4, 16 h pooled (M107); elutriated monocytes, activated LPS for 1 h (M108); elutriated monocytes, activated LPS for 6 h (M109); DC 70% CD1a+, from CD34+ GM-CSF, TNFα 12 days, resting (D101); DC 70% CD1a+, from CD34+ GM-CSF, TNFα 12 days, activated with PMA and ionomycin for 1 hr (D102); DC 70% CD1a+, from CD34+ GM-CSF, TNFα 12 days, activated with PMA and ionomycin for 6 hr (D103); DC 95% CD1a+, from CD34+ GM-CSF, TNFα 12 days FACS sorted, activated with PMA and ionomycin for 1, 6 h pooled (D104); DC 95% CD14+, ex CD34+ GM-CSF, TNFα 12 days FACS sorted, activated with PMA and ionomycin 1, 6 hr pooled (D105); DC CD1a+ CD86+, from CD34+ GM-CSF, TNFα 12 days FACS sorted, activated with PMA and ionomycin for 1, 6 h pooled (D106); DC from monocytes GM-CSF, IL-4 5 days, resting (D107); DC from monocytes GM-CSF, IL-4 5 days, resting (D108); DC from monocytes GM-CSF, IL-4 5 days, activated LPS 4, 16 h pooled (D109); DC from monocytes GM-CSF, IL-4 5 days, activated TNFα, monocyte supe for 4, 16 h pooled (D110); leiomyoma L11 benign tumor (X101); normal myometrium M5 (0115); malignant leiomyosarcoma GS1 (X103); lung fibroblast sarcoma line MRC5, activated with PMA and ionomycin for 1, 6 h pooled (C101); kidney epithelial carcinoma cell line CHA, activated with PMA and ionomycin for 1, 6 h pooled (C102); kidney fetal 28 wk male (0100); lung fetal 28 wk male (0101); liver fetal 28 wk male (0102); heart fetal 28 wk male (0103); brain fetal 28 wk male (0104); gallbladder fetal 28 wk male (0106); small intestine fetal 28 wk male (0107); adipose tissue fetal 28 wk male (0108); ovary fetal 25 wk female (0109); uterus fetal 25 wk female (0110); testes fetal 28 wk male (0111); spleen fetal 28 wk male (0112); adult placenta 28 wk (0113); and tonsil inflamed, from 12 year old (X100).

Similar samples may isolated in other species for evaluation. Histology may also be performed.

### IX. Production of mammalian OX2RH proteins

An appropriate, e.g., GST, fusion construct is engineered for expression, e.g., in E. coli. For example, a mouse OX2RH pGex plasmid is constructed and transformed into E. coli. Freshly transformed cells are grown, e.g., in LB medium containing 50 µg/ml ampicillin and induced with IPTG (Sigma, St. Louis, MO). After overnight induction, the bacteria are harvested and the pellets containing the OX2RH protein are isolated. The pellets are homogenized, e.g., in TE buffer (50 mM Tris-base pH 8.0, 10 mM EDTA and 2 mM pefabloc) in 2 liters. This material is passed through a microfluidizer (Microfluidics, Newton, MA) three times. The fluidized supernatant is spun down on a Sorvall GS-3 rotor for 1 h at 13,000 rpm. The resulting supernatant containing the OX2RH protein is filtered and passed over a glutathione-SEPHAROSE column equilibrated in 50 mM Tris-base pH 8.0. The fractions containing the OX2RH-GST fusion protein are pooled and cleaved, e.g., with thrombin (Enzyme Research Laboratories, Inc., South Bend, IN). The cleaved pool is then passed over a Q-SEPHAROSE column equilibrated in 50 mM Tris-base. Fractions containing OX2RH are pooled and diluted in cold distilled H₂O, to lower the conductivity, and passed back over a fresh Q-Sepharose column, alone or in succession with an immunoaffinity antibody column. Fractions containing the OX2RH protein are pooled, aliquoted, and stored in the -70° C freezer.

Various fusion constructs are made with OX2RH. Thus, e.g., fusion of the extracellular portions of the OX2RH2, H3, or H4 may be fused to intracellular portions of the DAP12 or to IgG domains or other labeling or functional domains. A portion of the appropriate gene is fused to an epitope tag, e.g., a FLAG tag, or to a two hybrid system construct. See, e.g., Fields and Song (1989) Nature 340:245-246.

The epitope tag may be used in an expression cloning procedure with detection with anti-FLAG antibodies to detect a binding partner, e.g., ligand for the respective receptor homolog. The two hybrid system may also be used to isolate proteins which specifically bind to an OX2RH.

Comparison of the CD spectrum with similar Ig superfamily receptor protein may suggest that the protein is correctly folded. See Hazuda, et al. (1969) J. Biol. Chem. 264:1689-1693; and Campbell, et al. (1979) Nature 282:341-342.

The reactivity of the OX2/OX2R in terms of binding properties, e.g., kinetics and functional effects, can be investigated. The interactions of the OX2R cytoplasmic domain can be determined using well established immunoprecipitation methods or genetic methods such as the yeast two-hybrid system. Transfection of the OX2RH into cells normally not expressing the proteins may be useful in physiological and signaling studies.

### X. Preparation of antibodies specific for OX2RHs

Appropriate species or strains, e.g., inbred Balb/c mice, are immunized intraperitoneally with recombinant forms of the protein, e.g., purified OX2RH or stable transfected NIH-3T3 cells. Animals are boosted at appropriate time points with protein, with or without additional adjuvant, to further stimulate antibody production. Serum is collected, or hybridomas produced with harvested spleens.

Alternatively, the animals, e.g., Balb/c mice, are immunized with cells transformed with the gene or fragments thereof, either endogenous or exogenous cells, or with isolated membranes enriched for expression of the antigen. Serum is collected at the appropriate time, typically after numerous further administrations. Various gene therapy techniques may be useful, e.g., in producing protein in situ, for generating an immune response. Serum or antibody preparations may be cross-absorbed or immunoselected to prepare substantially purified antibodies of defined specificity and high affinity. Thus, antibodies could be prepared which recognize various species counterparts, or antibodies which recognize specific species or groups of subsets, e.g., rodent, embodiments.

Monoclonal antibodies may be made. For example, splenocytes are fused with an appropriate fusion partner and hybridomas are selected in growth medium by standard procedures. Hybridoma supernatants are screened for the presence of antibodies which bind to the rat OX2RH1, e.g., by ELISA or other assay. Antibodies which specifically recognize specific OX2RH embodiments may also be selected or prepared.

In another method, synthetic peptides or purified protein are presented to an immune system to generate monoclonal or polyclonal antibodies. See, e.g., Coligan (ed. 1991) Current Protocols in Immunology Wiley/Greene; and Harlow and Lane (1989) Antibodies: A Laboratory Manual Cold Spring Harbor Press. In appropriate situations, the binding reagent is either labeled as described above, e.g., fluorescence or otherwise, or immobilized to a substrate for panning methods. Nucleic acids may also be introduced into cells in an animal to produce the antigen, which serves to elicit an immune response. See, e.g., Wang, et al. (1993) Proc. Nat'l. Acad. Sci. 90:4156-4160; Barry, et al. (1994) BioTechniques 16:616-619; and Xiang, et al. (1995) Immunity 2: 129-135.

### XI. Ligand binding and partner specificity

Means for testing of the binding selectivity and affinity are readily available. Surface plasmon resonance (see manufacturer's protocol; BIAcore manual, Pharmacia Biosensor) or other methods may be used to determine the ligand for the OX2RHs. The rat and mouse H1 bind to their species counterpart ligand OX2; the human H1 will be similarly tested. The H2 will be similarly tested against similar potential ligands, though the similarity of the extracellular domains of the H2 to the known receptor (rat and mouse H1) suggest the same or closely related ligand.

A receptor can be used as a specific binding reagent to identify its binding partner, by taking advantage of its specificity of binding, much like an antibody would be used. The binding receptor may be an OX2RH, or may involve, e.g., a complex of the OX2RH with another subunit. A binding reagent is either labeled as described above, e.g., fluorescence or otherwise, or immobilized to a substrate for panning methods.

The binding composition is used to screen an expression library made from a cell line which expresses a binding partner, i.e., ligand, preferably membrane associated. Standard staining techniques are used to detect or sort surface expressed ligand, or surface expressing transformed cells are screened by panning. Screening of intracellular expression is performed by various staining or immunofluorescence procedures. See also McMahan, et al. (1991) EMBO J. 10:2821-2832.

Alternatively, receptor reagents are used to affinity purify or sort out cells expressing a putative ligand. See, e.g., Sambrook, et al. or Ausubel, et al.

Another strategy is to screen for a membrane bound ligand by panning. The receptor cDNA is constructed as described above. Immobilization may be achieved by use of appropriate antibodies which recognize, e.g., a FLAG sequence on an OX2RH fusion construct, or by use of antibodies raised against the first antibodies. Recursive cycles of selection and amplification lead to enrichment of appropriate clones and eventual isolation of receptor expressing clones.

Phage expression libraries can be screened by mammalian OX2RH, e.g., labeled forms. Appropriate label techniques, e.g., anti-FLAG antibodies, will allow specific labeling of appropriate phage clones.

Upon confirmation of OX2-OX2RH binding, or identification of alternative ligands for the other homologs, signaling pathways will be tested. See, e.g., Preston, et al. (1997) Eur. J. Immunol. 27:1911-1918. Implications of the DAP12 involvement are also clear. See, e.g., Bakker, et al. (2000) Human Immunology 61:18-27; Lanier, et al. (1998) Immunity 8:693-701; Smith, et al. (1998) J. Immunol. 161:7-10; Gosselin, et al. (1999) J. Leukoc. Biol. 66:165-171; Tomasello, et al.(1998) J. Biol. Chem. 273:34115-34119; and McVicar, et al. (1998) J. Biol. Chem. 273:32934-32942. Similarly, or alternatively, DAP10 may be involved. See, e.g., Wu J, et al. (1999) Science 285:730-732; and Bauer, et al. (1999) Science 285:727-729.

In particular, the DAP12 coreceptor partner is in the same family as the T cell receptor subunit ζ and the FCεRγ, which possess ITIM motifs, and signal through the pathway involving the syk/zap70 protein tyrosine kinases. The DAP10 has the YxxM motif, which signals through or analogously to the PI3 kinase pathway.

Certain isoforms of the MHC class I receptors on NK cells lack ITIM sequences in their cytoplasmic domains and it has been proposed that these isoforms activate, rather than inhibit, NK cells. These activating receptors have very short intracellular regions lacking any signaling motifs and they all share a positively charged residue within their transmembrane domain, which suggested the association with an adapter molecule that is capable of signaling. DAP12, a type I disulfide-linked homodimer containing an ITAM non-covalently assembles with the human KIR2DS receptors. DAP12 has a negatively charged aspartic acid residue in its transmembrane region and corresponds to a reported 12-13 kD phosphoprotein that was found to co-immunoprecipitate with KIR2DS.

Upon receptor engagement, DAP12 becomes phosphorylated and recruits the Syk kinase, thus inducing a signaling cascade similar to T cell receptor. Besides being associated with KIR2DS, a receptor for HLA-C, DAP12 is also expressed at the cell surface of NK cells associated with the activating mouse Ly49D and Ly49H receptors recognizing H-2 and with the human CD94/NKG2C heterodimer receptor complex recognizing HLA-E.

Recent efforts to identify potential membrane signaling proteins by searching the EST databases have led to the identification of DAP10, a novel 10-kD surface adapter primarily expressed in hematopoietic cells. Although DAP10 has only limited homology with DAP12, its transmembrane domain contains a negatively charged residue that is conserved in the transmembrane regions of DAP12 and all of the CD3 subunits of the TCR. In addition, the conserved cysteine residues within the extracellular domain of DAP12 and the CD3 chains are also present in DAP10. Interestingly, the human DAP10 and DAP12 genes lie adjacent on chromosome 19q13.1, in opposite transcriptional orientation and separated by only approximately 130 base pairs, presumably as a result of gene duplication. One unique feature of DAP10 is its short, but conserved, cytoplasmic tail which, contains a YxxM signaling motif, a potential src-homology 2 (SH2) domain-binding site for the p85 regulatory subunit of the phosphatidylinositol 3-kinase (PI 3-kinase). The physical and functional association of various OX2RH with DAP12 or DAP10 can be determined.

### XII. Genetic analysis, animal studies

The sequences make available information and reagents useful for determination of the chromosomal mapping, disease marker correlation, and isolation and determination of the genetic structure of the respective genes. Intron/exon structure will be determined, and transgenic and deletion animals will be prepared. See, e.g., Goodnow (1992) "Transgenic Animals" in Roitt (ed.) Encyclopedia of Immunology, Academic Press, San Diego, pp. 1502-1504; Travis (1992) Science 256:1392-1394; Kuhn, et al. (1991) Science 254:707-710; Capecchi (1989) Science 244:1288; Robertson (ed. 1987) Teratocarcinomas and Embryonic Stem Cells: A Practical Approach, IRL Press, Oxford; Rosenberg (1992) J. Clinical Oncology 10:180-199; and Cournoyer and Caskey (1993) Ann. Rev. Immunol. 11:297-329.

To determine function of OX2-OX2R interaction in vitro and in vivo, an adenovirus construct was prepared that produced in soluble form, the extracellular region of OX2RH1 fused to human IgG. A control construct was prepared that produced in soluble form, only the human IgG backbone. In the first instance, supernatants containing these fusion proteins were produced by cellular infection in vitro to test whether the OX2R fusion protein had biological function on the basis of binding to normally expressed mouse OX2. In the first series of studies, tissue sections of mouse spleen from normal as well as OX2-gene knockout (KO) mice, were prepared and OX2R, or control fusion proteins were added, and these reagents detected by addition of an antibody binding to the human Fc portion of the fusion protein, and subsequent immunoperoxidase staining procedures to reveal binding. Weak binding only of the OX2R fusion protein, and only in normal but not OX2 KO mice, was detected on follicular dendritic cells and endothelial cells. Both cell types are know to express very high levels of the ligand OX2. Thus, the reagent bound to a physiological form of OX2 and no binding was observed in spleen where the ligand OX2 was absent due to gene targeting.

B cells are known to express the ligand OX2, but at lower level than on follicular dendritic cells and endothelial cells. Immunohistochemistry is not a particularly sensitive technique. Thus in a second study, the same fusion proteins were applied to isolated splenic leukocytes from normal and OX2 KO mouse and binding to B cells determined by flow cytometric analysis using mAb specific to the B220 molecule on B cells, and the more sensitive detection of the fusion proteins by secondary antibodies to human IgG coupled to phycoerythrin. In this case, all B cells in normal mice were labeled by the OX2R fusion protein but not by the control fusion protein. The interaction of the OX2R fusion protein with B cells was blocked by addition of a mAb called OX90 that is known to bind to the part of the mouse OX2 molecule that interacts with the OX2R. In addition, no binding above the background level seen with the control fusion protein was observed when OX2R fusion protein was added to B cells from OX2 KO mice.

The conclusions of these studies were: (1) that the OX2R fusion protein was biologically active and bound to OX2 on hematopoietic and non-hematopoietic cells; and (2) that the major ligand bound by OX2R is indeed the identified ligand OX2, on the basis of anti-OX2 (OX90) binding inhibition and lack of detectable binding to B cells from OX2 KO mice. These data cannot exclude the possibility that there are other ligands bound by the OX2R in addition to the known ligand OX2, as even flow cytometry had not detected cell-surface molecules expressed at very low level.

Transgenic mice can be generated by standard methods. Such animals are useful to determine the effects of deletion of the gene, in specific tissues, or completely throughout the organism. Such may provide interesting insight into development of the animal or particular tissues in various stages. Moreover, the effect on various responses to biological stress can be evaluated. See, e.g., Hogan, et al. (1995) Manipulating the Mouse Embryo: A Laboratory Manual (2d ed.) Cold Spring Harbor Laboratory Press. Likewise, deletion mice, e.g., knock out mice may be generated.

These animals will be subject to animal models to study the function of the genes in vivo. See, e.g., Gorczynski, et al. (1999) J. Immunol. 163:1654-1660; Mankoo, et al. (1999) Nature 400:69-73; Gorczynski, et al. (1999) Transplant. Proc. 31:577-578; and Gorczynski L, et al. (1999) J. Immunol. 162:774-781. Of particular interest will be the roles of macrophages or other myeloid cell populations, e.g., in the blood, lymphoid tissues, or solid organs, including the microglia in the nervous system. Tests of susceptibility to infection, autoimmune inflammation, and neural degeneration are indicated. Both antagonist and agonists will be useful reagents in the in vitro or in vivo models described or made available.

### XIII. Screening for Substances Likely to Have Therapeutic Value

The biological effect of the OX2R/OX2 interaction can be investigated by using antibody reactive with OX2R (an experimental substitute for OX2) to crosslink OX2R molecules in the macrophage cell surface membrane, and looking for changes in, e.g., nitric oxide production or phosphorylation of signaling proteins.

The effects of perturbing the OX2R/OX2 interaction can be tested using macrophages carrying OX2R, by exposing them to a cross-linking binding partner such as a mAb for OX2R (e.g., OX102) or a recombinant multivalent version of OX2 in the presence and absence of the candidate substance. Comparing activity of the macrophages (e.g., nitric oxide production or phosphorylation of signalling proteins) in the presence or absence of the candidate compound will indicate whether the candidate substance has a modulatory effect (e.g., inhibition or enhancement).

Candidate substances can also be tested in well established models of diseases in which macrophages are involved in the pathology of the disease, such as autoimmunity. For instance, established models such as experimental allergic encephalomyelitis can be used, as described and exemplified above, e.g., with the models exhibiting accelerated onset of the autoimmune disease experimental autoimmune encephalomyelitis (EAE) in mice. OX2R mimetics may be advantageous in chronic conditions such as chronic granulomatosis. Combinations of agonists or antagonists of the OX2/OX2R signaling may be combined with existing therapeutics for such conditions. See Physicians' Desk Reference Medical Economics Co, Montvale, NJ.

Analyses such as the above will indicate ways in which perturbation of the OX2R/OX2 interaction may be therapeutically beneficial. For example, the invention provides the means to make recombinant versions of OX2RH which can be used in conjunction with the available OX2 proteins to screen for possible pharmacological reagents which block the interaction. The availability of interacting proteins provides the means for high throughput small molecule screening programs, as are used in the pharmacology industry. See, e.g., meetings on High Throughput Screening, International Business Communications, Southborough, MA 01772-1749. Interactions through the cytoplasmic region of OX2RH are likely therapeutic targets and knowledge of the sequence and its interactions provide a means to develop pharmacological reagents through similar screening methods.

### XIV. Structure activity relationship

Information on the criticality of particular residues is determined using standard procedures and analysis. Standard mutagenesis analysis is performed, e.g., by generating many different variants at determined positions, e.g., at the positions identified above, and evaluating biological activities of the variants. This may be performed to the extent of determining positions which modify activity, or to focus on specific positions to determine the residues which can be substituted to either retain, block, or modulate biological activity.

Alternatively, analysis of natural variants can indicate what positions tolerate natural mutations. This may result from populational analysis of variation among individuals, or across strains or species. Samples from selected individuals are analyzed, e.g., by PCR analysis and sequencing. This allows evaluation of population polymorphisms.

All citations herein are incorporated herein by reference to the same extent as if each individual publication or patent application was specifically and individually indicated to be incorporated by reference.

Many modifications and variations of this invention can be made without departing from its spirit and scope, as will be apparent to those skilled in the art. The specific embodiments described herein are offered by way of example only, and the invention is to be limited by the terms of the appended claims, along with the full scope of equivalents to which such claims are entitled; and the invention is not to be limited by the specific embodiments that have been presented herein by way of example.

In other definitions of the invention, embodiments are provided as described in the following clauses:
1. A composition of matter selected from:
   a1) a substantially pure or recombinant polypeptide comprising at least three distinct nonoverlapping segments of at least four amino acids identical to segments of SEQ ID NO: 2;
   a2) a substantially pure or recombinant polypeptide comprising at least two distinct nonoverlapping segments of at least five amino acids identical to segments of SEQ ID NO: 2;
   a3) a natural sequence rodent OX2RH1 polypeptide comprising mature SEQ ID NO: 2;
   a4) a fusion polypeptide comprising rat OX2RH1 sequence;
   b1) a substantially pure or recombinant polypeptide comprising at least three distinct nonoverlapping segments of at least four amino acids identical to segments of SEQ ID NO: 4;
   b2) a substantially pure or recombinant polypeptide comprising at least two distinct nonoverlapping segments of at least five amino acids identical to segments of SEQ ID NO: 4;
   b3) a natural sequence rodent OX2RH1 polypeptide comprising mature SEQ ID NO: 4;
   b4) a fusion polypeptide comprising human OX2RH1 sequence;
   c1) a substantially pure or recombinant polypeptide comprising at least three distinct nonoverlapping segments of at least four amino acids identical to segments of SEQ ID NO: 6;
   c2) a substantially pure or recombinant polypeptide comprising at least two distinct nonoverlapping segments of at least five amino acids identical to segments of SEQ ID NO: 6;
   c3) a natural sequence rodent OX2RH1 polypeptide comprising mature SEQ ID NO: 6;
   c4) a fusion polypeptide comprising mouse OX2RH1 sequence;
   d1) a substantially pure or recombinant polypeptide comprising at least three distinct nonoverlapping segments of at least four amino acids identical to segments of SEQ ID NO: 8;
   d2) a substantially pure or recombinant polypeptide comprising at least two distinct nonoverlapping segments of at least five amino acids identical to segments of SEQ ID NO: 8;
   d3) a natural sequence rodent OX2RH1 polypeptide comprising mature SEQ ID NO: 8;
   d4) a fusion polypeptide comprising human OX2RH2 sequence;
   e1) a substantially pure or recombinant polypeptide comprising at least three distinct nonoverlapping segments of at least four amino acids identical to segments of SEQ ID NO: 10;
   e2) a substantially pure or recombinant polypeptide comprising at least two distinct nonoverlapping segments of at least five amino acids identical to segments of SEQ ID NO: 10;
   e3) a natural sequence rodent OX2RH2 polypeptide comprising mature SEQ ID NO: 10;
   e4) a fusion polypeptide comprising mouse OX2RH2 sequence;
   f1) a substantially pure or recombinant polypeptide comprising at least three distinct nonoverlapping segments of at least four amino acids identical to segments of SEQ ID NO: 12;
   f2) a substantially pure or recombinant polypeptide comprising at least two distinct nonoverlapping segments of at least five amino acids identical to segments of SEQ ID NO: 12;
   f3) a natural sequence rodent OX2RH3 comprising mature SEQ ID NO: 12;
   f4) a fusion polypeptide comprising mouse OX2RH3 sequence;
   g1) a substantially pure or recombinant polypeptide comprising at least three distinct nonoverlapping segments of at least four amino acids identical to segments of SEQ ID NO: 20;
   g2) a substantially pure or recombinant polypeptide comprising at least two distinct nonoverlapping segments of at least five amino acids identical to segments of SEQ ID NO: 20;
   g3) a natural sequence primate OX2RH1.2 polypeptide comprising mature SEQ ID NO: 20;
   g4) a fusion polypeptide comprising primate OX2RH1.2 sequence;
   h1) a substantially pure or recombinant polypeptide comprising at least three distinct nonoverlapping segments of at least four amino acids identical to segments of SEQ ID NO: 23;
   h2) a substantially pure or recombinant polypeptide comprising at least two distinct nonoverlapping segments of at least five amino acids identical to segments of SEQ ID NO: 23;
   h3) a natural sequence rodent OX2RH4 polypeptide comprising mature SEQ ID NO: 23; or
   h4) a fusion polypeptide comprising mouse OX2RH4 sequence.
2. A binding compound comprising an antigen binding site from an antibody, which specifically binds to a natural OX2RH polypeptide of 1, wherein:
   a) said binding compound is in a container;
   b) said OX2RH polypeptide is from a rodent or primate;
   c) said binding compound is an Fv, Fab, or Fab2 fragment;
   d) said binding compound is conjugated to another chemical moiety; or
   e) said antibody:
      i) is raised against a peptide sequence of a mature polypeptide of Tables 1-3;
      ii) is raised against a mature OX2RH;
      iii) is raised to a purified mammalian OX2RH;
      iv) is immunoselected;
      v) is a polyclonal antibody;
      vi) binds to a denatured OX2RH;
      vii) exhibits a Kd to antigen of at least 30 µM;
      viii) is attached to a solid substrate, including a bead or plastic membrane;
      ix) is in a sterile composition; or
      x) is detectably labeled, including a fluorescent label.
3. An isolated or recombinant nucleic acid encoding said OX2RH polypeptide of 1, wherein said:
   a) OX2RH is from a mammal; or
   b) said nucleic acid:
      i) encodes an antigenic peptide sequence of Tables 1-3;
      ii) encodes a plurality of antigenic peptide sequences of Tables 1-3;
      iii) exhibits identity over at least thirteen nucleotides to a natural cDNA encoding said segment;
      iv) is an expression vector;
      v) further comprises an origin of replication;
      vi) is from a natural source;
      vii) comprises a detectable label;
      viii) comprises synthetic nucleotide sequence;
      ix) is less than 6 kb, preferably less than 3 kb;
      x) is from a primate or rodent;
      xi) comprises a natural full length coding sequence;
      xii) is a hybridization probe for a gene encoding said OX2RH;
      xiii) further encodes DAP12 or DAP10; or
      xiv) is a PCR primer, PCR product, or mutagenesis primer.
4. A nucleic acid which:
   a) hybridizes under wash conditions of 30 minutes at 40° C and less than 2M salt to the coding portion of SEQ ID NO: 1, 3, 5, 7, 9, 11, 19, or 22; or
   b) exhibits identity over a stretch of at least about 30 nucleotides to a primate or rodent OX2RH cDNA.
5. A method of modulating physiology or development of a cell or tissue culture cells comprising contacting said cell with an agonist or antagonist of a mammalian OX2RH.
6. The method of 5, wherein said:
   a) modulating physiology is:
      i) enhancing meloid function; or
      ii) enhancing immunity;
   b) agonist or antagonist attentuates OX2 mediated signaling to said cell; or
   c) said antagonist is:
      i) an antibody to said OX2RH;
      ii) a soluble OX2RH construct;
      iii) a soluble OX2RH-Ig fusion; or
      iv) an OX2R antisense nucleic acid.
7. The method of 6, wherein:
   a) said modulating of physiology is enhancement of meloid cell function in vitro, and said antagonist is an OX2 mutein; or
   b) said modulating of physiology is enhancement of immunity in an animal being systemically treated with said antagonist.
8. A method for identification of a non-OX2 ligand for an OX2R, said method comprising screening a library of genes from an OX2 knock out mouse for binding to an OX2R-Ig fusion protein, and identifying genes which bind to said fusion protein.

### Sequence Listing Statement

The undersigned agent for applicant hereby declares that the information recorded on the diskette is identical in content to the information in the written Sequence Listing. The sequence listings do not go beyond the disclosure in the application as filed.

SEQUENCE SUBMISSION

SEQ ID NO: 1 is rodent OX2R nucleotide sequence.
SEQ ID NO: 2 is rodent OX2R polypeptide sequence.
SEQ ID NO: 3 is primate OX2R homolog 1 nucleotide sequence.
SEQ ID NO: 4 is primate OX2R homolog 1 polypeptide sequence.
SEQ ID NO: 5 is rodent OX2R homolog 1 nucleotide sequence.
SEQ ID NO: 6 is rodent OX2R homolog 1 polypeptide sequence.
SEQ ID NO: 7 is primate OX2R homolog 2 nucleic acid sequence.
SEQ ID NO: 8 is primate OX2R homolog 2 polypeptide sequence.
SEQ ID NO: 9 is rodent OX2R homolog 2 nucleic acid sequence.
SEQ ID NO: 10 is rodent OX2R homolog 2 polypeptide sequence.
SEQ ID NO: 11 is rodent OX2R homolog 3 nucleic acid sequence.
SEQ ID NO: 12 is rodent OX2R homolog 3 polypeptide sequence.
SEQ ID NO: 13 is rodent OX2R polypeptide encoding sequence.
SEQ ID NO: 14 is primate OX2R homolog 1 polypeptide encoding sequence.
SEQ ID NO: 15 is rodent OX2R hiomolog 1 polypeptide encoding sequence.
SEQ ID NO: 16 is primate OX2R homolog 2 polypeptide encoding sequence.
SEQ ID NO: 17 is rodent OX2R homolog 2 polypeptide encoding sequence.
SEQ ID NO: 18 is rodent OX2R homolog 3 polypeptide encoding sequence.
SEQ ID NO: 19 is primate OX2R homolog 1.2 nucleotide sequence.
SEQ ID NO: 20 is primate OX2R homolog 1.2 polypeptide sequence.
SEQ ID NO: 21 is primate OX2R homolog 1.2 polypeptide encoding sequence.
SEQ ID NO: 22 is rodent OX2R homolog 4 nucleic acid sequence.
SEQ ID NO: 23 is rodent OX2R homolog 4 polypeptide sequence.
SEQ ID NO: 24 is rodent OX2R homolog 4 polypeptide encoding sequence.

## Claims

1. An isolated polypeptide comprising the amino acid sequence set forth in SEQ ID NO: 4.

2. The polypeptide of claim 1 comprising the mature amino acid sequence of a polypeptide comprising the amino acid sequence of SEQ ID NO: 4.

3. The polypeptide of claim 1 comprising the extracellular domain sequence of polypeptide comprising the amino acid sequence of SEQ ID NO: 4.

4. A fusion protein comprising the polypeptide of any of claims 1 to 3 fused to a heterologous protein.

5. An isolated nucleic acid encoding the polypeptide of any of claims 1 to 3.

6. The nucleic acid of claim 5 comprising the nucleotide sequence of SEQ ID NO: 3.

7. An isolated antibody or antigen-binding fragment thereof which specifically binds the polypeptide of any of claims 1 to 3.

8. The antibody or fragment of claim 7 which is a monoclonal antibody, a polyclonal antibody, an Fab or a F(ab)2.

9. The antibody or fragment of claim 7 or claim 8 which is a monoclonal antibody.

10. A pharmaceutical composition comprising the antibody or fragment of any of claims 7 to 9 and a pharmaceutically acceptable carrier.

11. The polypeptide of any of claims 1 to 3for use as a medicament.

12. The nucleic acid of claim 5 or claim 6 for use as a medicament.

13. The antibody of any of claims 7 to 9 for use as a medicament.
